# EUROPEAN PATENT APPLICATION

(11) **EP 4 714 385 A1**
(43) Date of publication of application: **25.03.2026**
(21) Application number: 25204343.5
(22) Date of filing: 24.09.2025
(51) Int. Cl.: A61B 34/10, A61B 34/00

(54) **METHODS FOR SURGICAL PLANNING AND VISUALIZATION OF PATIENT ANATOMY**

(30) Priority: 24.09.2024 US 202463698367 P
(71) Applicant: Stryker Corporation, Portage, MI 49002 (US)
(72) Inventor: LAMBERS, Floor Mariet, Portage, 49002 (US); NEUMAN, Markus, Portage, 49002 (US); DO, Chau, Portage, 49002 (US); ACLO, Mary Katherine, Portage, 49002 (US); MORILLO, Gaia, Portage, 49002 (US); LINGNER, Kai, Portage, 49002 (US)
(74) Representative: V.O.

(57) **Abstract**

A method for visualizing a patient shoulder anatomy is provided. The method includes receiving image data of the patient shoulder anatomy, identifying a boundary of a lesion on a humerus based on the image data, generating one or more 3D models based on a segmentation of the image data, determining a location of a glenoid track corresponding to a contact between the humerus and a glenoid based on the one or more 3D models, generating a first virtual object based on the location of the glenoid track, and displaying: at least a portion of a rendering of the one or more 3D models, the boundary of the lesion, and the first virtual object.

## Description

### CROSS REFERENCE TO RELATED APPLICATIONS

This application claims priority to U.S. Provisional Application No. 63/698,367, filed on September 24, 2024, which is hereby incorporated by reference in its entirety.

### BACKGROUND

The glenohumeral joint is highly mobile and susceptible to dislocation. When healthy, the humerus is attached to the glenoid by the muscles of a rotator cuff, padded by soft tissue, and freely rotates relative to the glenoid. A healthy glenohumeral joint allows the shoulder to have a large and comfortable range of motion. However, shoulder dislocation is a common injury that results in the removal of the humerus from the glenoid. After one occurrence of a shoulder dislocation, subsequent dislocation becomes more likely due to increased instability of the glenohumeral joint. This damage may be in the form of a lesion on the humeral head or a lesion on the glenoid and impacts the engagement between the humerus and the glenoid, leading to joint instability. Surgical procedures exist to resolve these lesions, but surgical planning must be completed to determine which procedures may be suitable. Therefore, some assessment of the glenohumeral joint must be completed to determine the condition of the shoulder and what prospective surgeries may be recommended.

An assessment of the shoulder anatomy may inform the likelihood of future dislocation of the glenohumeral joint. Depending on this assessment, surgeons can generate a surgical plan according to the needs of the patient, which could include soft tissue repair, bone grafting, capsular plication, capsular shift or joint replacement. Without this assessment, surgeons have very little guidance for decision making to restore the health of the patient's glenohumeral joint. To complete an assessment, surgeons may visualize the patient shoulder anatomy and complete an instability analysis of the joint. A 3D model of the joint may be generated from image data captured by an imaging system and may be adjusted by a surgeon to provide information to evaluate the state of the patient shoulder anatomy. Current practices are time-intensive, laborious, and prone to inconsistency.

### SUMMARY

Other objects, features and advantages of the present invention will be readily appreciated as the same becomes better understood after reading the subsequent description taken in connection with the accompanying drawings.

According to a first aspect, a method of visualizing a patient shoulder anatomy is provided. The method includes receiving image data of the patient shoulder anatomy, identifying a boundary of a lesion on a humerus based on the image data, and generating one or more 3D models based on a segmentation of the image data. The method also includes determining a location of a glenoid track corresponding to a contact between the humerus and a glenoid based on the one or more 3D models, generating a first virtual object based on the location of the glenoid track, and displaying at least a portion of a rendering of the one or more 3D models, the boundary of the lesion, and the first virtual object.

According to a second aspect, a method of visualizing a patient shoulder anatomy is provided. The method includes receiving image data of the patient shoulder anatomy including at least a portion of soft tissue and bones of a glenohumeral joint. The method also includes generating one or more 3D models based on a segmentation of the image data, determining a location of at least one insertion point of the soft tissue corresponding to an attachment of soft tissue to the bones of the glenohumeral joint, and generating a first virtual object based on the at least one insertion point of the soft tissue. The method also includes determining the location of a glenoid track corresponding to a contact between a humerus and a glenoid based on the one or more 3D models and the first virtual object, generating a second virtual object based on the location of the glenoid track, and displaying at least a portion of a rendering of the one or more 3D models, the first virtual object, and the second virtual object.

According to a third aspect, a method of visualizing a patient shoulder anatomy is provided. The method includes receiving image data of the patient shoulder anatomy including a glenoid and a humerus. The method also includes generating one or more 3D models based on a segmentation of the image data, where the one or more 3D models may include a glenoid 3D model, generating a geometric primitive based on the glenoid 3D model, and determining a glenoid width based on the geometric primitive and the glenoid 3D model. The method also includes determining a location of a glenoid track based on the glenoid width, generating a virtual object based on the location of the glenoid track, and displaying at least a portion of a rendering of the one or more 3D models, and the virtual object based on the location of the glenoid track.

According to a fourth aspect, a method of visualizing a patient shoulder anatomy is provided. The method includes receiving a first virtual object representing a planned bone block for joint reconstruction, the planned bone block including a reconstruction dimension. The method also includes receiving a second virtual object representing an existing engagement between a humerus and a glenoid based on the patient shoulder anatomy, determining a reconstruction rating representing a modified engagement between the humerus and the glenoid based on the reconstruction dimension and the second virtual object, and displaying the reconstruction rating.

According to a fifth aspect, a method of assessing a patient shoulder anatomy is provided. The method includes receiving characteristics of a glenoid track corresponding to an engagement between a humerus and a glenoid. The method also includes receiving characteristics of a healthy glenoid track corresponding to an engagement between a healthy humerus and a healthy glenoid, determining a dimension of a bone block implant based on the characteristics of the glenoid track and the healthy glenoid track, and displaying an indicator based on the dimension.

According to a sixth aspect, a method of visualizing a patient shoulder anatomy is provided. The method includes receiving image data of the patient shoulder anatomy. The method also includes identifying a boundary of a lesion on a humerus based on the image data, generating one or more 3D models based on a segmentation of the image data, identifying a humeral neck axis based on a portion of the humerus in the one or more 3D models, and identifying a humeral head apex based on the humeral neck axis and the one or more 3D models. The method also includes generating a first set of lines perpendicular to the humeral neck axis, generating a second set of lines perpendicular to the first set of lines and passing through the humeral head apex, and displaying: at least a portion of a rendering of the one or more 3D models, the humeral head apex, the first set of lines, the second set of lines, and the boundary of the lesion.

According to a seventh aspect, a method of visualizing a patient shoulder anatomy is provided. The method includes receiving image data of the patient shoulder anatomy. The method also includes identifying a boundary of a lesion on a humerus based on the image data, generating one or more 3D models based on segmentation of the image data, and includes identifying a humeral head apex based on the one or more 3D models. The method also includes determining at least one distance based on the boundary of the lesion and the humeral head apex and displaying: at least a portion of a rendering of the one or more 3D models, the boundary of the lesion, the humeral head apex, and at least one distance between the boundary of the lesion and the humeral head apex.

According to an eighth aspect, a method of visualizing a patient shoulder anatomy is provided. The method includes receiving image data of the patient shoulder anatomy, identifying a boundary of a lesion on a humerus based on the image data, and generating one or more 3D models based on a segmentation of the image data. The method also includes identifying a humeral neck axis based on the one or more 3D models, identifying a lesion line based on the one or more 3D models, determining an angle based on the lesion line and the humeral neck axis, and displaying: at least a portion of a rendering of the one or more 3D models, the boundary of the lesion, and the angle between the lesion line and the humeral neck axis.

Any of the above aspects can be combined in part or in whole with any other aspect. Any of the above aspects, whether combined in part or in whole, can be further combined with any of the following implementations, in full or in part.

The method of visualizing a patient shoulder anatomy may further include determining a location of a healthy glenoid track corresponding to the contact between the humerus and a healthy glenoid based on the one or more 3D models and generating a second virtual object based on the location of the healthy glenoid track. The second virtual object may be a healthy glenoid track object and may further include displaying the healthy glenoid track object. The one or more 3D models may include a glenoid 3D model, the first virtual object may be a glenoid track object, and determining the location of the glenoid track may include determining a glenoid width corresponding to the patient shoulder anatomy based on the glenoid 3D model. Determining the glenoid width may include determining a bone loss measure corresponding to the patient shoulder anatomy based on the glenoid 3D model, determining a healthy glenoid width corresponding to a healthy glenoid based on the glenoid 3D model, applying statistical shape model fitting to the glenoid 3D model, determining a width of a contralateral glenoid of the patient shoulder anatomy, and generating a circle representative of the healthy glenoid width based on the glenoid 3D model, then measuring a diameter of the circle. Determining the bone loss measure may include generating a circle representative of a healthy glenoid width based on the glenoid 3D model determining the bone loss measure based on a diameter of the circle. The method of visualizing a patient shoulder anatomy may further include determining at least one attachment point of soft tissue to a bone of the patient shoulder anatomy and generating a virtual object corresponding to at least one attachment point of soft tissue. The virtual object corresponding to at least one attachment point of soft tissue may be further defined as a third virtual object and generating the first virtual object may include determining a boundary of the first virtual object based on the third virtual object, the healthy glenoid width, the bone loss measure, and a threshold value. Determining the boundary of the first virtual object may include multiplying the healthy glenoid width by the threshold value and subtracting the bone loss measure. The boundary of the first virtual object may be further defined as a first boundary and generating a second virtual object based on a location of a healthy glenoid track corresponding to the contact between the humerus and a healthy glenoid may include determining a second boundary of the healthy glenoid track based on the third virtual object, the healthy glenoid width, and the threshold value. Determining the second boundary may include multiplying the healthy glenoid width by the threshold value.

The method of visualizing patient anatomy may further include identifying a representation of the lesion based on the image data by providing at least a portion of the image data as an input to a deep learning network and receiving the representation of the lesion as an output from the deep learning network. The one or more 3D models may include a humerus 3D model and the method may further include displaying the representation of the lesion on the rendering of the humerus 3D model. The boundary of the lesion on the humerus may be identified by applying one or more algorithms to the one or more 3D models, the one or more algorithms selected from the group comprising: statistical shape modeling, watershed analysis, edge detection, and curvature analysis. The method of visualizing patient shoulder anatomy may further include determining an impact rating representing joint engagement based on the boundary of the lesion and a boundary of the first virtual object and displaying an indicator based on the impact rating. The indicator may include a bone width necessary to restore a glenoid width to a healthy glenoid width.

Determining the location of at least one insertion point of the soft tissue corresponding to the attachment of soft tissue to the bones of the glenohumeral joint may include applying a machine learning model to the image data of the patient shoulder anatomy, applying curvature analysis to the image data of the patient shoulder anatomy, and fitting a plane based on a head of the humerus 3D model and a distance derived from a radius of the head of the humerus 3D model.

Determining the glenoid width may be based on a geometric primitive and may include determining a center of the glenoid 3D model of the patient shoulder anatomy, determining a center of the geometric primitive, generating a first virtual object connecting the center of the glenoid 3D model and the center of the geometric primitive, and generating a second virtual object perpendicular to the first virtual object. Determining the glenoid width based on the geometric primitive may further include determining a glenoid rim based on the patient shoulder anatomy, determining a bone loss edge based on the glenoid rim inside of the second virtual object, determining a bone loss measure by measuring a distance between the bone loss edge and the second virtual object, and determining a healthy glenoid width based on a diameter of the second virtual object.

The method of visualizing patient shoulder anatomy may include determining a reconstructed glenoid track width based on the reconstruction dimension and the second virtual object. Determining the reconstructed glenoid track width may include determining a bone loss measure corresponding to the patient shoulder anatomy. Determining the bone loss measure may include generating a circle representative of a healthy glenoid width based on the patient shoulder anatomy and determining the bone loss measure based on a diameter of the circle. Determining the reconstructed glenoid track width may include multiplying a diameter of the circle by a threshold value, subtracting the bone loss measure, and adding the reconstruction dimension, or be based on the reconstruction dimension and a width of a glenoid track based on the patient shoulder anatomy, and may further include multiplying the reconstructed glenoid width by the threshold value. Determining the reconstruction dimension may be based on a threshold dimension of the reconstructed glenoid track width or a threshold glenoid bone loss value. Displaying the reconstruction rating may include showing the reconstruction dimension.

The method of visualizing patient shoulder anatomy may include determining a reconstruction position based on the characteristics of the glenoid track and the healthy glenoid track. The characteristics of the glenoid track may include a glenoid track width based on the patient shoulder anatomy, or a healthy glenoid track width based on the patient shoulder anatomy. Determining the dimension of the bone block implant may include multiplying the glenoid track width by a threshold to determine a reconstruction width and comparing the reconstruction width to the healthy glenoid track width. The method may further include determining a coracoid dimension based on image data and a statistical shape model and comparing the coracoid dimension with the dimension of the bone block implant. The indicator may be based on the comparison of the coracoid dimension to the dimension of the bone block implant. The method may further include displaying the reconstruction position on a portion of a rendering of one or more 3D models generated from a segmentation of image data of the patient shoulder anatomy.

The method of visualizing patient shoulder anatomy may include identifying a humeral head apex. The humeral head apex may be based on a humeral reference center. The first set of lines may be axially spaced along the humeral neck axis and indicate lateral-medial positions relative to the patient shoulder anatomy, the second set of lines may be radially spaced about the humeral neck axis and indicate superior-inferior positions relative to the patient shoulder anatomy, and the distance between the boundary of the lesion and the humeral head apex may be a geodesic distance or rotational angle. The one or more 3D models may include a humerus 3D model. The humerus 3D model may include an articular surface and a humeral head, and identifying the humeral neck axis may include fitting an articular sphere to the articular surface of the humerus 3D model. Identifying the humeral neck axis may further include identifying a contour of the humeral head based on an articular margin of the humerus 3D model and generating a virtual object based on the contour. Identifying the articular margin of the humerus 3D model may include determining an articular margin center based on the virtual object representing the articular margin. The humeral neck axis may be perpendicular to the virtual object representing the articular margin and is based on the articular surface of a humeral head and the articular margin center. The lesion line may be based on the articular margin center and the boundary of the lesion, and the boundary of the lesion may be further defined as a medial edge of the lesion.

The method of visualizing patient shoulder anatomy may include surgical planning, e.g. including surgical planning of soft tissue repair, bone grafting, capsular plication, capsular shift or joint replacement. The surgical plan, a proposal for surgical plan, or input data for a (proposal for a) surgical plan, may be generated in an automated fashion by an automated system. The method of visualizing patient shoulder anatomy may include displaying a surgical plan for a medical procedure or providing options and/or outcomes for various surgical planning purposes or displaying other features of the patient shoulder anatomy.

### BRIEF DESCRIPTION OF THE DRAWINGS

Advantages of the invention will be readily appreciated as the same becomes better understood by reference to the following detailed description when considered in connection with the accompanying drawings, wherein:
FIG. 1 is a perspective view of an exemplary layout of an operating room including a surgical planning system, according to one implementation.
FIG. 2 is a screenshot displaying renderings of 3D models of a patient glenoid and humerus that may be shown on a display of the surgical planning system according to one implementation.
FIG. 3A is a screenshot displaying a rendering of a 3D model of a patient humeral head including a glenoid track object that may be shown on the display according to one implementation.
FIG. 3B is a screenshot displaying a rendering of a 3D model of the patient humeral head of FIG. 3A including a healthy glenoid track object that may be shown on the display according to one implementation.
FIG. 4 is a screenshot displaying a rendering of a 3D model of the patient glenoid of FIG. 2 including a glenoid width that may be shown on the display according to one implementation.
FIGS. 5A and 5B show how a controller of the surgical planning system of FIG. 1 may be configured to determine the glenoid width of FIG. 4, according to one implementation.
FIG. 5C depicts a method of determining a plane of a best fit circle object, according to an implementation utilizing a glenoid point projection.
FIG. 5D depicts a method of determining a position of the best fit circle object, according to an implementation utilizing anchor point projection.
FIG. 5E depicts the method of determining the position of the best fit circle object, including shifting the best fit circle object to the projected anchor points.
FIG. 6 shows patient image data that includes rotator cuff muscles and will be used to describe how the controller is configured to apply a segmentation algorithm to the patient image data to determine a soft tissue insertion line, according to one implementation.
FIGS. 7 - 8B show patient image data that includes a humerus and will be used to describe how the controller of the surgical planning system of FIG. 1 may be configured to determine the soft tissue insertion line of FIG. 6, according to various implementations.
FIG. 9A is a perspective representation of a 3D model of a patient after a segmentation algorithm has been applied to patient image data to determine the presence and/or location of a lesion object on the patient humeral head of FIG. 3A, according to one implementation.
FIG. 9B is a perspective representation of a 3D model of a patient after one or more post-processing algorithms have been applied to the 3D model of FIG. 9A, according to one implementation.
FIG. 10A depicts a method of determining the presence and/or location of the lesion object of FIG. 9A, according to an implementation utilizing a distance map.
FIG. 10B depicts a method of depicting a lesion object in relation to image data that includes a humeral head portion according to one implementation.
FIGS. 11-13 depict methods of determining the presence and/or location of the lesion object of FIG. 9A, as well as how the lesion may be represented in a screenshot that may be shown on the display according to various implementations.
FIGS. 14A-14C are screenshots displaying renderings of 3D models of the patient humeral head of FIG. 3A, the lesion object of FIG. 9A, and the glenoid track object of FIG. 3B that may be shown on the display according to some implementations.
FIGS. 15A-15C are screenshots displaying renderings of 3D models of the patient humeral head of FIG. 3, the lesion object of FIG. 9A, and the healthy glenoid track object of FIG. 3A that may be shown on the display according to some implementations.
FIG. 16 shows a method of determining the impact of the lesion object of FIG. 9A on track engagement, according to one implementation.
FIG. 17A is a screenshot displaying a notification to communicate the impact of the lesion object of FIG. 9A on track engagement to a user that may be shown on the display according to one implementation.
FIG. 17B is a screenshot displaying a notification to communicate the recurrent dislocation risk based on the position of a lesion object to a user that may be shown on the display according to one implementation.
FIG. 17C is a screenshot displaying a notification to communicate the impact of the lesion object of FIG. 17B on track engagement to a user that may be shown on the display according to an alternative implementation.
FIG. 18 shows a method of determining a reconstruction dimension of a bone block implant, according to one implementation.
FIG. 19 is a screenshot displaying the bone block implant of FIG. 18 and renderings of the 3D models of the patient glenoid and humerus of FIG. 2 that may be shown on the display according to one implementation.
FIGS. 20-22 depict flowcharts illustrating steps of determining anatomical locations of interest and visualizing the anatomical locations of interest relative to a rendering of a 3D model of a patient shoulder anatomy, according to some implementations.
FIGS. 23 and 24 depict flowcharts illustrating methods of bone block implant planning, according to some implementations.
FIG. 25 shows one method of determining the location of one or more anatomical locations, according to one implementation.
FIGS. 26A and 26B are screenshots displaying renderings of 3D models including a set of planar lines relative to a lesion object that may be shown on the display according to some implementations.
FIGS. 27A and 27B are screenshots displaying renderings of 3D models including a set of clockface lines relative to a lesion object that may be shown on the display according to some implementations.
FIG. 28 is a screenshot displaying a rendering of a 3D model including a geodesic distance between the border of a lesion object and an anatomical location of interest that may be shown on the display according to one implementation.
FIG. 29 illustrates a method of determining a humerus neck axis, according to one implementation.
FIG. 30 illustrates a method of determining a lesion line, according to one implementation.
FIG. 31 is a screenshot displaying a rendering of a 3D model including a degree measurement of rotation of the humerus of FIG. 2 that may be shown on the display according to one implementation.
FIG. 32 is a flowchart illustrating a method of generating and visualizing sets of reference shapes relative to a lesion object and a 3D model of a patient shoulder anatomy, according to one implementation.
FIGS. 33-34 are flowcharts illustrating methods of generating and visualizing quantifiable measurements related to a lesion object and a 3D model of a patient shoulder anatomy, according to some implementations.
FIG. 35A is a screenshot displaying a rendering of a 3D model of a patient after a segmentation algorithm has been applied to patient image data to determine the presence and/or location of a lesion object on the patient humeral head, according to one implementation, with the lesion object being indicated has have a normal dimension in the top figure, a caution dimension in the middle figure, and a warning dimension in the bottom figure.
FIG. 35B is a screenshot displaying a notification regarding a dimension of the lesion object of FIG. 35A to a user that may be shown on the display according to an alternative implementation.
FIG. 36A and FIG. 36B show a method of comparing the location of Hill-Sachs lesion to certain reference planes, according to one implementation.
FIG. 36C shows a screenshot displaying renderings of 3D models including certain reference planes relative to a lesion object that may be shown on the display according to some implementations, the screenshot including an indicator of the lesion object having a normal location.
FIG. 36D shows a screenshot displaying renderings of 3D models including certain reference planes relative to a lesion object that may be shown on the display according to some implementations, the screenshot including an indicator of the lesion object having a caution location.
FIG. 36E shows a screenshot displaying renderings of 3D models including certain reference planes relative to a lesion object that may be shown on the display according to some implementations, the screenshot including an indicator of the lesion object having a warning location.
FIG. 36F is a screenshot displaying a notification to communicate the position of the lesion object of FIG. 36E to a user that may be shown on the display according to an alternative implementation.
FIG. 37A-C shows a screenshot displaying axial, coronal and sagittal 2D renderings of 3D models of a patient glenoid and humerus and outlines of renderings of a premorbid 3D model of a patient glenoid that may be shown on a display of the surgical planning system according to one implementation.
FIG. 37D shows a screenshot displaying renderings of 3D models of a patient glenoid and a rendering of a portion of a premorbid 3D model of a patient glenoid that may be shown on a display of the surgical planning system according to one implementation.
FIG. 38 is a screenshot displaying a graphical user interface and a rendering of a 3D model of a scapula and a humerus and a best fit circle object including various measurements anatomy that may be shown on the display according to one implementation.
FIG. 39A is a screenshot displaying a rendering of a 3D model of a patient glenoid, a rendering of a portion of a premorbid 3D model of a patient glenoid, and a best fit circle object that may be shown on a display of the surgical planning system according to one implementation.
FIG. 39B is a screenshot displaying a rendering of a 3D model of a patient glenoid, a rendering of a portion of a premorbid 3D model of a patient glenoid and a best fit circle object that may be shown on a display of the surgical planning system according to one implementation, with the 3D model of the patient glenoid being partially obscured by portions of the premorbid 3D model.
FIG. 39C is a screenshot displaying a rendering of a 3D model of a patient glenoid, a rendering of a portion of a premorbid 3D model of a patient glenoid and a best fit circle object that may be shown on a display of the surgical planning system according to one implementation, with the bone loss edge of the best fit circle object being shown in a first position.
FIG. 39D is a screenshot displaying a rendering of a 3D model of a patient glenoid, a rendering of a portion of a premorbid 3D model of a patient glenoid and a best fit circle object that may be shown on a display of the surgical planning system according to one implementation, with the bone loss edge of the best fit circle object being shown in a second position, different from the position in FIG. 39C.
FIG. 39E is a screenshot displaying a rendering of a 3D model of a patient glenoid, a rendering of a portion of a premorbid 3D model of a patient glenoid and a best fit circle object that may be shown on a display of the surgical planning system according to one implementation, with the bone loss edge of the best fit circle object being shown in a first orientation.
FIG. 39F is a screenshot displaying a rendering of 3D model of a patient glenoid, a rendering of a portion of a premorbid 3D model of a patient glenoid and a best fit circle object that may be shown on a display of the surgical planning system according to one implementation, with the bone loss edge of the best fit circle object being shown in a second orientation, different from the orientation of FIG. 39E.
FIG. 39G is a screenshot displaying a rendering of 3D model of a patient glenoid, a rendering of a portion of a premorbid 3D model of a patient glenoid and a best fit circle object that may be shown on a display of the surgical planning system according to one implementation, with the best fit circle object shown having a first circle center.
FIG. 39H is a screenshot displaying a rendering of 3D model of a patient glenoid, a rendering of a portion of a premorbid 3D model of a patient glenoid and a best fit circle object that may be shown on a display of the surgical planning system according to one implementation, the best fit circle object shown having a second circle center, different from the circle center shown in FIG. 39G.
FIG. 40A depicts a humerus including a plurality of radiopaque beads.
FIG. 40B depicts a chart that includes the clockface position of the plurality of radiopaque beads of FIG. 40A for a plurality of non-patient persons against the percentage of humeral width, including an average line.
FIG. 40C depicts a representative humerus with the various clockface positions of FIG. 40B.

### DETAILED DESCRIPTION OF THE INVENTION

### I. Example System Overview

Referring to the Figures wherein like numerals indicate like or corresponding parts throughout the several views, a surgical planning system 100 including a controller 110 and methods for using the same are shown throughout. It is to be understood that some Figures may represent a portion and/or the entirety of a graphical user interface, and the presented views do not limit the graphical user interface to any configuration shown herein. The graphical user interface may include any combination of the Figures and those not specifically shown may still be utilized by the surgical planning system 100.

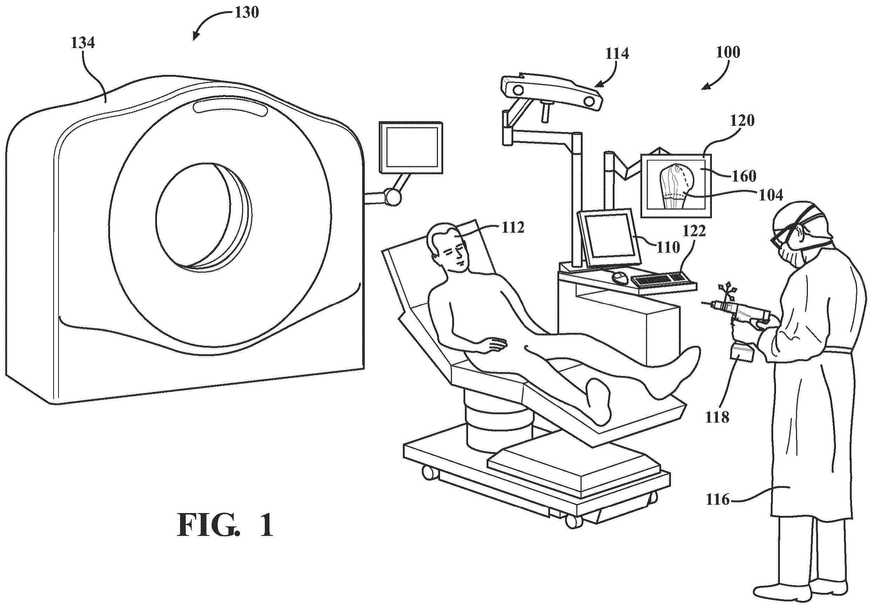

FIG. 1 is a perspective view of the surgical planning system 100 configured to assist a user 116 in visualizing patient shoulder anatomy and/or planning a prospective surgery on a patient 112. The surgical planning system 100 may include a tool 118, a display 120, an imaging system 130, a user input device 122, and a localizer 114. The controller 110 may be configured to communicate with the imaging system 130, the display 120, the user input device 122, and/or the localizer 114. For example, there may be a wired or wireless connection between the controller 110, an imaging system, such as a CT scanner 130, the localizer 114, the user input device 122, and the display 120. The controller 110 may facilitate communication between the various elements 130, 114, 122, 120 of the system 100. The controller 110 may be part of a personal computer, laptop computer, tablet computer, other suitable computing device, or the plurality of suitable computing devices. In some implementations, the controller 110 may be integrated with the display 120 or the imaging system 130, or it may be implemented by multiple elements of the surgical planning system 100. The controller 110 may be configured to receive, send, or process data between the display 120 and the imaging system 130, or other computing devices. Further, the controller 110 may be configured to receive input from the user input device 122 to run software, send data, receive data, or manipulate the data shown on the display 120.

In some implementations, the localizer 114 is an optical localizer and includes a camera unit with an outer casing that houses one or more optical sensors configured to sense movement of the various trackers. To this end, any one or more of the trackers may include active markers (not shown in detail). The active markers may include light emitting diodes (LEDs). Alternatively, the trackers may have passive markers, such as reflectors which reflect light emitted from the camera unit or another predetermined light source. In other implementations, the localizer 114 may be electromagnetically (EM) based. For example, the navigation system may include an EM transceiver coupled to a navigation controller or controller 110. The localizer 114 may also be radio frequency (RF) based. In such a case, the localizer 114 may include an RF transceiver coupled to the navigation controller and/or to the controller 110. Here, the trackers may include RF emitters or transponders, which may be passive or may be actively energized. The RF transceiver transmits an RF tracking signal, and the RF emitters respond with RF signals such that tracked states are communicated to (or interpreted by) the navigation controller. The navigation controller can determine location of the RF tracker by virtue of the distance of the RF emitter or transponder relative to the RF transceiver and/or the angle (direction) of the RF emitter or transponder relative to the RF transceiver.

The display 120 may be a monitor, the screen of a laptop computer, or an extended reality display device. For example, the display 120 may be a headset configured to be worn by the user 116. In some implementations, the display 120 may be configured as an extended reality device configured to execute any of the graphical functions described herein. The extended reality device may be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user 116. The real-world views may be acquired directly by the eyes of the user 116 or may be a real-world video stream captured by one or more cameras of the extended reality device. When a head mounted device is utilized, the head-mounted device may include a transparent lens or one or more display screens positioned directly in front of the eyes of the user 116 to display the computer-generated graphics relative to the real-world views.

The controller 110 may be configured to cause the display 120 to display various screens for assisting the user 116 in visualizing patient shoulder anatomy and/or planning a prospective surgery on a patient 112 as described herein. In some implementations, the controller 110 may be configured to display a graphical user interface (GUI) 160 including interactable elements such as check boxes, buttons, sliders, drop lists, etc. on the display 120. According to some aspects, the GUI 160 may allow the user to further control the position/orientation of renderings and/or models displayed on the display 120.

The user input device 122 may be engaged by the user 116 to provide input to the controller 110 and/or manipulate objects shown on the display 120, such as interactive elements of the GUI 160, for example by clicking, dragging, scrolling, typing, or any combination of these actions to provide input to the controller 110. In some implementations, the user input device 122 may be a keyboard, mouse, touch pen, track ball, a microphone, a navigated instrument, or a combination thereof. Additionally or alternatively, the user input device 122 may be implemented as a touch screen, such as integrated with the display 120, that allows the user 116 to directly interact with objects shown on the display, such as interactive elements of the GUI 160, via touch inputs/gestures through the touch screen.

The imaging system 130 may include an imaging device, such as a CT machine 134, an MRI machine, an X-ray machine, or any other type of intra-operative and/or pre-operative imaging device, along with a display and computer. Depending on the implementation, the imaging device may generate different types of image data, such as a fluoroscopy image, an X-ray image, an MRI image, or a CT image. For example, if the imaging device is a CT machine 134, the imaging system 130 may generate CT image data. In some implementations, the imaging device may be further configured to obtain 3D models of the anatomy being imaged, for example, the patient shoulder anatomy. The imaging system 130 may be configured to generate one or more types of imaging data or combine types of imaging data. The imaging system 130 may transmit the image data obtained by the imaging device to the controller 110 to be processed, displayed, or otherwise manipulated by the controller 110.

The controller 110 may utilize a segmentation algorithm, such as a deep learning model, to generate the 3D models of patient shoulder anatomy from the image data. The controller 110 may be configured to use the algorithm to generate a two-dimensional and/or three-dimensional model including at least a portion of patient shoulder anatomy from the image data. The controller 110 may be configured to display the 3D models generated by the algorithm on the display 120. The 3D model may further include indicators corresponding to anatomical locations, or virtual objects representing anatomical landmarks, or other modifications not specifically described herein. Further, the controller 110 may be configured to utilize segmentation to facilitate alert zone planning, desired tool boundaries, implant positions and/or orientation, and other features of surgical navigation and preoperative planning.

Additionally, the user may provide input to the controller 110 using the user input device 122 to manually or semi-automatically edit the 3D model generated by the algorithm. Exemplary manual and semi-automatic segmentation tools are described in U.S. Patent Publication No. 2019/0340765, which is hereby incorporated by reference. Alternatively, the controller 110 may receive the image data along with an output of a segmentation algorithm (the 3D models) directly from the imaging system 130.

As previously mentioned, the controller 110 may be configured to cause the display 120 to show a GUI 160, patient image data, or combinations thereof. Patient image data (e.g., pre-operative patient images or intraoperative patient images) and/or renderings of 3D models of the patient shoulder anatomy may be displayed within dedicated windows of the GUI 160. The display 120 may be configured for the user 116 to interact with objects displayed by the GUI 160 (such as renderings of 3D models) via the user input device 122. Further, the controller 110 may be configured to cause the display 120 to show representations of anatomical landmarks of the patient shoulder anatomy, measurement values corresponding to the patient shoulder anatomy, and/or representations of reconstructions of the patient shoulder anatomy. In addition, the controller 110 may also be configured to simulate motion of one or more components of the patient shoulder anatomy on the display 120. The controller 110 may be configured to cause the display 120 to show a surgical plan for a medical procedure or provide options and outcomes for various surgical planning purposes or show other features of the patient shoulder anatomy. Other features of the visualizing patient shoulder anatomy may exist, and those not specifically discussed herein may also be shown on the display 120.

In some implementations, the GUI 160 may include a three-dimensional (3-D) view window, a view options window, a patient information window, an implant family window, a workflow-specific tasks window, and an alignment measures window. The 3-D view window allows the user to view and interact with medical imaging data, 3-D bone models, and 3-D implant component CAD models. The view options window provides widgets to allow the user to quickly change the view of the models of the bone, models of the implant components, and alignment axes, to a desired view. The patient information window displays the patient's information such as name, identification number, gender, surgical procedure, and operating side (e.g., left humerus, right humerus). The implant family window provides drop-down menus to allow the user to select and re-select a desired implant component from a library of implant components. The workflow-specific tasks window includes various widgets to provide several functions illustratively including: guiding the user throughout different stages of the planning procedure; allowing the user to select and re-select desired alignment goals from a set of alignment goals; allowing a user to adjust one or more components that impact measurement(s); allowing the user to adjust the implant component(s) and bone models in desired clinical directions; displaying measured values of the alignment and position of the component(s) on the bone(s); and displaying a summary of the plan. The window may display the alignment and position of the implant components on the bone models such as the humerus - glenoid angle, humeral neck axis alignment, and projected reconstruction / bone block alignment. Overall, the layout of the GUI 160 provides the user with a convenient roadmap and visual display to successfully plan a shoulder arthroplasty procedure, bone graft, or lesion repair.

The GUI 160 may include one or more configurations, each of which may include objects for displaying and/or interacting with the views shown in the Figures. As mentioned above, the GUI 160 may be configured to allow the user 116 to manipulate the information shown on the display 120. For example, the user 116 may interact with the GUI 160 to view one or more renderings of the 3D models of patient shoulder anatomy by rotating, switching views, or otherwise manipulating the renderings of the 3D models. The user 116 may interact with the GUI 160 to view the patient image data from the imaging system 130. The GUI 160 may be configured to prompt the user 116 to enter information or generate surgical plans in accordance with instructions entered by the user 116. The user 116 may use the GUI 160 and/or the user input device 122 to input patient data or modify surgical plans. The patient data, in addition to the patient images, may include additional information related to the type of surgical procedures being planned, the patient's anatomical features, the patient's specific medical condition, and/or operating settings for the surgical procedures. The user 116 may mark locations of interest on the 3D models displayed by display 120 using the GUI 160. For example, the GUI 160 may include buttons to hide and/or show portions of the renderings of the 3D models, measurements such as distance between locations of interest, or other aspects of the 3D models. Utilizing the user input device 122, the user 116 may click to drag the image on the display 120 to rotate the view of the rendered 3D model, translate the view of the model, pan and/or zoom, or otherwise manipulate the information shown on the display 120 in any suitable way. The controller 110 may record the actions of the user 116 to facilitate future surgical planning, or it may record desired views, slices, or other manipulations of the renderings of the 3D model. For example, the user 116 may adjust parameters of a surgical plan for shoulder surgery (such as making modifications to an automatic and/or pre-set plan). These adjustments may be recorded by the controller 110 for future reference, and to facilitate surgical planning. Additionally, the user 116 may select a button contained within the GUI 160 to record a particular perspective of the rendered 3D model on the display 120. After further manipulation of the rendered 3D model, the user 116 may refer back to the recorded perspective to facilitate surgical planning. The user 116 may input various anatomical dimensions related to the patient shoulder anatomy, such as the size and shape of a humerus 144 and other anatomical structures of the patient shoulder anatomy. The user input device 122 and/or the GUI 160 may also be configured to allow the user 116 to select, edit, or manipulate the patient data. For example, the user 116 may identify and/or select anatomical features from the patient data via the user input device 122 (clicking with a mouse, touch input, dragging to select, etc.).

### II. Surgical Planning System

Prospective surgical planning on patient shoulder anatomy may depend upon the state of the patient shoulder anatomy. For example, in healthy shoulder anatomy, a humerus is functionally connected to a glenoid via soft tissue, which includes the muscles and tendons of a rotator cuff. However, in injured shoulder anatomy, the presence and/or location of a lesion may interfere with the functional connection. More specifically, the presence and/or location of a lesion may influence the engagement between a glenoid and a humerus, which may increase the risk of additional shoulder injuries.

The surgical planning system 100 may be used to plan shoulder surgery, such as by analyzing 3D models representative of at least a portion of the patient shoulder anatomy. The analysis may include determining and visualizing specific anatomical features on the patient shoulder anatomy, which may include a glenoid, a humerus, and/or other anatomical locations not disclosed herein. The analysis may further include determining and visualizing the presence and/or location of a lesion relative to the patient shoulder anatomy. For example, in the illustrated implementation, the surgical planning system 100 may be used to identify a lesion object 210 on a humeral head portion 146, also known as a Hills-Sachs lesion. Although not shown, there may also be a lesion on a glenoid, which may be known as a Bankart lesion. As described above, the 3D models may be generated automatically with a segmentation algorithm and/or a deep learning algorithm. The controller 110 of the surgical planning system 100 may be configured to automatically identify the anatomical locations of interest, determine the impact of a lesion to the patient shoulder anatomy, and display the anatomical locations of interest (and/or the lesion) relative to the 3D models. The impact on the patient shoulder anatomy resulting from the lesion may be understood as the risk of subsequent shoulder injury, and/or a change to the interaction between a glenoid and a humerus. For example, the impact on patient shoulder anatomy from a shoulder injury (e.g., a lesion) may be that there is reduced contact area between a glenoid and a humerus due to the lesion, which may lead to a higher risk of subsequent injury. The controller 110 may be configured to present information to the user 116 via the display 120 relevant to the state of patient shoulder anatomy to facilitate future surgical planning.

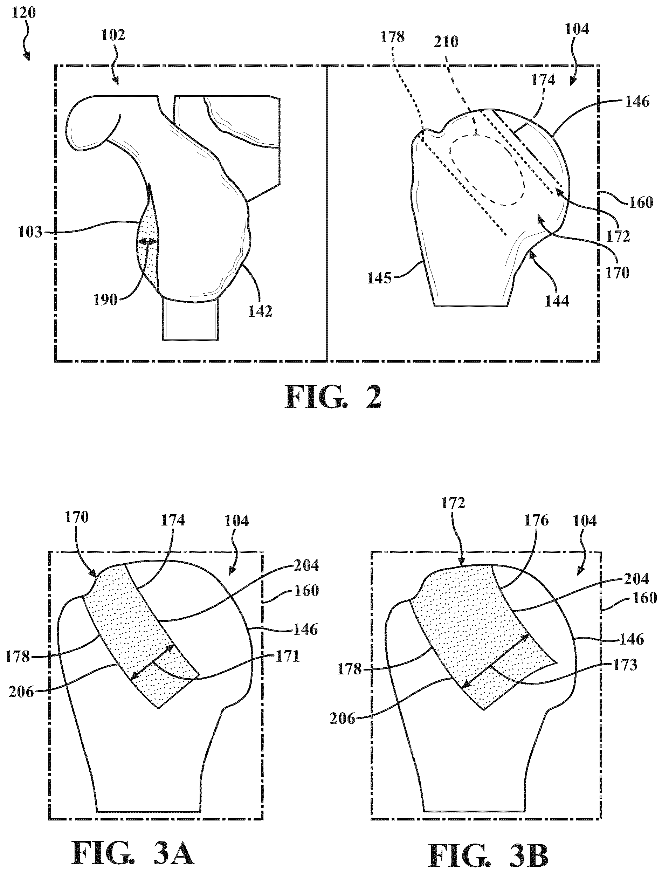

In order to facilitate surgical planning, the controller 110 may be configured to generate a glenoid 3D model 102, a humerus 3D model 104, and/or 3D models of other patient anatomy from image data. Further, the controller 110 may cause the display 120 to show representations of the patient shoulder anatomy on the display 120 (e.g. the glenoid 3D model 102, the humerus 3D model 104, etc.) and may further generate and/or cause the display 120 to show virtual objects, such as a glenoid track object 170 and a lesion object 210 relative to the 3D models on the display 120. FIG. 2 illustrates a screenshot displaying such objects that may be shown on the display 120 according to one example. The virtual objects (which may be referred to as "objects" or otherwise will be identified in this description) may be based on the image data of patient shoulder anatomy and may include an area, a volume, a point, a line, a plane, or any other shape not disclosed herein to represent anatomical aspects of patient shoulder anatomy (such as a glenoid and/or a healthy patient glenoid). Additionally, the controller 110 may identify portions of the glenoid 3D model 102 and the humerus 3D model 104 that correspond to real portions of patient shoulder anatomy. For example, referring to FIG. 2, a humeral neck portion 145 and a humeral head portion 146 of the humerus 3D model 104 may be used as relevant locations to overlay and display one or more virtual objects relative to the 3D models. It should be appreciated that the term 'screenshot' is used herein to refer to either a portion or the entirety of the information presented on the display 120 at a given time. For example, a screenshot could refer to the contents of a single section of the information presented on the display 120, or to the contents of all sections of the information presented on the display 120 in implementations where multiple sections of information are presented simultaneously.

The controller 110 may be configured to automatically identify the presence and/or location of a lesion on the patient shoulder anatomy prior to, concurrently, or after the generation of the 3D models. Based on the presence and/or location of the lesion, the controller 110 may be configured to generate a lesion object 210 and cause the display 120 to show the lesion object 210 relative to one or more 3D models, such as the humerus 3D model 104. The lesion object 210 may include a representation of a lesion present on the shoulder anatomy 142, such as a Hills-Sachs lesion. Advantageously, the automation of these surgical planning processes saves the surgeon time and effort and improves the accuracy and reliability of the surgical planning process.

### III. Segmentation Algorithm

One exemplary way of automated segmentation is described in U.S. Patent No. 8,971,606, entitled, "Method for automatically identifying the contours of a predefined bone, derived methods and corresponding computer program products", the disclosure of which is hereby incorporated by reference. There may be various other ways in which to perform automated segmentation, and the techniques are not limited to automated segmentation using techniques described in U.S. Patent No. 8,971,606. As one example, segmentation of the CT image data to yield segmented objects includes comparisons of voxel intensity in the image data to determine bony anatomy and comparisons to estimated sizes of bony anatomy to determine a segmented object. Moreover, as described above, the example techniques may be performed with non-automated segmentation techniques, where a medical professional evaluates the image data to segment anatomical objects, or some combination of automation and user input for segmenting anatomical objects. A computing device, such as the controller 110, may generate segmented image data of the patient anatomy in order to create anatomical objects. It should be appreciated that the controller 110 may receive the segmented image data from other computing devices, such as the imaging system 130.

In one or more examples, the controller 110 may utilize image data to compare (e.g., size, shape, orientation, etc.) against a fitted statistical shape model (SSM) as a way to determine characteristics of the patient anatomy prior to the patient suffering the injury or disease. In some examples, the controller 110 may compare 3D point data of non-pathological points of anatomical objects of patient anatomy in the image data to points in the SSM. With reference to Figure 2, the controller 110 may deploy a statistical shape model to generate a predicted surface 103 of a healthy glenoid. More specifically, the controller 110 may be configured to fit a statistical shape model to the image data of patient shoulder anatomy to generate a premorbid prediction of the surface of the glenoid, i.e., a prediction of the surface of the glenoid before the occurrence of a pathology. The statistical shape model may be created based on defining principal modes of variation based on healthy shoulder data sets.

Other methods of segmentation are described in International Patent Publication No. 2020205248, entitled "Pre-morbid characterization of anatomical object using statistical shape modeling(ssm)" and/or U.S. Patent Application Ser. No. 17/607323, entitled "Automated planning of shoulder stability enhancement surgeries", the disclosures of both of which are hereby incorporated by reference in their entirety.

### IV. Identification of Glenoid Track

In patient shoulder anatomy, a glenoid interacts with a humeral head to provide the range of motion integral to a glenohumeral joint. In a glenohumeral joint of a healthy patient, the humeral head rotates freely within the glenoid. The area on a humeral head portion 146 of a humerus 3D model 104 that interacts with a glenoid 3D model 102 may be identified as the glenoid track object 170. The glenoid track object 170 includes the surface of the humeral head portion 146 which contacts the glenoid 3D model 102 as the humeral head portion 146 rotates and may also represent the engagement between a glenoid and a humerus. As shown in FIG. 3A, the controller 110 may be configured to generate the glenoid track object 170 based on a glenoid track width 171. Referring to FIG. 3B, the controller 110 may be configured to generate a healthy glenoid track object 172 to represent the surface area of humeral head that contacts a glenoid of a healthy patient shoulder anatomy and includes a healthy glenoid track width 173. The healthy glenoid track object 172 may be referred to as the reconstructed or restored glenoid track 172. In an injured patient, the glenoid track width 171 may be less than the healthy glenoid track width 173, which increases the likelihood of disrupted track engagement. The controller 110 may be configured to cause the display 120 to visualize any of the above anatomical locations and/or representations of anatomical features of patient shoulder anatomy and/or the healthy patient shoulder anatomy. For example, the controller 110 may be configured to cause the display 120 to visualize any of the above anatomical locations and/or representations of the glenoid track object 170, the healthy glenoid track object 172, the lesion object 210, and other objects described throughout. To accurately assess the state of the patient shoulder anatomy, the user 116 may compare the patient shoulder anatomy to a projected healthy shoulder anatomy. As shown in FIGS. 3A and 3B, to automate and increase the accuracy of the aforementioned comparison, the controller 110 may be configured to compare the healthy glenoid track width 173 to the glenoid track width 171 and provide an indication that the glenoid track is reduced.

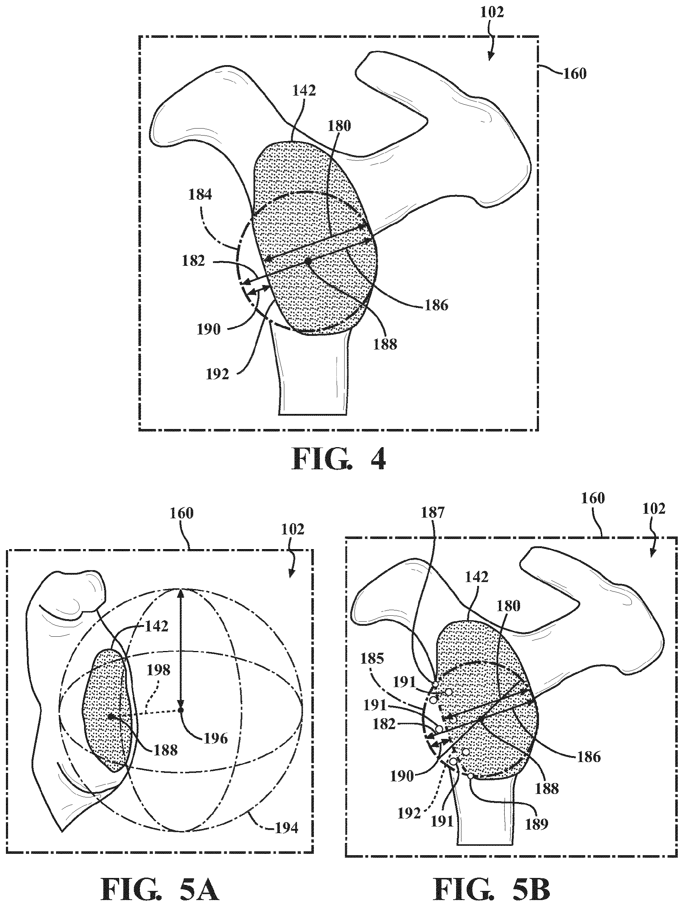

The controller 110 may be configured to model a healthy patient glenoid, absent of any anatomical anomalies. The healthy patient glenoid may also be referred to as a reconstructed patient glenoid, a healthy glenoid, or a premorbid patient glenoid. Now referring to FIG. 4, the healthy patient glenoid may be modeled by a best fit circle object 184. The best fit circle object 184 may be manually generated by the user 116 or automatically generated by the controller 110 (e.g., as described below). The best fit circle object 184 is fit to the posterior and inferior parts of a glenoid to best represent the healthy patient glenoid. Additionally, or alternatively, the controller 110 may be configured to determine the healthy patient glenoid by referencing a contralateral glenoid. The contralateral glenoid refers to the glenoid on the opposite side of the body of the patient 112, which may be uninjured and anatomically similar to the healthy patient glenoid. The contralateral glenoid may be used to project a healthy patient glenoid on a glenoid. For example, the imaging system 130 may be configured to obtain imaging data of the contralateral glenoid of the patient 112, and transmit the imaging data to the controller 110, which may cause the display 120 to show a 3D model of the contralateral glenoid. The 3D model of the contralateral glenoid may be mirrored before overlaying the contralateral glenoid model onto the glenoid 3D model 102, allowing for a more accurate approximation of the size and shape of the healthy patient glenoid. The width and/or shape of the contralateral glenoid may be measured by the user 116 by interacting with a displayed measuring tool such a digital ruler using the user input device 122 and/or automatically determined by the controller 110 and utilized to model the healthy patient glenoid. The controller 110 may then cause the display 120 to show the 3D model of a glenoid and/or the model of the healthy patient glenoid on the display 120.

As shown in FIG. 4, after generating a model of the healthy glenoid such as the best fit circle object 184, the controller 110 may determine a glenoid width 180 and/or a healthy glenoid width 182. The glenoid width 180 may be determined from the 3D model with a measuring tool, such as a digital ruler, or determined automatically by the controller 110 based on the 3D model. The healthy glenoid width 182 may be similarly determined from the 3D model with the measuring tool. The healthy glenoid width 182 may also be determined by measuring the diameter of the best fit circle object 184. Additionally or alternatively, the controller 110 may allow the user 116 to input a request via the user input device 122 to automatically measure the glenoid width 180 and/or the healthy glenoid width 182 and display the result.

After determining the glenoid width 180 and/or the healthy glenoid width 182, the controller 110 may determine a glenoid bone loss measure and the glenoid track width 171. The glenoid bone loss measure may be representative of glenoid bone loss, and may include a width, ratio, volume, or other quantitative value representative of the bone loss. In some examples, the glenoid bone loss measure may be a glenoid bone loss width 190, which may be equal to the difference between the glenoid width 180 and the healthy glenoid width 182. Still referring to FIG. 4, the controller 110 may thus be configured to determine the glenoid bone loss measure by subtracting the glenoid width 180 from the healthy glenoid width 182. The glenoid track width 171 may be calculated for the glenoid track object 170 and the healthy glenoid track width 173 may be calculated for the healthy glenoid track object 172. The healthy glenoid track object 172 may be determined by multiplying the healthy glenoid width 182 by a scaling factor. The scaling factor is based on anatomical characteristics, such as soft tissue laxity. The glenoid track width 171 may be a certain percentage of the glenoid width 180. In some implementations, this threshold value is at least 75%, 80%, or 85%. In one example, the threshold value may be 83% of the glenoid width.

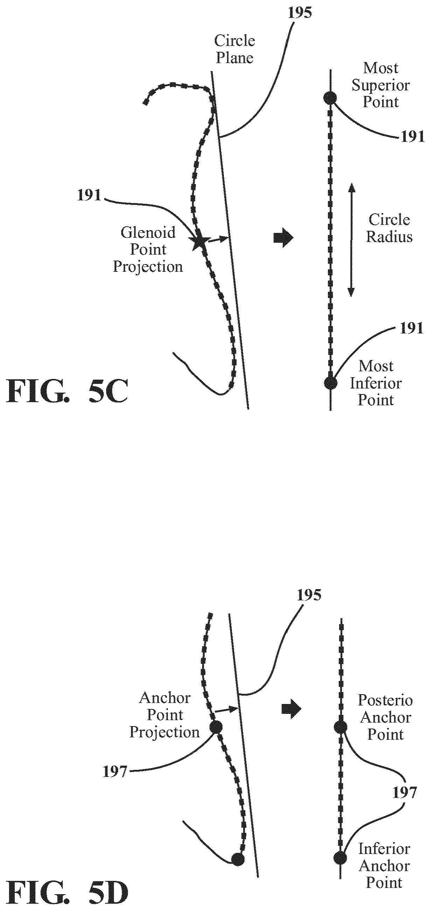

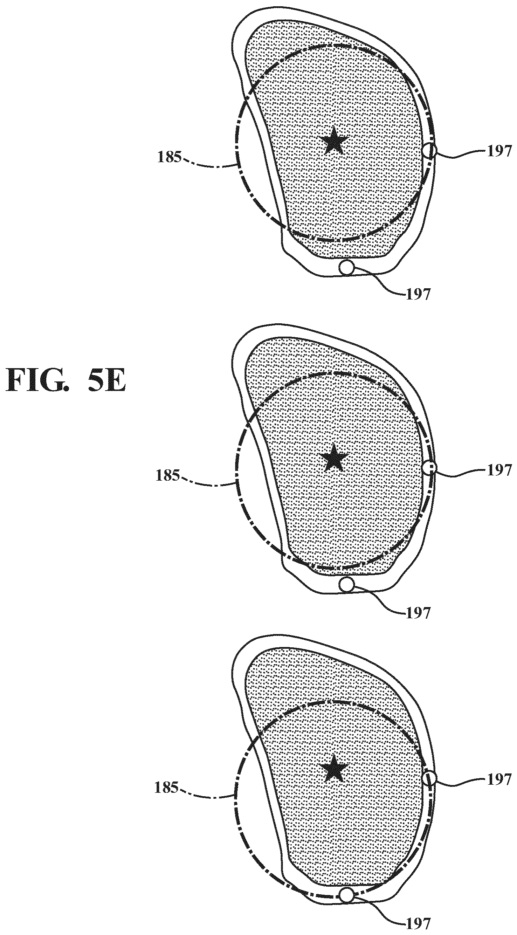

The glenoid bone loss measure may also be determined by fitting a spherical object 194 or another geometrical primitive to the glenoid 3D model 102. As shown in FIG. 5A, the spherical object 194 may be visualized relative to the glenoid 3D model 102. The spherical object 194 may approximate the articular surface of the glenoid 3D model. The controller 110 may be configured to determine a center 196 of the spherical object 194 and/or a glenoid center 188. The controller 110 may be further configured to determine a circle diameter 186 of a circle 185 based on the radius of the spherical object 194 and/or a factor. The factor may be a standard anatomical value and/or relationship, such as the relationship between the glenoid width 180 and the height of a glenoid. The controller 110 may be configured to fit the spherical object 194 to the glenoid 3D model by taking one or more of these factors into account. The controller 110 may be configured to utilize one or more of these factors to automatically determine the glenoid width 180. Many other anatomical features may influence the position and/or size of the spherical object 194, and therefore impact the circle diameter 186, and those not specifically described herein may be utilized. The controller 110 may further generate a line 198 between the glenoid center 188 and the center 196 of the spherical object 194.

The controller 110 may generate the circle 185 perpendicular to the line 198 and positioned at an inferior rim of the glenoid 3D model 102. Now referring to FIG. 5B, the circle 185 may include a portion of the edge of the glenoid 3D model 102, delineated by the intersections between the circle 185 and the edge of the glenoid 3D model 102. These intersections are shown in FIG. 5B as a first intersection object 187 and second intersection object 189, and the portion of the boundary of the glenoid between the first and second intersection objects 187, 189 may be defined as a bone loss edge 192. The first and second intersection objects 187, 189 may be determined in various manners. In one exemplary manner, the first intersection object is based on a point between the most inferior point of the glenoid area points and the most inferior point of the surface of the glenoid 3D model 102. Ray tracing may be utilized to determine the coordinates of these points. The controller 110 may be configured to determine the glenoid bone loss width 190 by measuring the maximum distance between the bone loss edge 192 and the edge of the circle 185. Additionally or alternatively, the controller 110 may be configured to determine the glenoid bone loss measure as the glenoid bone loss width 190 divided by the diameter 186 of the circle 185. To calculate the glenoid track width 171, the controller 110 may subtract the glenoid bone loss measure from the healthy glenoid track width 173. Alternatively, the glenoid track width may be calculated by subtracting the glenoid bone loss measure from the threshold value multiplied by the healthy glenoid width.

Referring again to FIG. 5B, in another configuration, the bone loss edge 192 may defined as a best fit line through the anterior glenoid rim points 191 bound by the circle object 185.

It should be appreciated that the circle object 185 can be fit in various ways, including in one non-limiting implementation, such as by projecting points 191 of a glenoid on a circle plane to define a size of the circle, and the position of the circle object being defined by anchor points projected on the circle plane based on the segmentation of the glenoid to ensure optimal representation of the patient's anatomy.

More specifically, in one implementation, a scapular mesh of the patient's image data is segmented by labeling vertices according to their anatomical region. These regions include the glenoid face, acromion, coracoid, and remaining scapular surface. Vertex labeling may be performed using a fast marching algorithm, where seed points are initialized within each anatomical region. Boundaries between regions are inferred based on geometric features such as sharp curvature transitions, which often correspond to anatomical separations.

For the glenoid region, additional points may be sampled to enhance mesh resolution, with the number of points scaled according to the size of the glenoid surface. A best-fit sphere may be computed for the glenoid surface by generating a glenoid sphere (see description of spherical object 194 above) using these glenoid points 191 to approximate the articular surface curvature. The "en face" orientation of the glenoid may be defined by a vector connecting the centroid of the glenoid point cloud to the center of the best-fit sphere.

To further refine the glenoid representation, a glenoid mesh may be generated via a Boolean intersection between the scapular mesh and the best-fit sphere, which is translated medially by 5-10 mm. This medial shift ensures that the intersected region captures the relevant anatomical surface while excluding extraneous scapular geometry.

All glenoid points 191 are then projected onto a circular plane 195 that is perpendicular to the en face orientation. This circular plane 195 is positioned just lateral to the most lateral point of the glenoid. The radius of the circular plane 195 is determined by the vertical height difference between the most superior and most inferior glenoid points 191, scaled by a factor, such as 0.39 or 0.4, to create the best fit circle object 185. This projection facilitates standardized visualization and analysis of glenoid morphology in a normalized coordinate system.

The position of the best fit circle object 185 on the circular plane 195 is defined using posterior and inferior anchor points 197, which are derived as follows. The posterior anchor point 197 is calculated as a weighted position between the most posterior/inferior point on the glenoid mesh and the most posterior/inferior point of the glenoid point cloud. This posterior anchor point 197 is then projected onto the circular plane.

Initially, the best fit circle object 185 may be centered at the centroid of the projected glenoid points 191. The best fit circle object 185 may then be translated posteriorly to align with the posterior anchor point 197, and subsequently shifted inferiorly to align with the inferior anchor point 197. This method of fitting the best fit circle object 185 ensures consistent and anatomically meaningful placement of the best fit circle object 185, facilitating standardized analysis and visualization of glenoid morphology.

As described above, the user can adjust aspects of the best fit circle object 185 to best fit the patient's anatomy. By selecting the "pen" button next to circle adjustments, the user can change the various handles and interact with the best fit circle object 185 to alter the resultant measurements derived from the best fit circle object 185. It should be appreciated that in some implementations, it is possible to change the orientation of the glenoid while making adjustments, but the best fit circle object 185 disappears when the scapula is rotated more than 10° off the en face orientation.

It should be appreciated that the measurements in the bottom window (glenoid bone loss, glenoid width, and missing bone width) are automatically updated as the user adjusts various aspects of the best fit circle object 185, including, but not limited to, the circle center, the circle diameter, the orientation of the bone loss edge 192, and the position of the bone loss edge 192. A bone loss orientation should be understood to be the line perpendicular to the bone loss edge line and passing through the center of the circle.

It is also possible to toggle on/off a curvature heatmap of the scapula while making adjustments to aspects of the best fit circle object 185. The curvature heatmap of the scapula may provide a heatmap of the area of greatest curvature of the glenoid 3D model 102, such as portions of the glenoid 3D model 102 having the highest average curvature value. By providing this curvature heatmap, the user is better able to understand and appreciate the bone edge and/or irregularities of the patient bone.

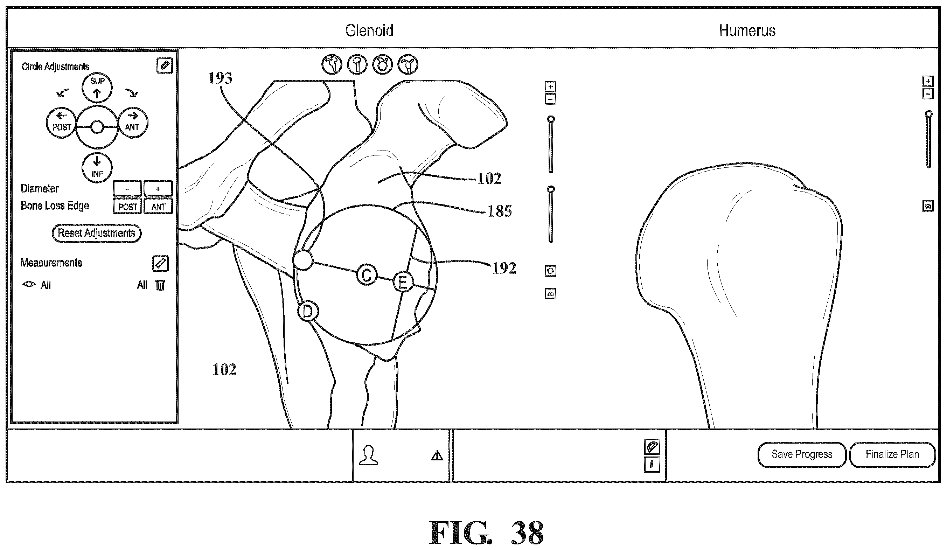

With reference to FIG. 38, the user can change the center of the circle 185 by clicking on the circle center "C", moving the circle to another position, and clicking another time to lock into place. Alternatively, the arrows in the side menu can be clicked to position the circle more superior (SUP), inferior (INF), anterior (ANT), or posterior (POST) in small increments. The center position "C" is constrained within the glenoid object.

The diameter of the circle object 185 can be changed by clicking on the circle handle "D", moving outwards to increase and inwards to decrease the diameter, and clicking another time to lock into place. Alternatively, the plus and minus buttons in the side menu can be used to change the circle diameter. The system may employ constraints on minimal and maximal diameter relative to aspects of the patient's anatomy.

The orientation of the bone loss edge 192 can be changed by in the user clicking on the button 193 with the arrow, sliding the orientation along the circle, and clicking another time to lock the orientation into place. Alternatively, the arrows in the side menu can be clicked to position the bone loss edge more horizontally or vertically. The system may employ constraints on maximal rotation upwards and downwards.

The possition of the bone loss edge 192 can be changed by clicking on the circle handle "E", moving posterior to increase and anterior to decrease the glenoid bone loss measurement, and clicking another time to lock into place. Alternatively, the posterior (POST) and anterior (ANT) buttons in the side menu can be used to change the bone loss edge. (the position of the bone loss edge is constrained between the circle center and the circle radius of the circle 185.

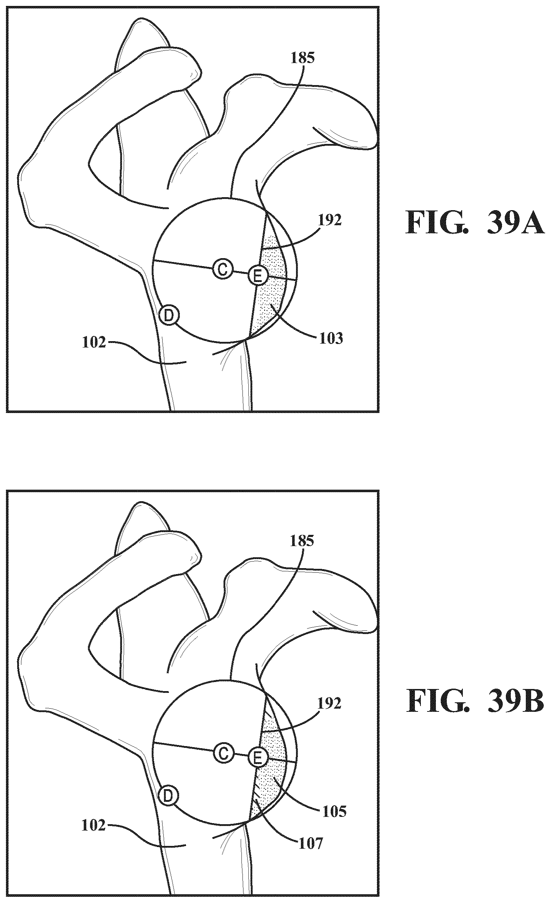

With respect to FIG. 39A-39H, the controller 110 may allow to view a predicted surface 103 of a healthy glenoid superimposed on the glenoid 3D model 102 while making adjustments to aspects of the best fit circle object 185.

Referring to FIG. 39A-39B, the controller 110 provides for a view of the premorbid glenoid model shown superimposed on the glenoid 3D model 102. The controller 110 may be configured to truncate portions of the premorbid glenoid model 103 such that only the area of the anterior glenoid from the premorbid model 103 is rendered with the glenoid 3D model 102. The truncation may be based on a glenoid surface patch of the underlying statistical shape model (numbered vertices) used to create the premorbid glenoid 3D model 103 and/or the position and orientation of the bone loss edge 192. If this occurs, then only portions of the premorbid glenoid model that are part of the glenoid surface and that are outside of the bone loss edge 192 are rendered. For clarity, it should be appreciated that when the statistical shape model gets fit to the patient specific morphology, it will be knowable which triangle representing patient bone is within the glenoid surface patch.

The portion of the premorbid bone 3D model is shown by area 103 generally (Figure 39A). With reference to FIG. 39B, the surface 103 may be more generally referred to as surfaces 105 and surfaces 107, shown with different hashing patterns. If the glenoid 3D model 102 is rendered transparently, additional information regarding the premorbid bone model can be appreciated. For example, if the surface of the premorbid bone is 'inside' of the patient's bone surface, it can be rendered in a first color, pattern, transparency or shading, and if the surface prediction of the surface of the premorbid bone is 'outside' of the patient's bone surface, it can be rendered in a second color, pattern, transparency, or shading, different from the first. The user can select the opacity of bone the glenoid 3D model and/or the opacity of the premorbid bone. As depicted in Figures 39A-39H, the surfaces 105 represent surfaces of the premorbid bone model that are 'outside' of the patient's bone surface, whereas surfaces 107 represent surfaces of the premorbid bone model that are 'inside' of the patient's bone surface.

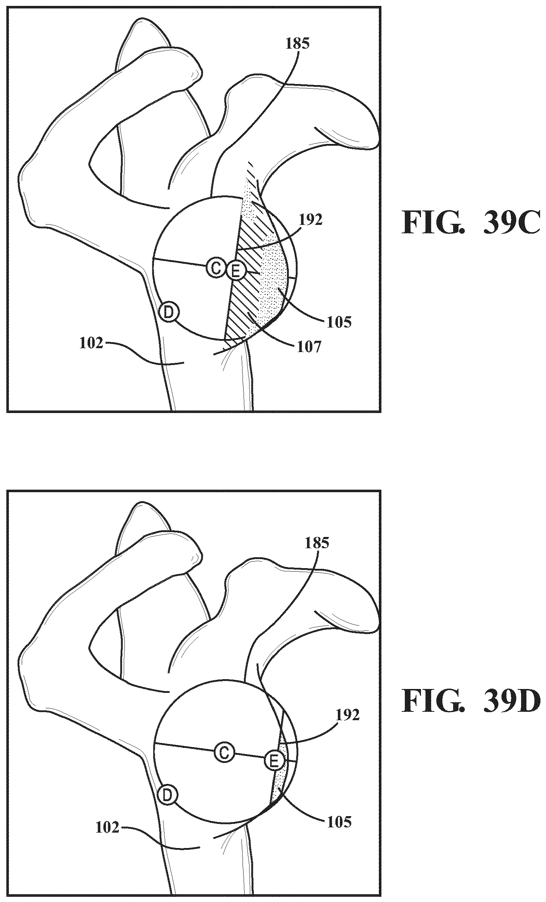

With reference to FIG. 39C and 39D, it is clear that the bone loss edge 192 is in a different position in Figure 39C and in Figure 39D. When the user adjusts the position of the bone loss edge 192, the controller 110 renders different portions of the premorbid glenoid model accordingly. With reference to FIG. 39C specifically, more of the premorbid glenoid model is shown than with respect to FIG. 39D. This can be appreciated by comparing the total area occupied by hashing areas of surfaces 105 and 107 in FIG. 39C collectively versus the total area occupied by the area of surface 105 in FIG. 39D.

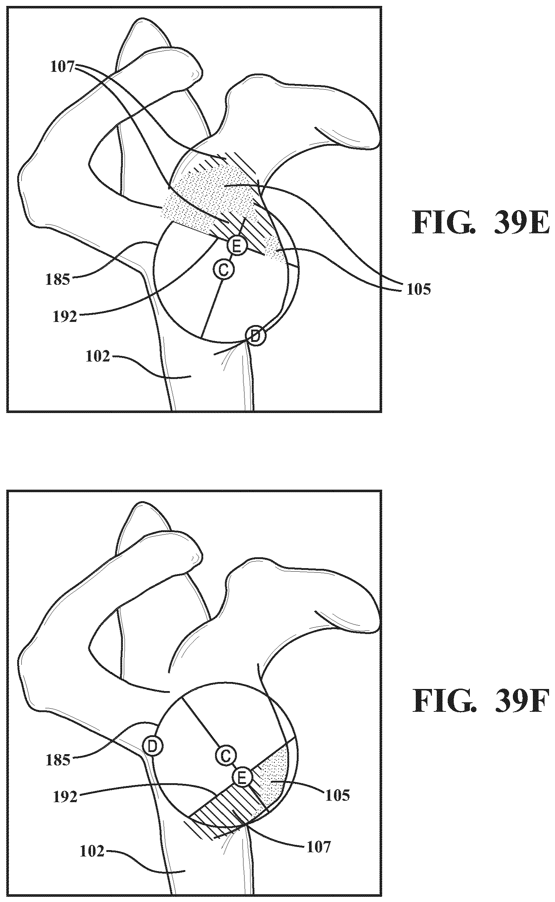

Similar to FIG. 39E and 39F, when the user adjusts the orientation of the bone loss edge 192, the controller 110 renders different portions of the premorbid glenoid model accordingly. With reference to FIG. 39E specifically, more of the premorbid glenoid model is shown than with respect to FIG. 39F. This can be appreciated by comparing the total area occupied by hashing areas of surfaces 105 and 107 in FIG. 39E collectively versus the total area occupied by hashing areas of surfaces 105 and 107 in FIG. 39F.

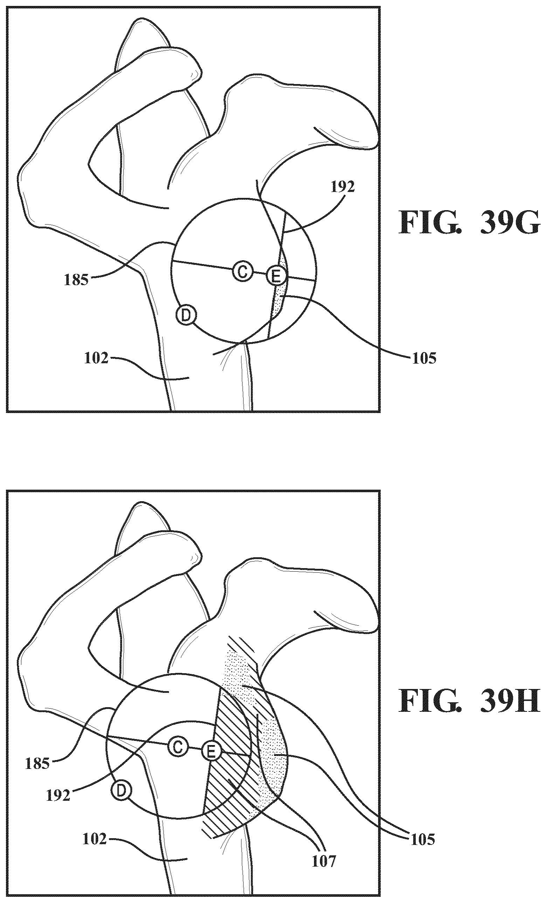

Still further, with reference to FIG. 39G and 39H, when the user adjusts the circle center and accordingly the boundary of the best fit circle object 185, the controller 110 renders different portions of the premorbid glenoid model accordingly. With reference to FIG. 39G specifically, less of the premorbid glenoid model is shown than with respect to FIG. 39H. This can be appreciated by comparing the total area occupied by hashing area of the surface 105 in FIG. 39G collectively versus the total area occupied by hashing areas of the surfaces 105 and 107 in FIG. 39H.

It should be appreciated that changing the circle diameter of the best fit circle object 185 does not influence the rendering of the different portions of the premorbid glenoid model because it does not influence the position and/or orientation of the bone loss edge 192.

### V. Identification of Soft Tissue Insertion

Referring back to FIG. 3A, the glenoid track object 170 may include a medial border 204 and a lateral border 206 to delineate the glenoid track object 170 from the rest of the humeral head portion 146. The lateral border of the glenoid track object 170 may be based on one or more anatomical landmarks. Each of the one or more anatomical landmarks may be representative of a soft tissue insertion point, such as a tendon insertion and/or an attachment of the muscles of a rotator cuff. The controller 110 may be configured to determine a location of the one or more anatomical landmarks on the humeral head portion 146.

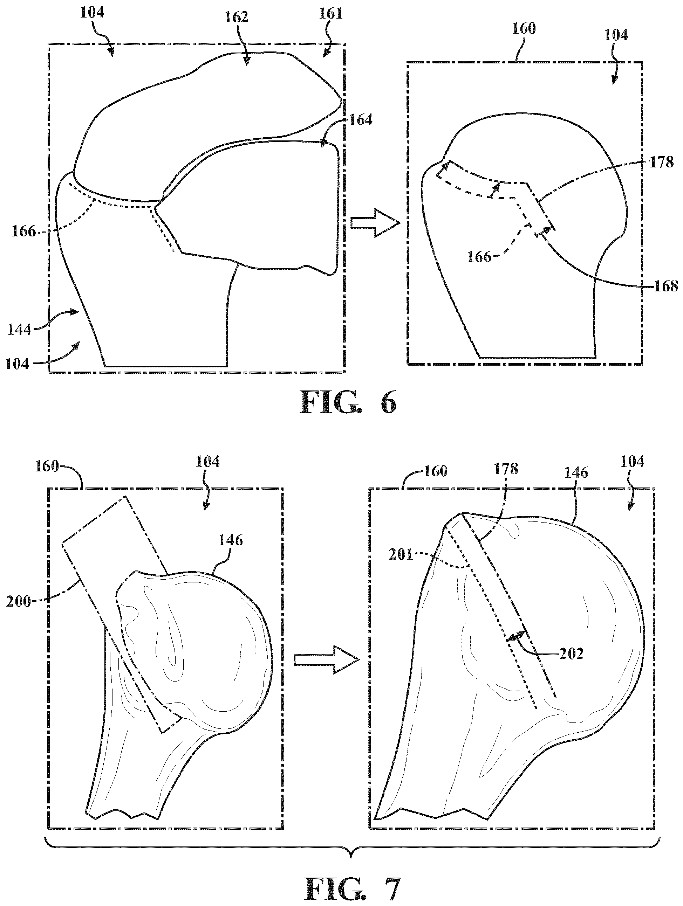

Referring to FIG. 6, the controller 110 or other computing device may employ a segmentation algorithm to segment image data of a rotator cuff muscles of the patient 112 (such as a supraspinatus and an infraspinatus) and generate 3D models, which include a rotator cuff model 161, a supraspinatus 3D model 162 and an infraspinatus 3D model 164. Other muscles may also be found in the image data of the patient 112, and those not disclosed herein may be utilized in addition to the supraspinatus and the infraspinatus. The segmentation algorithm may detect an insertion location 166 of a tendon based on the positions of the supraspinatus 3D model 162 and the infraspinatus 3D model 164 relative to the humerus 3D model 104. The controller 110 may determine one or more such locations, or locations of other soft tissue attachment points, and compute an anatomical average between those locations to generate a portion of the humerus 3D model 104 representing the insertion location 166 of the tendon. The anatomical average represents an estimation of the attachment of a rotator cuff to a humerus determined by the controller 110. The controller 110 may be configured to identify a center of the portion of the humerus 3D model 104 representing the insertion location 166. The center may optionally be translated a distance 168 based on the anatomical average of soft tissue attachment points in a medial or lateral direction, or by a fixed threshold. The insertion location 166 may represent the attachment location of the muscles of a rotator cuff to a humerus. The image data may be obtained by the imaging system 130, which may generate 3D CT image data, 3D MRI image data, or any other type of medical imaging data not disclosed herein. The landmarks, such as the soft tissue insertion location, may be represented by the landmarks stored in the statistical shape model, which becomes patient-specific by virtue of the statistical shape model being fit to the image data of the patient in question.

Referring to FIG. 7, in another aspect, the controller 110 may be configured to identify the location of one or more anatomical landmarks using curvature analysis. Curvature analysis may refer to an implementation that includes determining a plane 200 or line 201 which represents the area of greatest curvature of the humeral head portion 146, such as having the highest average curvature value. Anatomically, the plane 200 or line 201 representing the area of greatest curvature on the humeral head portion 146 may also be chosen to represent a most lateral insertion point of a rotator cuff. As such, the controller 110 may be further configured to adjust the plane 200 by an adjustment value 202 to represent the insertion of a rotator cuff or anatomical locations representing the attachment of the muscles of a rotator cuff. The adjustment value 202 may be based on common anatomical features, or otherwise determined for each patient 112, such as being based on a value indexed to the patient using various patient landmarks. Other objects are also contemplated for representing the area of greatest curvature other than the plane on the humeral head portion 146, such as a line or other shape, and those not specifically described herein may be utilized.

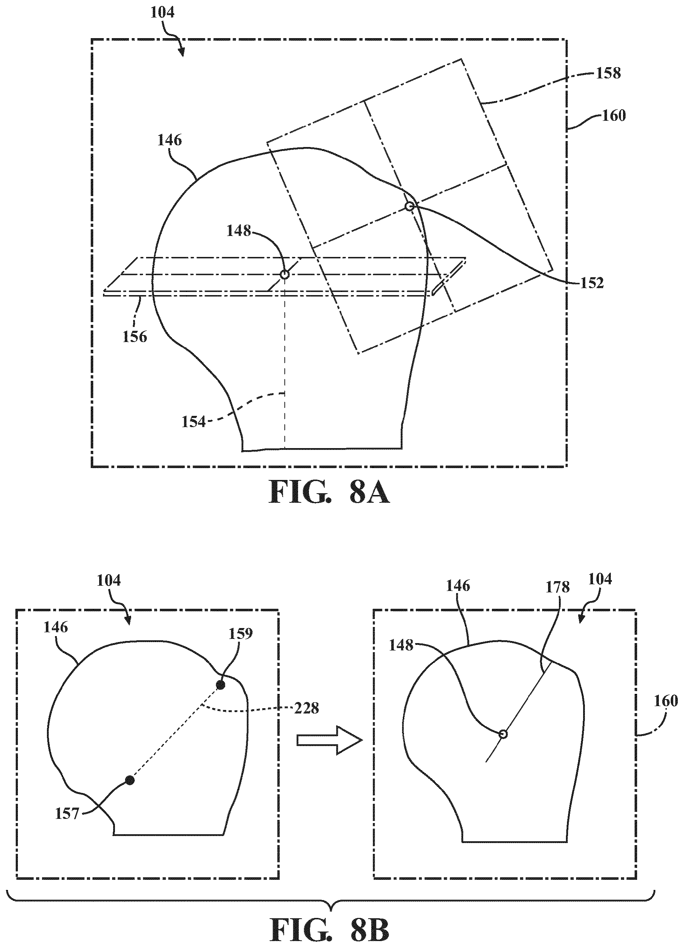

Another approach to identifying the location of the insertion of a rotator cuff is shown in FIGS. 8A and 8B, wherein the controller 110 may be configured to identify one or more anatomical locations including a humeral head center, a humeral shaft axis, an intertubercular sulcus, a rotator cable, and/or a lateral most point on the tuberculum majoris using the segmentation techniques described throughout. Other anatomical locations may be identified that are not disclosed here, and these anatomical locations may be represented by a virtual point, line, or other objects not disclosed herein. For example, the controller 110 may be configured to generate a humeral head center point 148, a humeral shaft axis object 154, an intertubercular sulcus point 157, a rotator cable point 159, and/or a lateral most point 152 on the tuberculum majoris and display these virtual objects in relation to the renderings of the 3D models. The controller 110 may be further configured to generate an axial plane 156 based on the humeral head center point 148 and/or a coronal plane 158 based on the lateral most point 152. The axial plane 156 may be based on the humeral shaft axis object 154. In some implementations, the axial plane 156 may be perpendicular to the humeral shaft axis object 154 and include the humeral head center point 148. In some implementations, the axial plane 156 may be differently related to the humeral shaft axis object 154, or even unrelated to the humeral shaft axis object 154. The coronal plane 158 may be based on the axial plane 156. In some implementations, the coronal plane 158 may be perpendicular to the axial plane 156 and include the lateral most point 152. In other implementations, the coronal plane 158 may be differently related to the axial plane 156, or even unrelated to the axial plane 156.

The controller 110 may be configured to determine a relationship 228 between the location of the intertubercular sulcus point 157 and the location of the rotator cable point 159. The relationship 228 may be represented as a line, a plane, or any other shape, and may be projected to represent the insertion of a rotator cuff. The projection direction of the relationship 228 may be based upon the coronal plane 158, for example, being perpendicular to the coronal plane 158. In one implementation, the relationship is represented by a plane 228, which contains the intertubercular sulcus point 157 and the location of the rotator cable point 159. The plane 228 is perpendicular to the coronal plane 158 and is shifted to include the humeral head center point 148. The controller 110 may be configured to generate a virtual object 178 (referred to below as the soft tissue insertion line 178) that intersects the humeral head center point 148 and the plane 228, the virtual object 178 representing the insertion of a rotator cuff. Other anatomical relationships based upon the coronal plane 158, the axial plane 156, and/or other anatomical features disclosed above may exist, and those not specifically disclosed herein may be utilized by the controller 110 to determine the location of the insertion of a rotator cuff.

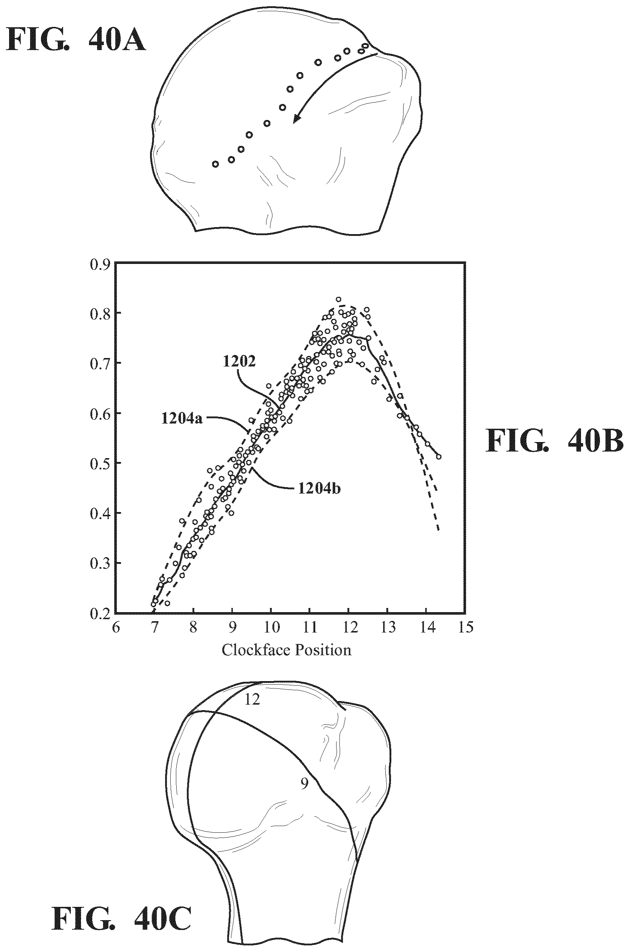

In another example, with reference to FIGS. 40A-40C, in yet another configuration, a soft tissue insertion line for the patient may be based on collecting data for a plurality of non-patient persons. With reference to FIG. 40A, to collect such data, a plurality of radiopaque beads may be placed on the humerus for each person of the plurality of the non-patient persons. In particular, the plurality of radiopaque beads are spaced about the humerus along the medial insertion line of the rotator cuff according to slightly varying clockface positions. The clockface positions for the humerus are shown in FIG. 40C, with the clockface positions of 12 and 9 being seen in the provided view.

After placement of the plurality of beads on each non-patient person, each non-patient person is imaged. Then, the medial-lateral position of the rotator cuff insertion for each bead for each non-patient person is determined for each bead at each of the various clockface positions. The more medially placed beads may annotate the articular margin for each of the non-patient persons. The plot of these medial-lateral positions (plotted as a percentage of medial-lateral width) is depicted in FIG. 40B for each bead for each person for plurality of non-patient persons.

The line 1202 in FIG. 40B shows the average medial-lateral position of the rotator cuff insertion over the plurality of non-patient persons for each clockface position. The line 1204a represents an upper confidence bound and line 1204b represents a lower confidence bound). The values of the line 1202 or similar regression may be used to obtain the position of the soft tissue insertion line for the patient's specific anatomy, in instances where no radiopaque beads have been placed). By determining the medial-lateral width for a particular patient at each clockface position, a user can determine the medial insertion line (referred to as the insertion virtual object) for that particular patient. This insertion virtual object can be used to further plan the appropriate surgical approach as will be described below.

More particularly, as described above, this patient-specific line may be described as the soft tissue insertion line or insertion virtual object described above and is representative of the rotator cuff insertion. It may be rendered on the image of the patient anatomy, and may be registered to the patient's humerus 3D model, the scapula 3D model, or combination thereof using known techniques, such as by using shape models. Thus, this method may include receiving an average rotational insertion data set and registering the average rotational insertion data to the patient-specific humerus 3D model to represent a patient specific insertion virtual object representative of the likely position of the rotator cuff insertion. In summary, the position of the insertion virtual object described above may be determined using this approach as opposed to using the other approaches described above.

As mentioned above, the controller 110 may be further configured to generate the soft tissue insertion line 178 representing the one or more anatomical landmarks and/or the location of the insertion of a rotator cuff. The soft tissue insertion line 178 may alternatively be generated as a plane or any other geometrical shape to represent the lateral border 206 of the glenoid track object 170. For example, as shown in FIG. 2, the display may be configured to show the soft tissue insertion line 178 in relation to the humerus 3D model 104 of the patient shoulder anatomy. Further, the controller 110 may be configured to cause the display to show the soft tissue insertion line 178 on the surface of the humeral head portion 146 as a plane or other shape representing the location of the insertion of a rotator cuff. The soft tissue insertion line 178 may also be manipulated and/or adjusted to better reflect patient shoulder anatomy by the user 116 via the user input device 122. For example, the user 116 may touch the display 120 and drag the soft tissue insertion line 178 to a desired location.

### VI. Display of Glenoid Track

The controller 110 may be configured to cause the display 120 to show the glenoid track object 170 relative to the humerus 3D model 104. After determining the location of the glenoid track object 170, the controller 110 may be configured to generate the glenoid track object 170 and/or the healthy glenoid track object 172 based on the location of the glenoid track object 170. As shown in FIG. 3A, the glenoid track object 170 is shown as a portion of the area of the humeral head portion 146. Similarly, the healthy glenoid track object 172 may be a line, a plane, a surface area, or other suitable shape. As one example, the healthy glenoid track object 172 is shown in FIG. 3B as a portion of the surface of the humeral head portion 146 corresponding to a healthy glenoid track area. In some implementations, the controller 110 may be configured to generate a glenoid track line 174 and/or a healthy glenoid track line 176 representing a first medial border of the glenoid track object 170 and/or a second medial border of the healthy glenoid track object 172.

As shown in FIG. 2, the glenoid track line 174 and the healthy glenoid track line 176 may be represented on the 3D model of the humeral head portion 146 and shown on the display 120. In some implementations, the glenoid track line 174 and/or the healthy glenoid track line 176 may represent different portions of the glenoid track object 170. The soft tissue insertion line 178 may represent the lateral boundary of the glenoid track object 170. The controller 110 may be configured to determine the location of the glenoid track line 174 and/or the healthy glenoid track line 176 based on the location of the soft tissue insertion line 178 and the glenoid width 180. For example, after determining the location of the soft tissue insertion line 178 based on one or more anatomical landmarks, the controller 110 may determine the location of the glenoid track line 174 by shifting the soft tissue insertion line 178 by the glenoid width 180. Similarly, the controller 110 may determine the location of the healthy glenoid track line 176 by shifting the soft tissue insertion line 178 by the healthy glenoid width 182. The glenoid track line 174, the healthy glenoid track line 176, and the soft tissue insertion line 178 may be shown in relation to each other and to the humerus 3D model 104 and/or the humeral head portion 146 on the display 120.

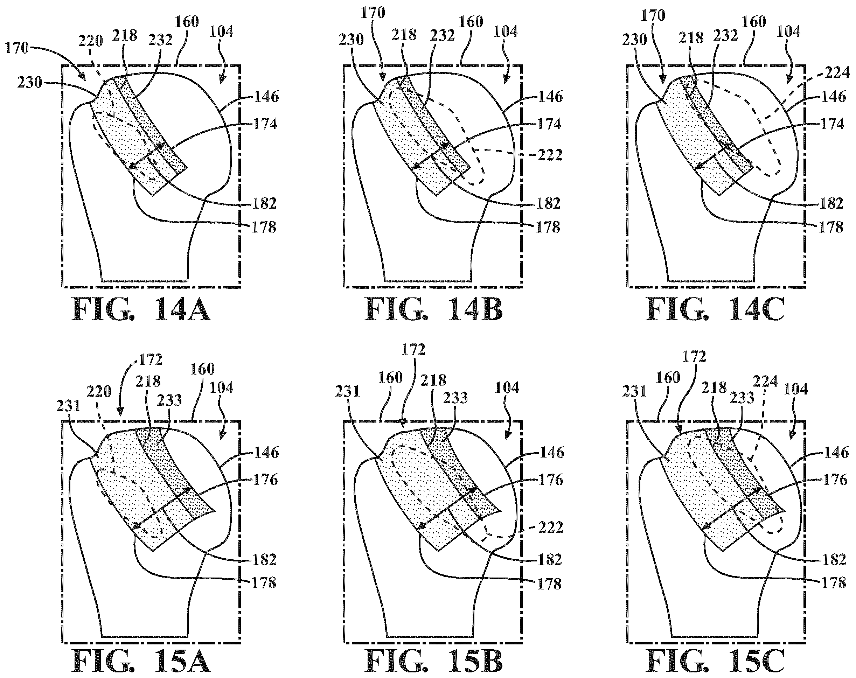

The controller 110 may also be configured to divide the area of the glenoid track object 170 and/or the healthy glenoid track object 172 into two or more sections to delineate a proportion of the glenoid track object 170. Referring to FIG. 14A, a first area 230 represents up to 75% of the glenoid width 180, and a second area 232 represents the remaining portion of the glenoid width 180. The controller 110 may be configured to generate a divide indicator object 218 based on the configuration of the first area 230 and the second area 232 and show the divide indicator object 218 on the display 120. The divide indicator object 218 is illustrated in FIG. 14A as a line, although in some implementations, it may be a plane or other shape configured to indicate the boundary between the first area 230 and the second area 232. This disclosure does not limit the areas to the ranges listed above, and other ranges and/or areas are contemplated. For example, the area of the glenoid track object 170 and/or the healthy glenoid track object 172 may be divided into more than two areas, or not divided at all. In addition, the controller 110 may be configured to generate more than one divide indicator object 218 depending on the number of areas that the glenoid track object 170 and/or the healthy glenoid track object 172 is divided into. By dividing the glenoid track object 170, a more granular understanding of the joint can be discerned, particularly, the division provides for a better understanding of whether the lesion is on-track, near-track, or off-track. The division of the glenoid track object 170 into multiple areas may also help the user 116 take into account the impact of soft tissue laxity on the glenoid track.

### VII. Identification of Lesion

The surgical planning system 100 may be configured to identify and/or visualize anatomical anomalies associated with the patient shoulder anatomy. The controller 110 and/or the imaging system 130 may be configured to automatically identify anatomical anomalies in the patient image data, or the user 116 may view the patient image data on the display 120 and manually identify anatomical anomalies. For example, the controller 110 may be configured to carry out a method for identifying the presence and/or location of the lesion object 210 on the humeral head portion 146, which may be known as a Hills-Sachs lesion. In some implementations, the controller 110 may be configured to carry out a method for identifying the presence and/or location of the lesion object 210 on a glenoid, which may be known as a Bankart lesion. In the exemplary configuration described below, the surgical planning system 100 is configured to identify the presence and/or location of a Hills-Sachs lesion.

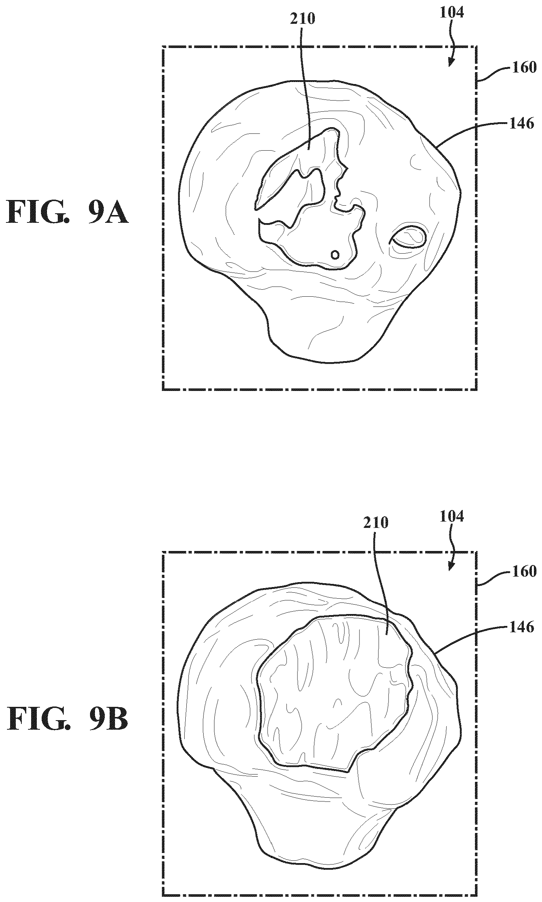

Referring to FIG. 9A, the controller 110 or other computing device may apply a segmentation algorithm to the patient image data to create the 3D model and identify the presence and/or location of the lesion object 210 on the humeral head portion 146. The segmentation algorithm may be used to segment image data of patient shoulder anatomy in order to detect the presence and/or location of a lesion and generate the lesion object 210. In FIG. 9A, the lesion object 210 is a Hills-Sachs lesion on the surface of the humeral head portion 146. However, other types of lesions (e.g., a Bankart lesion) may be identified by the surgical planning system 100. The controller 110 may determine the presence and/or location of one or more lesions based on the output of the segmentation. In some implementations, the controller 110 may be further configured to run a deep learning algorithm to segment the image data of the patient shoulder anatomy, and/or to identify the lesion object 210 on a humerus prior to, during, or after segmentation. The deep learning algorithm may also be configured to detect the surface area of the lesion object 210 and/or the border of the lesion object 210. The detection of the lesion may be initially based on the image data of the relevant patient shoulder anatomy. In some implementations, the deep learning algorithm may provide an output of the region in the form of a binary mask that includes the lesion object 210 in the voxel space. The mask may then be mapped onto a 3D surface model of the segmented humeral head portion 146 to provide a 3D visual of the lesion object 210. The deep learning algorithm may be trained with data from a population of persons and manually identified lesion objects 210 to accurately identify lesion objects 210 on the humeral head portion 146. Other image processing algorithms are also contemplated for identifying the location of one or more anatomical landmarks, and those not specifically described herein may be utilized, such as using an active appearance model.

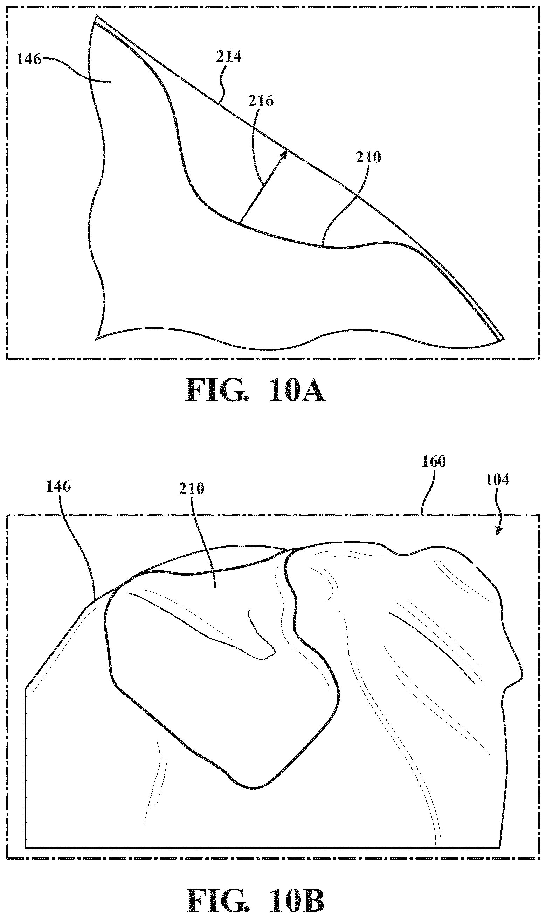

Referring to FIGS. 10A and 10B, the controller 110 may be configured to apply a healthy surface prediction algorithm to the patient image data and/or the 3D model of patient shoulder anatomy to detect the lesion object 210. For example, the controller 110 may deploy a statistical shape model to generate a predicted surface 214 of a healthy humeral head. More specifically, the controller 110 may be configured to fit a statistical shape model to the image data of patient shoulder anatomy to generate a premorbid prediction of the surface of the humeral head, i.e., a prediction of the surface of the humeral head before the occurrence of a pathology. Alternatively, the segmentation algorithm described above may be utilized to generate a surface based on a humeral mesh. Following the generation of a healthy surface prediction, the controller 110 may deploy an algorithm to generate a distance map of the humeral head portion 146, and the distance map may be utilized to detect the lesion object 210.

As shown in FIG. 10A, the distance map may indicate a lesion depth 216 from the surface of patient shoulder anatomy to the predicted surface 214 of the healthy patient shoulder anatomy. In the absence of the lesion object 210, the distance map may indicate minimal deviation between the predicted surface 214 of the healthy patient shoulder anatomy and the surface of the patient shoulder anatomy. In the presence of the lesion object 210, the distance map may indicate various distances between the surface of patient shoulder anatomy and the predicted surface 214 of the healthy patient shoulder anatomy. The controller 110 may be configured to generate a representation of the lesion object 210 based on the distance map. Further, as shown in FIG. 10B, the controller 110 may be configured to generate the lesion object 210 in relation to the surface of the humeral head portion 146 based on the changes in the lesion depth 216 between the surface of patient shoulder anatomy and the predicted surface 214 of the healthy patient shoulder anatomy.

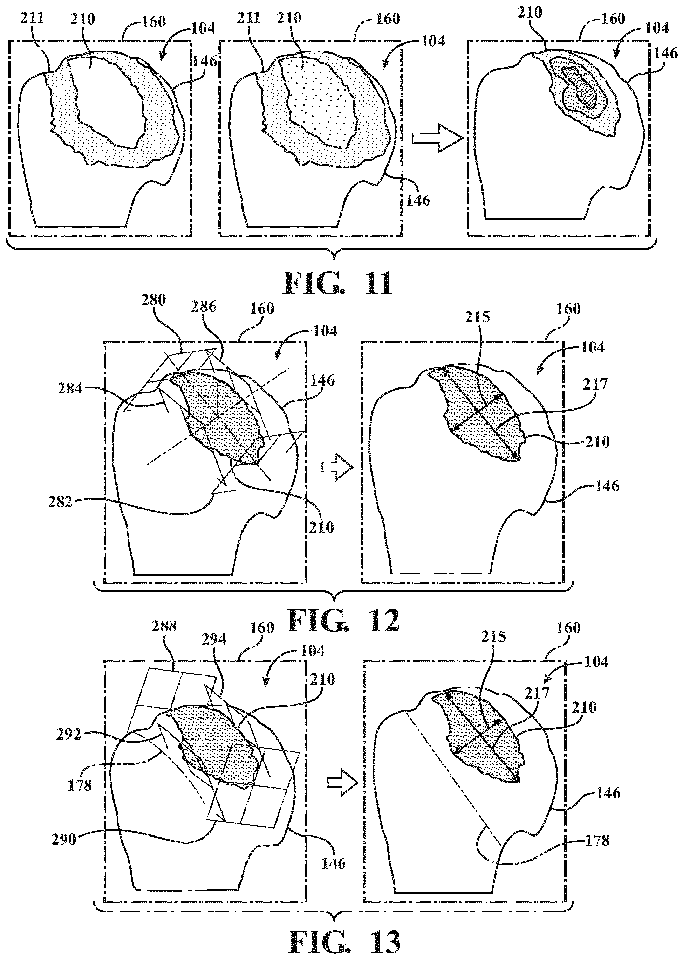

The controller 110 may be configured to cause the display 120 to show the lesion object 210 as a heatmap corresponding to the distance map described above. Referring to the leftmost images of FIG. 11, the lesion object 210 may be differentiated from the rest of the humeral head portion 146 by rendering a difference in color, shading, pattern, or any other visual difference on the display 120. The controller 110 may also be configured to determine a surface region 211 of the humeral head portion 146 that outlines the lesion object 210. The surface region 211 may generally have a donut-like shape. The controller 110 may be configured to determine the surface region 211 by defining a boundary spaced from and surrounding the lesion object 210. For instance, the controller 110 may define such boundary by "growing" the lesion object 210 (e.g., enlarging the lesion object by a set number of millimeters), or expanding the lesion object 210 by a dilation, or using other image processing techniques. The area between the boundary and the lesion object 210 may then be defined as the surface region 211. The surface region 211 on the humeral head portion 146 may similarly be differentiated from the rest of the humeral head portion 146 on the display 120 by a difference in color, shading, pattern, or any other visual difference. The controller 110 may be configured to cause the display 120 to show just the surface region 211 relative to the humerus 3D model 104, or just the lesion object 210 relative to the humerus 3D model 104. As such, the controller 110 may be configured to identify the surface region 211 and use the surface region 211 to extrapolate the healthy surface of the humeral head portion without displaying the surface region 211 on the display 120.

Still referring to FIG. 11, while the lesion object 210 by its nature may have a concave/irregular surface, the surface region 211, which is not a lesion, may represent an accurate shape of the humeral head portion 146. The controller 110 may thus be configured to use the surface region 211 to estimate and/or extrapolate a healthy surface of the humeral head portion 146 in the area of the lesion object 210. As shown in the rightmost image of FIG. 11, the controller 110 may then be configured to generate a heatmap by comparing the depth of the estimated/extrapolated healthy surface to the depth of the lesion object 210, and cause the display 120 to show the heatmap. The heatmap may represent different ranges of depths between the surface of the lesion object 210 and the estimated/extrapolated healthy surface with varying colors, shadings, patterns, or other visual differences. These areas of depth may correspond to the distance map of FIG. 10A, wherein there are variations within the depth of the lesion object 210. The user 116 may use the heatmap to help identify a location of maximum depth of a lesion and/or a location of minimum depth of a lesion based on a visual difference in accordance with these variations. The heatmap may also help with identifying the number, position and sizing of screws for a Remplissage repair, as one non-limiting example.

The controller 110 may be configured to determine a lesion width 215 of the lesion object 210, and a lesion length 217 of the lesion object 210. In some implementations, the controller 110 may be configured to determine inertia axes, or weighted mass centers of the lesion object 210. The controller 110 may be configured to generate one or more planes based on the inertia axes and measure the lesion width 215 and the lesion length 217 based on the one or more planes. Referring to FIG. 12, the controller 110 may be configured to generate a first plane 280 at the superior end of the lesion object 210, a second plane 282 at the inferior end of the lesion object 210, a third plane 284 at the lateral side of the lesion object 210, and a fourth plane 286 at the medial side of the lesion object 210. The lesion width 215 may be determined based on the first and second planes 280,282, and the lesion length 217 may be determined based on the third and fourth planes 284,286.

Additionally or alternatively, the controller 110 may be configured to determine the lesion width 215 and the lesion length 217 by measuring the lesion object 210 relative to the soft tissue insertion line 178. Referring to FIG. 13, the controller 110 may define a fifth plane 288 and a sixth plane 290 intersecting the humeral head portion 146 and the soft tissue insertion line 178. The fifth and sixth planes 288, 290 are virtual objects that may be shown on the display 120 relative to the humerus 3D model 104. The fifth plane 288 may include the superior end of the lesion object 210, as well as the superior end of the soft tissue insertion line 178. The sixth plane 290 may include the inferior end of the lesion object 210, as well as the inferior end of the soft tissue insertion line 178. After generating these planes 288, 290, the controller 110 may be configured to determine a distance between the fifth plane 288 and the sixth plane 290, which may be utilized to approximate the lesion length 217. Further, the controller 110 may be configured to determine the lesion width 215 by generating a seventh plane 292 and an eighth plane 294 parallel to the soft tissue insertion line 178. The medial-lateral position of the seventh and eighth planes 292, 294 may be determined by the controller 110 by aligning the seventh plane 292 with the lateral edge of the lesion object 210 and the eighth plane 294 with the medial edge of the lesion object 210. The controller 110 may be configured to measure the distance between the seventh and eighth planes 292, 294, which may be utilized to approximate the lesion length 217.

In some cases, referring back to FIG. 9A, the segmentation algorithm, the deep learning model, and/or the healthy surface prediction algorithm may provide a flawed representation of the lesion object 210. Although the approximate location and shape of the lesion object 210 may be identified accurately, it is possible that the boundaries of the lesion object 210 on the humeral head portion 146 remain undefined. Referring to FIG. 9B, the controller 110 may be further configured to apply one or more of the following algorithms: edge detection, watershed analysis, curvature analysis, and/or statistical shape modeling to further refine the boundaries of the lesion object 210. The controller 110 may be configured to apply one or more of these algorithms to the 3D models and/or image data. These algorithms may be referred to as post-processing algorithms and may include some algorithms not specifically described herein. Any one of the post-processing algorithms may be applied to the image data of patient shoulder anatomy to generate the 3D model through segmentation or may be applied to a segmented 3D model. The output of any one of the post-processing algorithms may be the 3D model including anatomical data, where the anatomical data includes the presence and/or location of a lesion on the patient shoulder anatomy. The controller 110 may be configured to cause the display 120 to show the lesion object 210. Referring to FIG. 9B, after the application of the post-processing algorithms, the boundaries of the lesion object 210 may be more accurately defined. In addition, the controller 110 may further be configured to receive input from the user from the user input device 122 to manipulate the boundaries of the lesion object 210 on the surface of the humeral head portion 146 to correct any flaws in the 3D model prior to post-processing.

### VIII. Display of Lesion

The controller 110 may be configured to generate the lesion object 210 after identifying the location of a lesion with the methods disclosed above. Shown in FIG. 14A, the lesion object 210 includes the surface area of the humeral head portion 146 occupied by a lesion. The boundary of the lesion object 210 may be determined by the controller 110 with one or more post processing algorithms and/or based on user input.

Referring to FIGS. 14A-15C, the controller may be configured to cause the display to show the lesion object 210 relative to the humerus 3D model 104 of the patient shoulder anatomy, specifically in relation to the humeral head portion 146. As shown in FIG. 14A, the lesion object 210 may be displayed in various manners, such as an outline of a shape, or as an opaque shape covering a portion of the surface area of the humeral head portion 146. The lesion object 210 may be displayed only relative to the humerus 3D model 104 and the humeral head portion 146, or in relation to one or more anatomical features and/or virtual objects representing anatomical features. Further, the lesion object 210 may be displayed relative to the glenoid track object 170, the glenoid track line 174, and/or the soft tissue insertion line 178. As shown in FIGS. 15A-15C, the lesion object 210 may also be displayed relative to the healthy glenoid track object 172, the healthy glenoid track line 176, and the soft tissue insertion line 178. The controller 110 may be configured to allow the user 116 to modify or remove the lesion object 210 from the display 120. For example, the user 116 using the user input device 122 may adjust a boundary of the lesion object 210 to include more of the patient shoulder anatomy, or less of the patient shoulder anatomy. The controller 110 may be configured to record certain views, slices, or configurations of the contents of the display 120, including renderings of the 3D models and the lesion object 210 relative to other anatomical features to facilitate or to facilitate clinical decision-making with regard to surgical procedures.

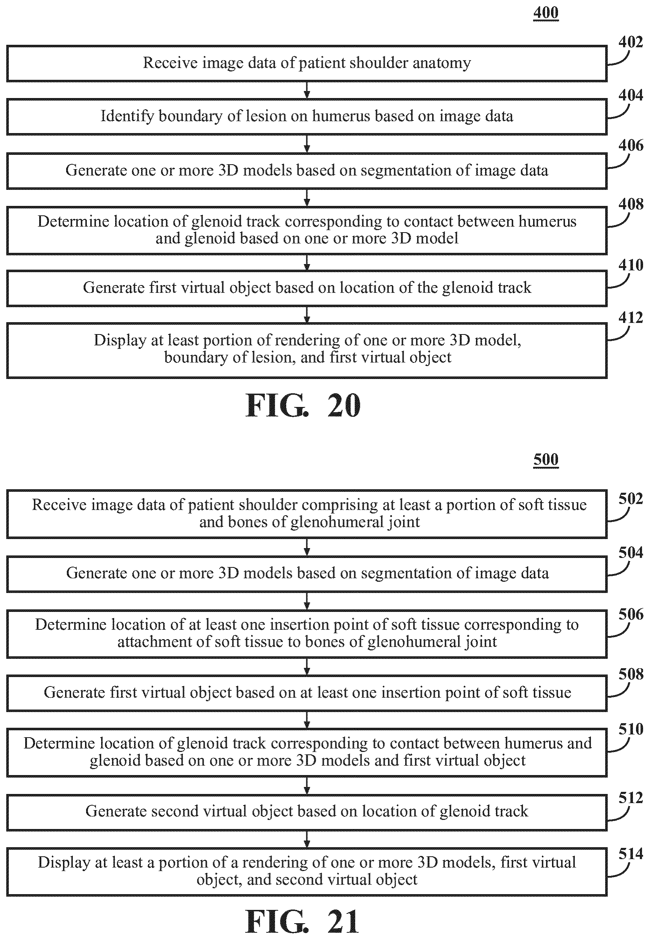

Having described the surgical planning system 100, a method 400 of visualizing patient shoulder anatomy using the surgical planning system 100 (i.e., the controller 110) is illustrated in FIG. 20. The method 400 may be executed pre-operatively or intra-operatively to plan one or more shoulder surgeries for the patient 112. At step 402, the controller 110 may receive image data of the patient shoulder anatomy, such as image data including a humerus and a glenoid of the patient 112. At step 404, the controller 110 may identify the boundary of a lesion on the humerus based on the image data, as described above in Section VII. At step 406, the controller 110 may generate one or more 3D models based on a segmentation of the image data, as described above in Section II. At step 408, the controller 110 may determine the location of a glenoid track corresponding to contact between the humerus and the glenoid based on the 3D models as described above in Section IV and shown in FIG 3A. At step 410, the controller 110 may generate a first virtual object based on the location of the glenoid track, like the glenoid track object 170 shown in FIG. 3A and described above in Section IV. At step 412, the controller 110 may cause the display 120 to show (simultaneously or independently) portions of renderings of the one or more 3D models, a boundary of the lesion object 210, and the glenoid track object 170, as shown in FIGS. 14A-14C and described above in Section VIII.

Another method 500 of visualizing patient shoulder anatomy involving the controller 110 and further involving the soft tissue insertion line 178 is illustrated in FIG. 21. Similar to the previously described method 400, the method 500 may be executed pre-operatively to plan one or more shoulder surgeries for the patient 112. At step 502, the controller 110 may receive image data of patient shoulder anatomy including soft tissue and bones of the glenohumeral joint, such as image data including a humerus, a glenoid, a supraspinatus, and an infraspinatus of the patient 112. At step 504, the controller 110 may generate one or more 3D models based on a segmentation of the image data, as described above in Section II. At step 506, the controller 110 may determine the location of the insertion point of soft tissue corresponding to attachment of soft tissue to bones of the glenohumeral joint, as shown in FIGS. 6-8 and described above in Section V. At step 508, the controller 110 may generate a first virtual object representing the insertion point of the soft tissue, described above as the soft tissue insertion line 178. At step, the controller 110 may determine the location of a glenoid track corresponding to the contact between the humerus and the glenoid based on the 3D model and the first virtual object, as shown in FIG. 3A and described above in Section IV. At step 512, the controller 110 may generate a second virtual object based on the location of the glenoid track, wherein the second virtual object is the glenoid track object 170 as shown in FIG. 3A and described above in Section IV. At step 514, the controller 110 may cause the display 120 to show (simultaneously or independently) portions of the renderings of the one or more 3D models, the first virtual object, and the second virtual object, as shown in FIGS. 3A and 3B and described above in Section VI.

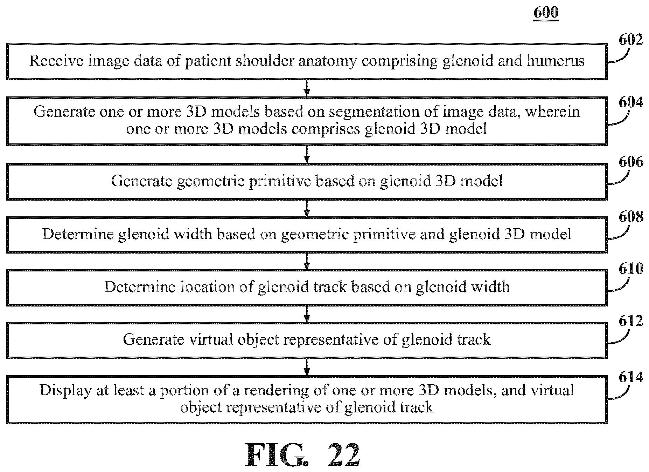

A method 600 of visualizing patient shoulder anatomy involving the controller 110 and further involving the glenoid width 180 is illustrated in FIG. 22. Again, the method 600 step may be executed pre-operatively or intra-operatively to plan one or more shoulder surgeries for the patient 112. At step 602, the controller 110 may receive image data of patient shoulder anatomy including a glenoid and a humerus of the patient 112. At step 604, the controller 110 may generate one or more 3D models based on a segmentation of the image data, wherein the one or more 3D models includes a glenoid 3D model 102, as described above in Section II. At step 606, the controller 110 may generate a geometric primitive 194 based on the glenoid 3D model 102, as shown in FIG. 5A and described above in Section IV. At step 608, the controller 110 may determine the glenoid width 180 based on the geometric primitive 194 and the glenoid 3D model 102, as shown in FIG. 5B and described above in Section IV. At step 610, the controller 110 may determine the location of the glenoid track based on the glenoid width 180. At step 612, the controller 110 may generate a virtual object based on the location of the glenoid track, wherein the virtual object is the glenoid track object 170 as described above in Section IV. At step 614, the controller 110 may cause the display 120 to show (simultaneously or independently) a portion of a rendering of the one or more 3D models and the glenoid track object 170, as shown in FIG. 3A and described by method 400.

Any of the above methods may be executed independently, or concurrently with the other disclosed methods, and any of the presented steps may be executed in any combination or order not disclosed herein. Any of the above methods may be carried out by the surgical planning system 100 to aid the user 116 in performing an assessment of the patient glenohumeral joint and/or determining the likelihood of a lesion having an impact on joint engagement.

### IX. Determination of Track Engagement

In addition to displaying the lesion object 210 relative to the humerus 3D model 104 and/or other virtual objects representing anatomical features, the system 100 may assess the effect of a lesion on the patient shoulder anatomy. The effect of one or more lesions on patient shoulder anatomy may be referred to as a track engagement. The track engagement may include a comparison between the healthy glenoid track object 172 and the glenoid track object 170, between the healthy glenoid track object 172 and the lesion object 210, between the glenoid track object 170 and the lesion object 210, or any combination of the above. The track engagement may also be assessed based on other objects representing anatomical features, and the track engagement is not limited to utilizing the comparisons disclosed herein. Many other criteria for track engagement exist, and those not disclosed herein may be utilized in some implementations of the system.

As shown in FIG. 2, the controller 110 may cause the display 120 to show renderings of two or more 3D models of patient shoulder anatomy simultaneously. For example, the display 120 may show a rendering of the humerus 3D model 104 with the glenoid track object 170, and the display 120 may concurrently show a 3D model of patient shoulder anatomy with the healthy glenoid track object 172. The user 116 may be able to compare the relative size and shape of the glenoid track object 170 to the healthy glenoid track object 172, and qualitatively determine the health of patient shoulder anatomy and potential need for shoulder surgery. For example, if the glenoid track object 170 is significantly smaller than the healthy glenoid track object 172, the user 116 may recommend a shoulder surgical procedure regardless of any other factors.

Further, as shown in FIGS. 14A-14C, the controller 110 may cause the display 120 to show a rendering of the humerus 3D model 104 of the patient shoulder anatomy, the glenoid track object 170, and the lesion objects 220, 222, and 224. Other anatomical features such as the glenoid track line 174, the divide indicator object 218, or the soft tissue insertion line 178 may also be displayed along with the glenoid track object 170. Analysis of the glenoid track object 170 and the lesion objects 220, 222, and 224 may yield one of the following assessments regarding the state of a lesion: on-track, off-track, or near-track. Determination of one or more of these states of the lesion may be qualitative and/or quantitative. The state of the lesion may be based on the proximity of the lesion objects 220, 222, and 224 to the glenoid track object 170, the position of the lesion objects 220, 222, and 224 relative to the glenoid track object 170, and/or the intersection between the lesion objects 220, 222, and 224 and the glenoid track object 170, the glenoid track line 174, the divide indicator object 218, and the soft tissue insertion line 178.

In some implementations, the state of a lesion (e.g., on-track, near-track, off-track) may be determined based on the intersection of the lesion objects 220, 222, 224 and anatomical features, such as the glenoid track line 174, the divide indicator object 218, and the soft tissue insertion line 178. For example, the lesion shown in FIG. 14A may be considered an on-track and identified as lesion object 220. The on-track state of lesion object 220 is assigned to this instance because the lesion object 220 intersects the first area 230 of the glenoid track object 170, and may not interfere with normal function of the glenohumeral joint or necessitate shoulder surgery planning. Thus, the controller 110 may classify the lesion object 220 as having the on-track state and the user can understand that this lesion object has minimal risk to track engagement. As another example, the lesion shown in FIG. 14B is off-track (see lesion object 222). The controller 110 may determine that lesion object 222 is considered to be an off-track lesion because lesion object 222 intersects the area of the humeral head portion 146 medial to the second area 232 of the glenoid track object 170 and/or extends beyond the glenoid track line 174. As yet another example, the lesion shown in FIG. 14C is off-track as lesion object 224 intersects the area of the humeral head portion 146 medial to the second area 232 of the glenoid track object 170. Off-track lesions are certain to impact track engagement and have a greater likelihood of a shoulder dislocation and may necessitate shoulder surgery. The user 116 may utilize the user input device 122 to interact with the controller 110 for surgical planning of such a shoulder surgery. For example, the user 116 may plan to complete a bone graft procedure to correct patient shoulder anatomy and resolve the off-track lesion to reduce the likelihood of recurrent shoulder instability. Other assessments of the position of the lesion may lead to other shoulder surgeries being contemplated, and those not specifically disclosed herein may be utilized.

With further reference to Figures 14A-14C, it should be appreciated that the location of the lesion objects 220, 222, 224 relative to the glenoid track object 170 is different in each of these examples. Furthermore, the dimension of the lesion objects 220, 222, 224 is different in each of these examples. Because of the different positions and/or dimensions of the lesion objects 220, 222, 224 and the resultant different overlap with the glenoid track object 170, the controller 110 may determine a different impact rating for each of these instances of the patient's shoulder anatomy (as will be described below).

Even further, as shown in FIGS. 15A-15C, the controller 110 may cause the display 120 to show a rendering of the humerus 3D model 104 of the patient shoulder anatomy, the healthy glenoid track object 172, and the lesion objects 220, 222, and 224. These figures show the same lesion objects 220, 222, 224 as depicted in FIGS. 14A-14C (i.e., they are in the same position and have the same dimensions as the lesion objects depicted in those figures). However, in FIGS. 15A-15C, the lesion objects 220, 222, 224 are depicted with respect to the healthy glenoid track object 172, whereas in FIGS. 14A-C, the lesion objects 220, 222, 224 are depicted with respect to glenoid track object 170.

Other virtual objects representing anatomical features such as the healthy glenoid track line 176, the divide indicator object 218, or the soft tissue insertion line 178 may also be displayed along with the healthy glenoid track object 172. Like the analysis of the glenoid track object 170 with respect to lesion objects 220, 222, and 224, analysis of the healthy glenoid track object 172 and the lesion objects 220, 222, and 224 may enable the controller 110 to determine the state of the lesion: on-track, off-track, or near-track in the case where the glenoid width is restored to a healthy width. The state of the lesion may be based on the proximity of the lesion objects 220, 222, and 224 to the healthy glenoid track object 172, the position of the lesion objects 220, 222, and 224 relative to the healthy glenoid track object 172, and the intersection between the lesion objects 220, 222, and 224 and the healthy glenoid track object 172. In some implementations, the state of the lesion may be based on the intersection of the lesion objects 220, 222, and 224 and virtual objects representing anatomical features, such as the healthy glenoid track line 176, the divide indicator object 218, and the soft tissue insertion line 178. For example, the lesion shown in FIG. 15A may be considered on-track as lesion object 220 intersects a first area 231 of the healthy glenoid track object 172 and does not extend medially past the divide indicator 218. Lesion object 222, as shown in FIG. 15B, is determined to be a near-track lesion as the lesion object 222 overlaps with the divide indicator object 218 and intersects a second area 233 of the healthy glenoid track object 172 and does not extend medially past the healthy glenoid track line 176. The near-track state of lesion object 222 indicates a lesion which may have an increased likelihood of interfering with the normal function of the glenohumeral joint and may be classified as having some impact on the track engagement, therefore increasing the likelihood for a shoulder dislocation and potentially requiring shoulder surgery. Referring to FIG. 15C, the lesion object 224 is considered to be off-track, as the lesion object 224 overlaps with the healthy glenoid track line 176 and intersects the area of the humeral head portion 146 medial to the second area 233. The lesion object 224 could alternatively be determined as off-track if it overlaps one or both of the soft tissue insertion line 178 or the glenoid track line 176. The off-track state of lesion object 224 indicates a greater risk of recurrent shoulder instability and may necessitate shoulder surgery, and the user 116 may utilize the user input device 122 to communicate with the controller 110 for surgical planning as described above.

It is possible that the lesion object 222 of patient shoulder anatomy may be considered off-track when compared to the glenoid track object 170 and may be classified as near track when the lesion object 222 of patient shoulder anatomy is compared to the healthy glenoid track object 172. In this situation, the user 116 may plan shoulder surgery to minimize the impact of the lesion object 210 on track engagement. Alternatively, the lesion object 224 of patient shoulder anatomy may be considered off-track when compared the glenoid track object 170 and may be classified as an off-track when compared to the healthy glenoid track object 172. In this situation, the user 116 may plan alternative or additional shoulder surgery to address the impact of the lesion on patient shoulder anatomy. By providing this information to the user, the user may be able to easily assess the impact of the lesion on track engagement and likelihood of recurrent shoulder instability, which may be particularly useful deciding between glenoid-sided and/or humeral-sided shoulder surgery.

The comparison between the lesion objects 220, 222, 224 described above and the glenoid track object 170 and/or the healthy glenoid track object 172 may serve one or more purposes. Primarily, the user 116 may utilize the comparison between the lesion objects and the healthy glenoid track object to determine a surgical plan suitable for the patient. Additionally, the user 116 may utilize the track engagement between the lesion objects and the healthy glenoid track object 172 as support for a more intensive shoulder surgery in the case of an off-track lesion, as standard restoration of the glenoid track may not be suitable for resolving the impact on track engagement by an off-track lesion.

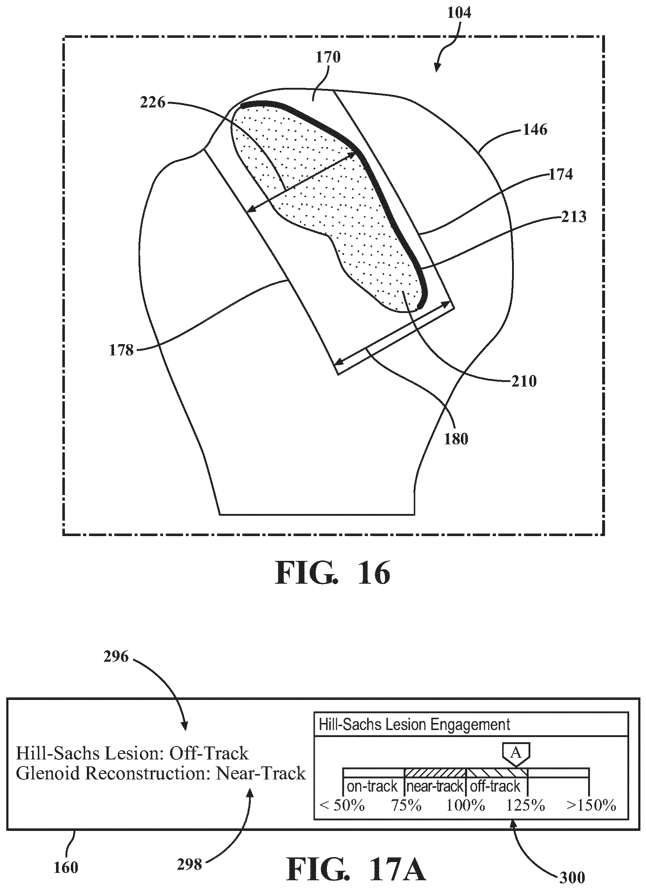

The analysis described above may be a manual process, or the controller 110 may be configured to determine the effect of the lesion objects on track engagement automatically. Referring to FIG. 16, one example of a method for automatically determining the effect of the lesion object 210 on track engagement is shown. In this example, the controller 110 may be configured to identify a medial border 213 of the lesion object 210 and measure a lesion distance 226. The lesion distance 226 may be the maximum distance between the medial border 213 of the lesion object 210 and the soft tissue insertion line 178. The lesion distance 226 may be manually measured on the 3D model by the user 116 or automatically determined by the controller 110 and shown on the display 120. The controller 110 and/or the user 116 may compare the lesion distance 226 to the glenoid width 180 multiplied by a threshold value. In some implementations, this threshold value is at least 75%, 80%, or 85%. In one example, the threshold value may be 83%, and the product of the glenoid width 180 and the threshold value may be known as a glenoid comparison value. If the lesion distance 226 is less than the glenoid comparison value and greater than 0.75 multiplied by the glenoid comparison value, the controller 110 may classify the lesion object 210 as a near-track lesion . Similarly to the analysis above, the near-track state may cause a user to understand that the lesion may impact the track engagement and necessitate shoulder surgery planning. If the lesion distance 226 is less than the glenoid comparison value multiplied by 0.75, the controller 110 may classify the lesion object 210 as an on-track lesion, which would not impact the track engagement and may not necessitate shoulder surgery planning. If the lesion distance 226 is greater than the glenoid comparison value, the controller 110 may classify the lesion object 210 as an off-track lesion, which would impact track engagement and necessitate shoulder surgery planning. The user 116 may also manually classify the lesion object 210 as an off-track, on-track, or near-track lesion based on the comparison between the lesion distance 226 and the glenoid width 180.

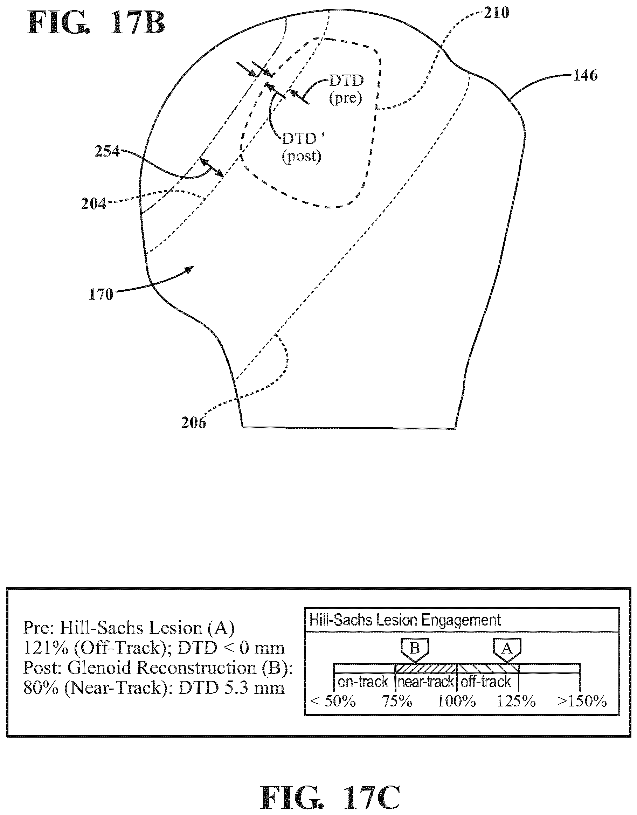

Additionally, the controller 110 may be configured to generate one or more track indicators to represent the track engagement. Referring to FIG. 17, the controller 110 may cause the display 120 to show a track indicator which may be referred to as a first signal 296 that the lesion object 210 is classified as an off-track lesion object 224. The first signal 296 may be configured to indicate other states of the lesion object 210, such as an on-track or near-track lesion. Another example of a track indicator could be referred to as a second signal 298 which may further differentiate between whether the lesion object 210 is classified relative to the glenoid track object 170 or the healthy glenoid track object 172. In FIG. 17, the second signal 298 indicates that when the lesion object 210 is compared to the healthy glenoid track object 172, the lesion may be classified as a near track lesion. Yet another example of a track indicator is an impact rating 300, which provides a degree of on-track or off-track regarding the state of a lesion. As shown in FIG 17, impact rating 300 may be shown as a sliding scale. In some implementations, the impact rating 300 may be a numerical scale to indicate severity, an image, or other signal to provide a degree of on-track or off-track. The controller 110 may be configured to cause the display 120 to show the track indicators to facilitate or to facilitate clinical decision-making with regard to surgical procedures. The configuration shown in FIG. 17 effectively summarizes the analysis of the patient shoulder anatomy, which advantageously saves time for the user 116 in assessing the condition of a glenohumeral joint. The automatic generation and display of the track indicators allows for accurate and concise analysis to be provided to the user 116 in planning shoulder surgery to address the state of the patient shoulder anatomy.

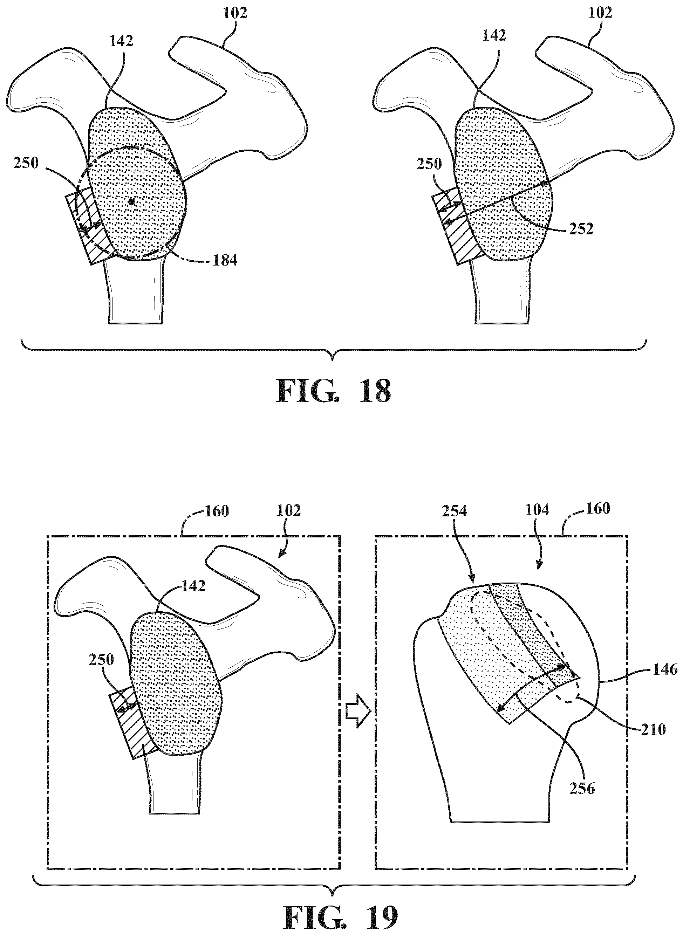

In addition or as an alternative to the one or more features described above, the controller 110 may be configured to calculate and display information regarding bone blocks to facilitate clinical decision-making with regard to surgical procedures. Referring to FIG. 18, a bone block may be utilized to restore the glenoid width 180 to the healthy glenoid width 182, and the controller 110 may be configured to determine a restoration dimension 250 to restore the glenoid width 180 to the healthy glenoid width 182. The restoration dimension 250 may be automatically determined by the controller 110, or manually determined by the user 116, and may be used as the dimension of a bone block for shoulder surgery planning. The controller 110 may be configured to automatically determine the restoration dimension 250 by comparing the glenoid width 180 to the healthy glenoid width 182. For example, the restoration dimension 250 may be determined by subtracting the glenoid width 180 from the healthy glenoid width 182. In some implementations, the restoration dimension 250 may be based on the width necessary to restore the glenoid width 180 to a certain percentage of the healthy glenoid width 182, for example, at least 85, 90, 95, 100, 105, or 110%. This percentage may be adjusted for each patient based on the patient shoulder anatomy and what restoration dimension 250 may be required to classify the lesion object 210 as an on-track lesion. The restoration dimension 250 may be provided as an output by the controller 110 based on any relationship between the glenoid width 180 and the healthy glenoid width 182. In some implementations, the restoration dimension 250 may be limited by a bone block size based on the anatomical features of patient shoulder anatomy, such as the size of the coracoid. The controller 110 may be configured to determine a reconstructed glenoid width 252 based on the restoration dimension 250 and the glenoid width 180.

Further, a reconstructed glenoid track width 256 may be determined based on the restoration dimension 250 and the glenoid width 180. As disclosed above in Section IV and shown in FIG. 18, the reconstructed glenoid track width 256 may be determined by multiplying the diameter of the best fit circle object 184 by a threshold value (e.g. 83%, 85%, 87%, or 90%), subtracting the glenoid bone loss measure, and adding the restoration dimension 250. Similarly, the reconstructed glenoid track width 256 may be determined by multiplying the reconstructed glenoid width 252 by a threshold value. Other methods of determining the reconstructed glenoid track width 256 may be used, and this disclosure is not limited to those specifically discussed herein.

Additionally or alternatively, the controller 110 may be configured to allow the user 116 to interact with the restoration dimension 250 using the user input device 122 until it is suitable for the patient 112. Referring to FIG. 19, the user 116 may adjust the restoration dimension 250 and allow the controller 110 to update renderings of the 3D models, the lesion object 210, and the glenoid track object 170. This process may be repeated one or more times until the lesion object 210 is resolved (e.g., the lesion object 210 is classified as an on-track lesion). The restoration dimension 250 may be recorded to facilitate future surgical planning, which may include bone grafting and/or other surgical procedures not disclosed herein. As disclosed above, the controller 110 may also calculate the reconstructed glenoid width 252 and the reconstructed glenoid track width 256 based on the restoration dimension 250.

The restoration dimension 250 may be a measurement, a visualization, or any other indication of a bone block reconstruction dimension. If the lesion is classified as a near-track lesion in the original status, the first signal 296 would indicate near-track, but if the reconstruction would bring the lesion on-track, the second signal 298 would indicate an on-track lesion. Further, as shown in FIG. 19 the restoration dimension 250 may be visualized as the reconstructed glenoid width 252, a reconstructed glenoid track 254, and/or the reconstructed glenoid track width 256, which are based on the glenoid track object 170 modified by the restoration dimension 250.

Referring to FIGS. 17B and 17C, the controller 110 may be configured to determine a distance to dislocation (DTD) metric based on the distance between the medial border 204 of the lesion object 210 and the medial border 204 of the glenoid track object 170. The value is negative if the most medial border 204 of the lesion object 210 is more medial than glenoid track object 170. The controller 110 may further be configured to determine a relative track value which is the relative value of the DTD to the glenoid track width represented by equation - 100 - (DTD/Glenoid track width). The relative track value provides a qualitative rating that allows a user to easily understand the recurrent dislocation risk. With reference to Figure 17B, the pre-reconstruction anatomy has a DTD value of less than 0 mm, and a post-reconstruction anatomy has a DTD value of 5.3 mm.

With further reference to Figure 17C, the controller 110 may be configured to display the relative track value before and after reconstruction (e.g., after use of a bone block). For example, in the example shown, the relative track value before reconstruction is displayed as 121%, which is indicated as marker A. The relative track value after reconstruction is displayed as 80%. The value of 80% would be qualitatively characterized as a near-track lesion object, whereas the value of 121% would be qualitatively characterized as an off-track lesion object. In other words, the controller 110 may provide quantitative and/or qualitative assessment of the lesion object to facilitate clinical decision-making with regard to surgical procedures.

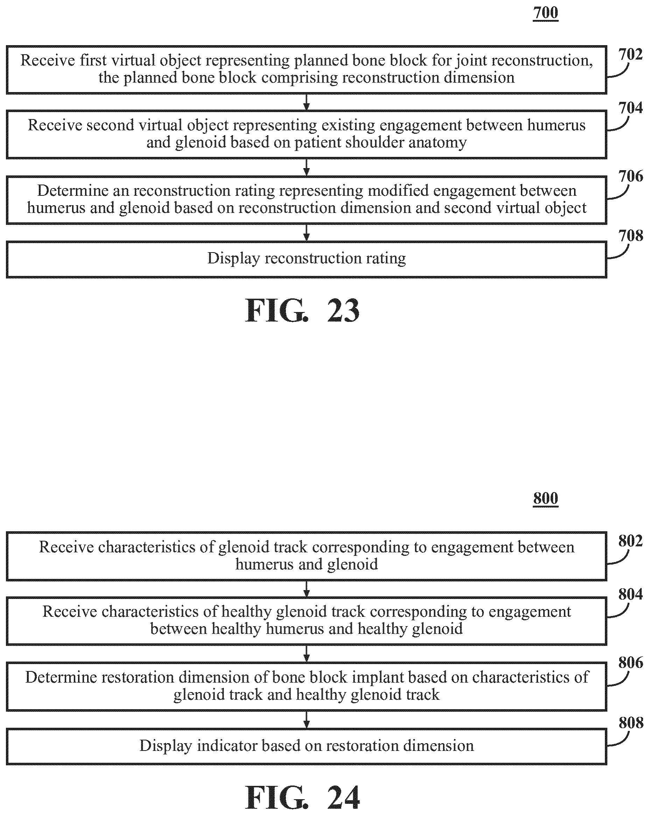

Having described the surgical planning system 100, a method 700 of visualizing patient shoulder anatomy using the surgical planning system 100 (i.e., the controller 110) is illustrated in FIG. 23. The method 700 may be executed pre-operatively or intra-operatively to plan one or more shoulder surgeries for the patient 112. At step 702, the controller 110 may receive a first virtual object representing a planned bone block for joint reconstruction, the planned bone block including the restoration dimension 250, as described in Section IX. At step 704, the controller 110 may receive a second virtual object representing engagement between a humerus and a glenoid based on the patient shoulder anatomy, wherein the second virtual object is the glenoid track object 170 and is described in Section IX. At step 706, the controller 110 may determine a reconstruction rating representing modified engagement between a humerus and a glenoid based on the restoration dimension 250 and glenoid track object 170, as described in Section IX. At step 708, the controller 110 may cause the display to show the reconstruction rating, as shown in FIG 19.

Another method 800 of assessing patient shoulder anatomy using the surgical planning system 100 (i.e., the controller 110) is illustrated in FIG. 24. The method 800 may be executed pre-operatively to plan one or more shoulder surgeries for the patient 112. At step 802, the controller 110 may receive characteristics of a glenoid track corresponding to engagement between a humerus and a glenoid, as described in Section IX. At step 804, the controller 110 may receive characteristics of a healthy glenoid track corresponding to engagement between a healthy humerus and a healthy glenoid, as described in Section IX. At step 806, the controller 110 may determine a restoration dimension 250 of a bone block implant based on characteristics of the glenoid track and the healthy glenoid track, or the characteristics of the glenoid track object 170 and the healthy glenoid track object 172, as described above in Section IX. At step 808, the controller 110 may cause the display to show an indicator based on the restoration dimension 250, also referred to as the reconstruction dimension 250, as shown in FIG. 19.

Any of the above methods may be executed independently, or concurrently with the other disclosed methods, and any of the presented steps may be executed in any combination or order not disclosed herein. Any of the above methods may be carried out by the surgical planning system 100 to aid the user 116 in performing an assessment of the patient glenohumeral joint and/or determining the likelihood of a lesion having an impact on joint engagement.

### X. Determination of Apex-Based Lines

The location of the lesion object 210 on the humeral head portion 146 may impact the engagement of a glenohumeral joint. In the implementations described above, the engagement of the glenohumeral joint was represented as the track engagement of the glenoid track object 170 on the humeral head portion 146. In other implementations, the engagement of the glenohumeral joint may be analyzed without the glenoid track object 170 and/or the healthy glenoid track object 172. Instead, the location of the lesion object 210 relative to certain anatomical points of interest on the humeral head portion 146 may be utilized to assess the likelihood of impact on joint engagement by the lesion object 210. To accurately assess the relative location of the lesion object 210, a set of reference objects may be utilized to compare the location of the lesion object 210 to known locations of the reference objects. For example, the controller 110 may be configured to cause the display 120 to show a 3D model, the reference objects, and the lesion object 210. The user 116 may then view the 3D model, the lesion object 210, and the reference objects on the display 120 to make a qualitative assessment of (1) the likelihood of impact on joint engagement by the lesion object 210, (2) the severity of the lesion object 210, and (3) whether shoulder surgical planning is necessary. The reference objects may include anatomical points of interest, a first set of lines, and/or a second set of lines, as further described below.

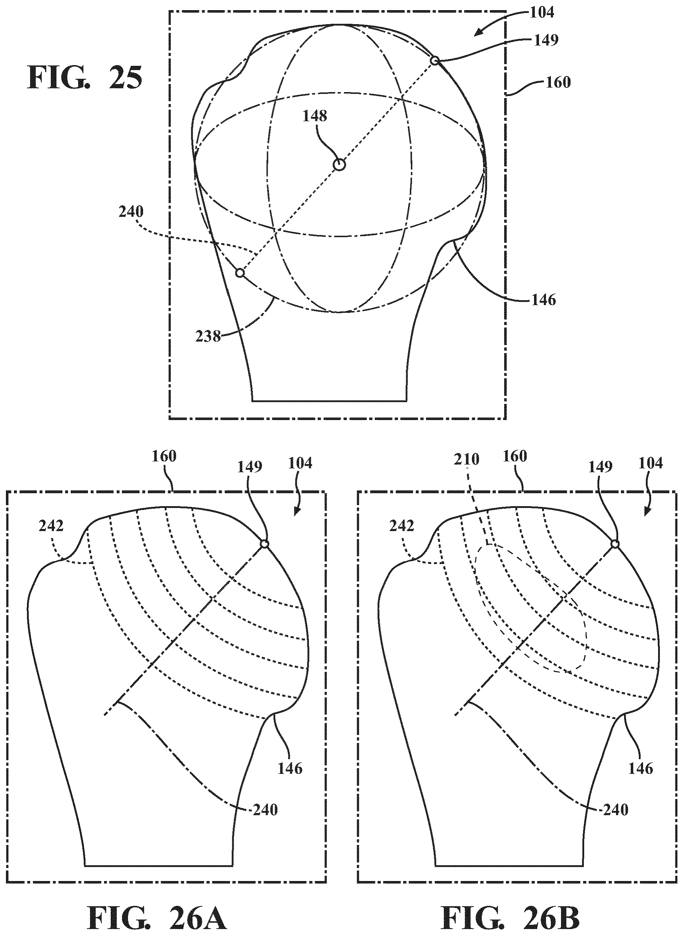

The anatomical points of interest may include a humeral neck axis, the humeral head center, and a humeral head apex, which may be represented by a humeral neck axis line 240, a humeral head center point 148, and a humeral head apex point 149. Referring to FIGS. 25-27B, the controller 110 may be configured to determine the location of the anatomical points of interest relative to the humeral head portion 146 by generating a humerus sphere 238 which approximates the size and shape of the humeral head portion 146. The humeral head center point 148 may be based on the center of the humerus sphere 238. The humerus sphere 238 may be fitted to the humeral head portion 146 by approximating various anatomical features such as the width, height, or shape of the humeral head portion 146, or other measurements not described herein. In some implementations, the controller 110 may be configured to apply a machine learning algorithm, such as a convolutional neural network, to the humerus 3D model 104 to automatically generate virtual objects representing relevant anatomical points of interest.

Referring specifically to FIG. 25, the humeral neck axis line 240 may be based on an axis extending through the humeral head center point 148 and the humeral head apex point 149. The controller 110 may be configured to automatically determine the location of the humeral head apex point 149 and the humeral head center point 148, or the location may be manually determined by the user 116. The location of the humeral head apex point 149 may be determined based on the location of the humeral head center point 148. The controller 110 may be configured to first generate the humerus sphere 238, then determine the humeral head center point 148 based on the humerus sphere 238, then determine the humeral head apex point 149 based on the humerus 3D model 104, and then determine the humeral neck axis line 240 based on the humeral head center point 148 and the humeral head apex point 149. In some implementations, the controller 110 may determine other anatomical points of interest or determine previously mentioned points of interest in a different order.

Referring specifically to FIGS. 26A-B, the controller 110 may be configured to generate a set of reference objects such as one or more sets of lines as described above. The first set of lines may be referred to as planar lines 242, horizon lines, or mediolateral lines, and the controller 110 may be configured to generate the planar lines 242 relative to the humeral head portion 146. As a result, the planar lines 242 are geodesic lines which represent an over-the-surface region on the humeral head portion 146. As shown in FIG. 26A, the planar lines 242 may be perpendicular to the humeral neck axis line 240 and represent the circumference of the humeral head portion 146 at set positions. The planar lines 242 may be spaced axially along the humeral neck axis line 240 at regular intervals and include at least one line. The intervals between each planar line 242 may be consistent with each other interval or different from other intervals.

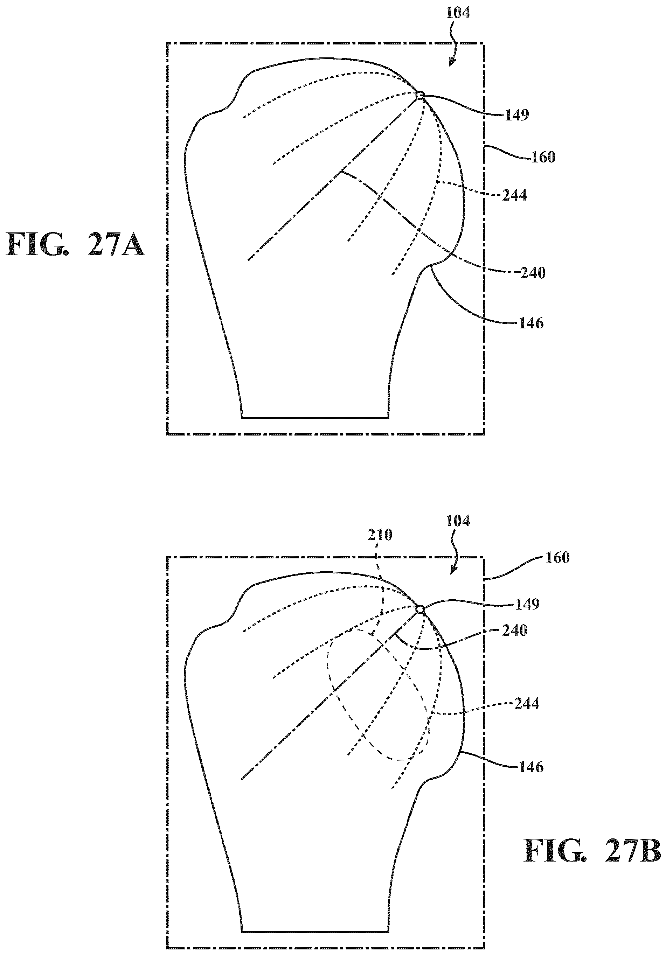

Referring specifically to FIGS. 27A-B, the second set of lines may be referred to as clockface lines 244 or superior inferior lines, and the controller 110 may be configured to generate the clockface lines 244 relative to the humeral head portion 146. As a result, the clockface lines 244 are also geodesic lines which represent an over-the-surface region on the humeral head portion 146. As shown in FIG. 27A, the clockface lines 244 may be perpendicular to the planar lines 242 and intersect the humeral head apex point 149. The clockface lines 244 may be spaced radially about the humeral neck axis line 240 at regular intervals and include at least one line. The intervals between each clockface line may include an angle, which may be consistent between each clockface line, or different between each clockface line, or the intervals may be different from each of the other intervals.

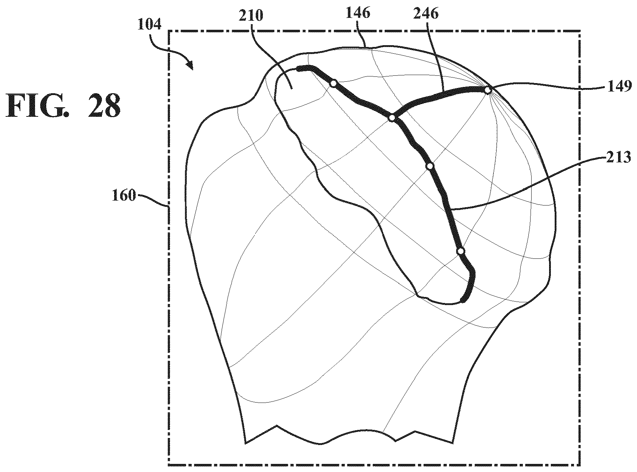

To determine the severity of the lesion object 210, the controller 110 may determine one or more quantitative measurements to quantify the location of the lesion object 210, such as the location of the lesion object 210 relative to the humeral head apex point 149. The presence and/or location of the lesion object 210 may be identified by the controller 110 and/or the user 116 using the same methods disclosed above. For example, the controller 110 may apply the segmentation algorithm or the deep learning network to the image data of the patient shoulder anatomy, use the healthy surface prediction algorithm, and utilize post processing algorithms to define a boundary of the lesion object 210 as described above. Referring to FIG. 28, the controller 110 may be configured to determine at least a geodesic distance 246 between the medial border 213 of the lesion object 210 and the humeral head apex point 149 (or the user 116 may manually measure the geodesic distance 246). In the illustrated implementation, the geodesic distance 246 represents a distance over the surface of the humeral head portion 146. A larger geodesic distance 246 usually indicates a lower severity lesion object 210, while a smaller geodesic distance 246 tends to indicate a greater severity lesion object 210. Alternatively or additionally, the controller 110 may be configured to determine at least a Euclidean distance between the medial border 213 of the lesion object 210 and the humeral head apex point 149 (or the user 116 may manually measure the Euclidean distance). Similarly, a larger Euclidean distance usually indicates a lower severity lesion object 210, while a smaller Euclidean distance usually indicates a greater severity lesion object 210. In some implementations, the controller 110 may be configured to determine and/or approximate the geodesic distance 246 based on a Euclidean distance and the radius of the humerus sphere 238.

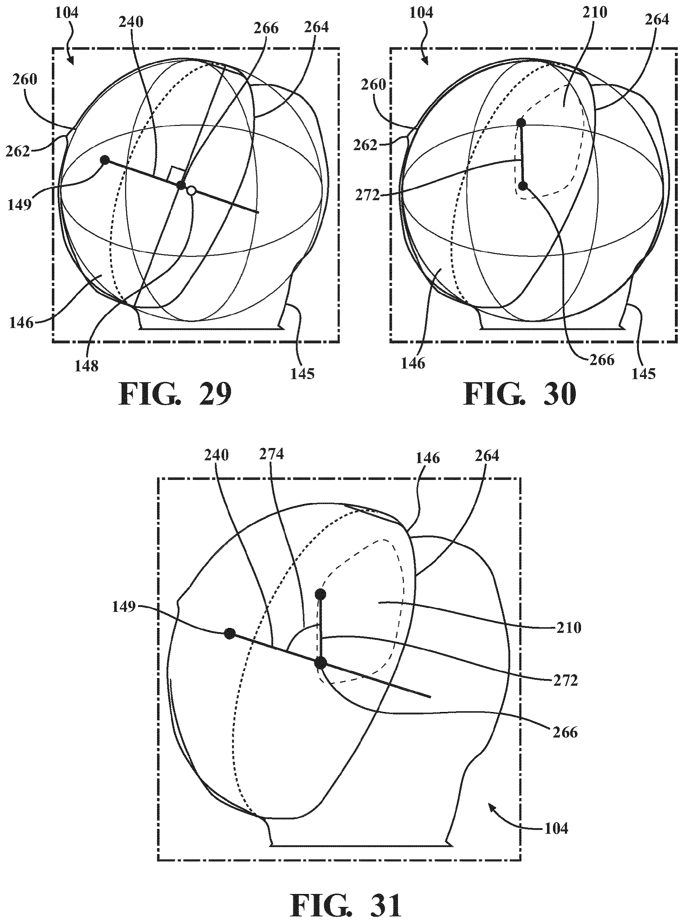

Additionally, the controller 110 may be configured to determine an angle 274 representative of the amount of possible rotation of a humerus relative to a glenoid, such as the amount of flexion and/or adduction. The amount of possible rotation may be defined as the rotation before the joint engagement is impacted by the lesion object 210, or as the limit of the range of motion for the patient shoulder anatomy. Referring to FIG. 29, the controller 110 may be configured to fit an articular sphere 262 to an articular surface 260 of the humeral head portion 146 to approximate an articular surface of the humerus. The articular sphere 262 may be fit to the articular surface 260 of the humeral head portion 146 and be bounded by the transition area between a humeral head surface and a humeral neck. Additionally, the controller 110 may be configured to automatically exclude the lesion object 210 from the articular surface, or a user 116 may manually interact with the user input device 122 to instruct the controller 110 to remove the lesion object 210 from the process of fitting the articular sphere 262. After fitting the articular sphere 262, the controller 110 may be configured to fit an articular margin plane 264 to an articular margin of a humeral head. The controller 110 may be configured to detect the articular margin plane with curvature analysis of the humeral head portion 146. The articular margin plane 264 may be fit based on points of greatest curvature on the humeral head portion 146, or a surface selection of triangles at the points of greatest curvature. The points of greatest curvature may be identified on a statistical shape model, or on pre-determined coordinates of structures, such as a humeral neck plane. In some implementations, the articular margin is identified as the point at which the humeral neck portion 145 attaches to the humeral head portion 146. The controller 110 may be configured to identify the humeral neck axis line 240 based on the articular margin plane 264, where the humeral neck axis line 240 is perpendicular to the articular margin plane 264, passes through the humeral head center point 148, and intersects the humeral head portion 146. In some implementations, the humeral head apex point 149 may be identified as the intersection between the humeral neck axis line 240 and the humeral head portion 146 medially.

After determining the humeral neck axis line 240, the controller 110 may be configured to identify a lesion line 272. Still referring to FIG. 29, the controller 110 may be configured to determine an articular margin center 266 based on the humeral neck axis line 240 and the articular sphere 262. As shown in FIG. 30, the lesion line 272 may be bounded by the articular margin center 266 and any point on the medial boundary of the lesion object 210.

To determine an angle 274 representative of the amount of possible rotation of a humerus relative to a glenoid, the controller 110 may be configured to generate the angle 274 between the humeral neck axis line 240 and the lesion line 272. Referring to FIG. 31, the angle 274 may be determined in relation to the humeral head portion 146. The controller 110 may be configured to cause the display to show the angle 274 relative to the humeral head portion 146 and the humerus 3D model 104. The angle 274 may be determined in addition to the geodesic distance 246, or it may be the sole quantitative measurement, and both measurements may be displayed in conjunction or in isolation. Other quantitative measurements to quantify the location of the lesion object 210 may exist, and those not specifically disclosed herein may still be utilized. In some implementations, multiple points on the medial boundary of the lesion object 210 may be chosen, and multiple angles may be determined between the alternate lesion line and the humeral neck axis line 240. After being determined, multiple angles may be displayed on the display 120 along with the humerus 3D model 104.

### XI. Display of Apex-Based Lines

The controller 110 may be configured to visualize the lesion object 210 relative to patient shoulder anatomy on the display 120. The controller 110 may be configured to cause the display 120 to show virtual objects representing the anatomical points of interest identified above relative to the humerus 3D model 104, the humeral head portion 146, and the lesion object 210. The controller 110 may also be configured to mark, highlight, or otherwise emphasize the location of these anatomical points of interest on the display 120. The controller 110 may record all or part of the visualization displayed on the display 120 to facilitate surgical planning.

The planar lines 242 and/or the clockface lines 244 may be represented as a set of lines, a set of planes, or any other virtual objects on the display 120. The controller 110 may be configured to cause the display 120 to show the planar lines 242 and/or the clockface lines 244 on the surface of the humeral head portion 146. Once the planar lines 242 are displayed, they may indicate a relative mediolateral position of anatomical features of the humeral head portion 146. Similarly, once the clockface lines 244 are displayed, they may indicate a relative superior-inferior position of anatomical features of the humeral head portion 146. As shown in FIGS. 26B and 27B, the humerus 3D model 104 may be displayed with the lesion object 210 relative to the humeral head apex point 149 and the planar lines 242 and/or the clockface lines 244 relative to the humeral head portion 146.

Referring back to FIGS. 26B and 27B, the user 116 may qualitatively assess the severity of the lesion object 210 on the surface of the humeral head portion 146 by viewing the position of the lesion object 210 relative to the planar lines 242 and/or the clockface lines 244. For example, the lesion object 210 may be considered less severe when it is located more laterally on the humeral head portion 146. By referencing the planar lines 242, the user 116 may consider that if the lesion object 210 is lateral of a threshold of the planar lines 242, then the lesion object 210 does not pose a risk. However, if the lesion object 210 is medial of the threshold of the planar lines 242, the user 116 may consider the lesion object 210 to necessitate surgery. Similarly, if the lesion object 210 is superior of a threshold of the clockface lines 244, the user 116 may conclude that the lesion object 210 is severe. If the lesion object 210 is inferior of the threshold of the clockface lines 244, the user 116 may conclude that the lesion object 210 likely does not impact track engagement and does not necessitate surgery. The threshold of the planar lines 242 and/or the clockface lines 244 may be adapted for each patient, or each configuration of the planar lines 242 and the clockface lines 244. Each user 116 may have a personal preference for the threshold, or it may be standard across patients. Referring to FIGS. 28 and 31, the quantitative measurements that quantify the location of the lesion object 210 may serve to confirm the assessment of the user 116. For example, there may be threshold values for the geodesic distance 246 and the angle 274 that cannot be surpassed if the lesion object 210 does not impact joint engagement. Similarly, there may be standard threshold values across patients, or patients may have threshold values specific to their anatomy.

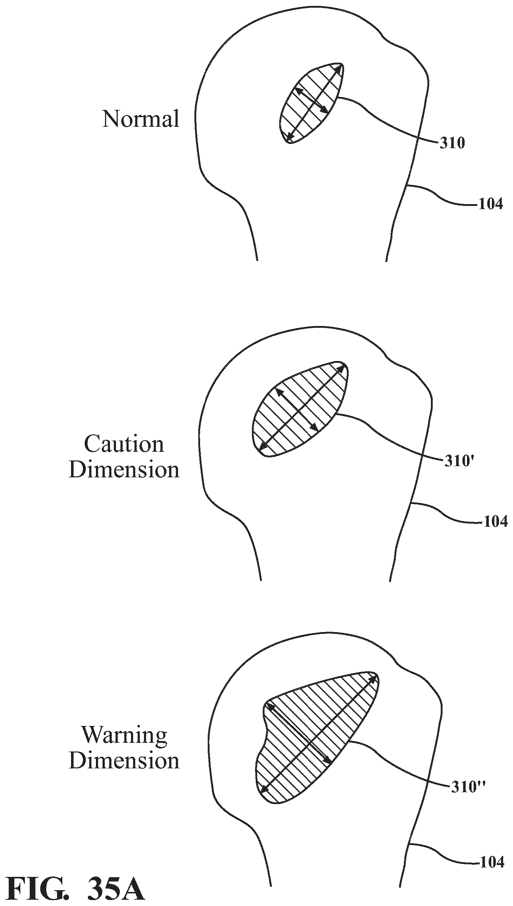

With respect to Figure 35A, the controller 110 may be configured to output a dimension indicator indicative of the relative dimensions of the lesion object 310, 310', 310" with respect to certain thresholds. In one potential configuration, the controller 110 may be configured to divide the 215 width of the lesion object 310 with the radius of the best fit sphere (see FIG. 25-27B) to obtain a relative width dimension measurement and compare that to one or more lesion width thresholds (normal width threshold, caution width threshold, and warning width threshold). Similarly, the controller 110 may be configured to divide the length 217 of the lesion object 310 with the radius of the best fit sphere to obtain a relative length dimension measurement and compare that to one or more lesion length thresholds (normal length threshold, caution length threshold, and warning length threshold). The controller 110 may be configured to provide the dimension indicator based on one or more of the dimensions and one or more the thresholds. For example, the controller 110 may provide the dimension indicator if one or more of the relative dimensions exceed at least one of the pertinent thresholds.

It should be appreciated that the dimension indicator can take various forms. For example, the lesion object 310 may be differentiated from the rest of the humerus 3D model by rendering a difference in color, shading, pattern, or any other visual difference on the display 120 to indicate the dimension indicator, green if the lesion object 310 does not exceed any normal thresholds, yellow if the lesion object 310 exceeds only caution thresholds, and red if the lesion object 310 exceeds at least one warning threshold.

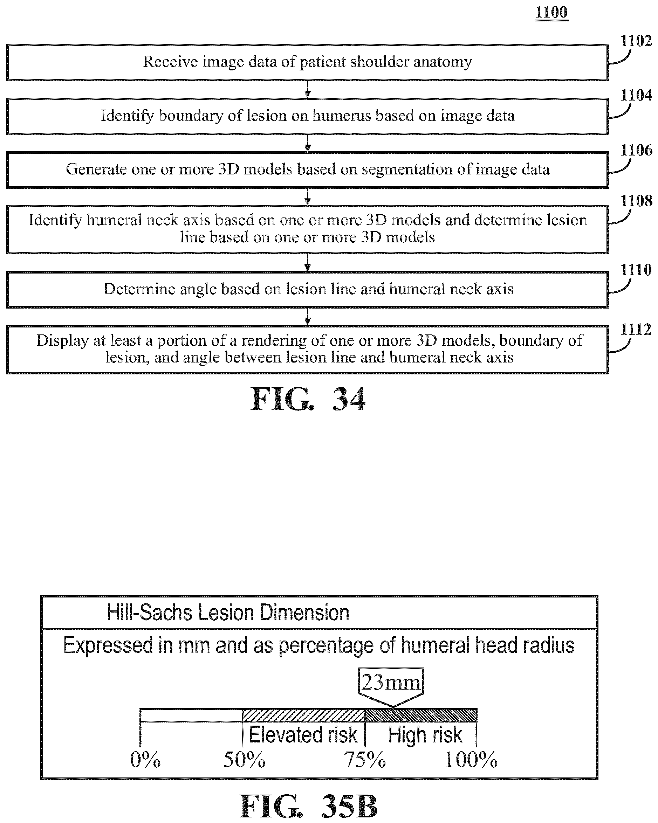

With respect to Figure 35B, the controller 110 may be configured to output a dimension indicator indicative of the relative dimension of the lesion object with respect to certain thresholds in on a sliding scale. As described above, in one potential configuration, the controller 110 may be configured to compare the lesion object dimensions with one or more thresholds and generate a lesion dimension indicator. The dimension indicator may be indicative of the risk of recurrence of dislocation. Considering the dimensions of lesion object 310" of the bottom figure of FIG. 35A, the controller 110 may cause the display 120 to show a lesion dimension indicator which may indicate the largest dimension of the lesion object in mm (typically the length) and a slider with percentage values relative to the humeral head radius. In the example, the lesion object is classified as having a high risk of recurrent dislocation because it has length dimension of 23 mm, which is approximately of 80% of the humeral head radius. In the provided screenshot, there are graduations and thresholds indicated, delineating between those lesion objects having an elevated risk (corresponding a percentage of humeral head radius being between 50 and 75%) and those lesion objects having a high risk (corresponding to a percentage of humeral head radius being between 75 and 100%). As shown in FIG. 35B, the lesion position indicator may be shown as a sliding scale. In some implementations, the lesion dimension indicator may be a numerical scale to indicate severity, an image, or other signal to provide the dimension. The controller 110 may be configured to cause the display 120 to show the lesion dimension indicator to facilitate surgical planning. The configuration shown in FIG. 35B effectively summarizes the analysis of the dimension of the lesion object, which advantageously saves time for the user in assessing the condition of a glenohumeral joint. The automatic generation and display of the lesion dimension indicator allows for accurate and concise analysis to be provided to the user 116 in planning shoulder surgery to address the state of the patient shoulder anatomy.

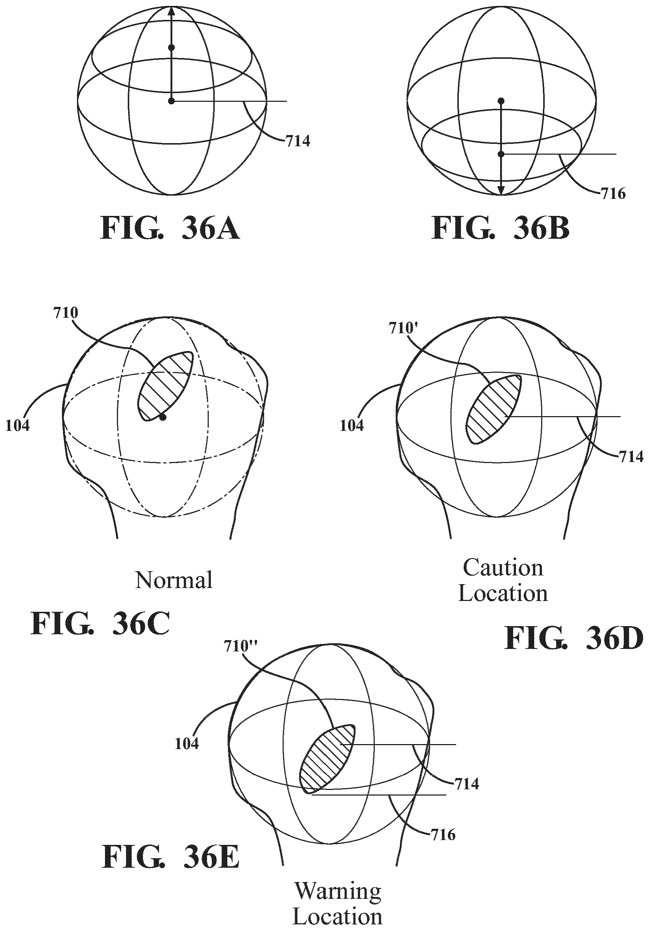

With respect to Figure 36A-36E, the controller 110 may be configured to output a position indicator indicative of the relative position of the lesion object 710, 710', 710" with respect to certain reference features. In one potential configuration, the controller 110 may be configured to compare the lesion object 710, 710', 710" with one or more reference planes). These reference planes may be positioned at different levels on the superior-inferior axis to indicate two levels of risk of a recurrent dislocation related to the inferior extent of the Hill-Sachs lesion. By relating the plane position of these reference planes to a factor relative to the humeral head radius allows the controller 110 to normalize the position to the humeral head size (as could an angle; however detecting whether the lesion object intersects and/or is below a plane is more straight-forward).

More particularly, with reference to Figure 36A, a first reference plane 714 may be defined at a position that is at the center of the humeral head along the superior-inferior axis. With reference to FIG. 36B, a second reference plane 716 may be created that is 50% of the humeral head radius distance below the center of the humeral head and along super-inferior axis. It should be appreciated that other plane locations may be used other than the center of humeral head and 50% of the humeral head radius distance below the center of the humeral head, such as at 25, 30, 35, 40, 45, 55, 60 % or intermediate values disposed therebetween.

These reference planes may be positioned at different levels on the superior-inferior axis to indicate two levels of risk of a recurrent dislocation related to the inferior extent of the Hill-Sachs lesion. By relating the plane position of these reference planes to a factor relative to the humeral head radius allows the controller 110 to normalize the position to the humeral head size (as could an angle; however, detecting whether the lesion object intersects and/or is below a plane is more straight-forward). The controller 110 is configured to provide an indicator if any position of the lesion extends beyond the defined reference planes.

With respect to Fig. 36C, because the lesion object 710 does not intersect the first reference plane 714, the position indicator may be indicative that the lesion object 710 is in a normal location because no portion of the lesion object 710 is below the plane 714 at the center of the humeral head. With respect to Fig. 36D, if the lesion object 710' intersects the first reference plane 714, the position indicator may be indicative that the lesion object 710' is in a caution location. With respect to Fig. 36E, if the lesion object 710" intersects the second reference plane 716, the position indicator may be indicative that the lesion object 710" is in a warning location.

It should be appreciated that the position indicator can take various forms. For example, the lesion object 710, 710', 710" may be differentiated from the rest of the humerus 3D model by rendering a difference in color, shading, pattern, or any other visual difference of the lesion objects 710, 710', 710" on the display 120 to provide the position indicator, green if the lesion object 710 does not intersect the first reference plane 714, yellow if the lesion object 710' intersects only the first reference plane 714, and red if the lesion object 710" intersects the second reference plane 716.

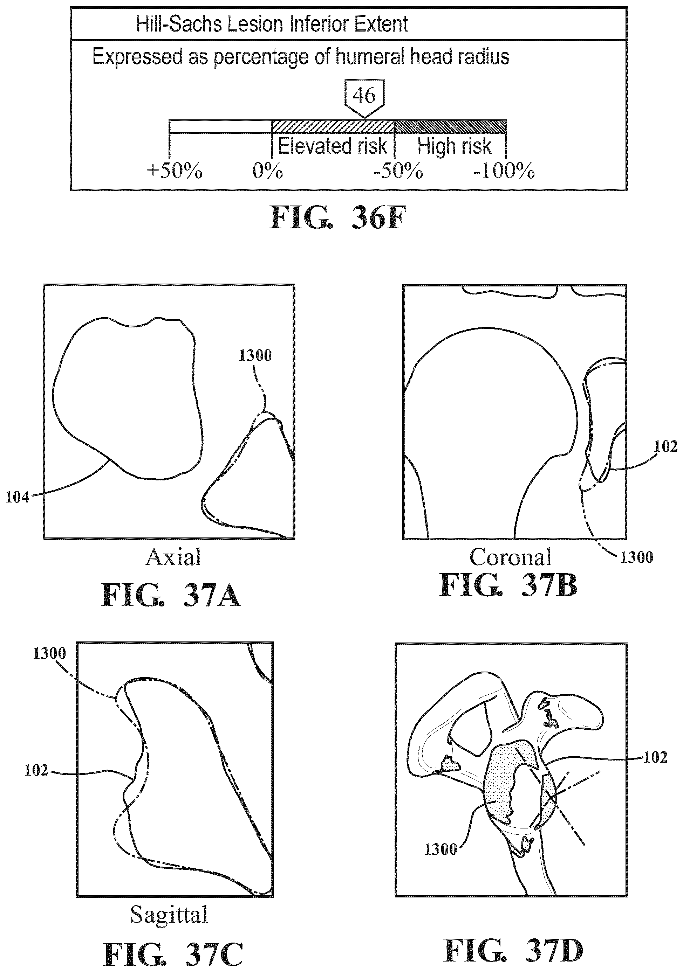

With respect to Figure 36F, the controller 110 may be configured to output a position indicator indicative of the relative position of the lesion object with respect to certain reference features in on a sliding scale. In one potential configuration, the controller 110 may be configured to compare the lesion object 710' with one or more reference planes, and generate a lesion position indicator based on the comparison. Referring to FIG. 36F and considering the position of lesion object 710' of FIG. 36D, the controller 110 may cause the display 120 to show a lesion position indicator which may indicate the inferior extent of the lesion object 710'. In the example, the lesion object is classified as having an elevated risk of recurrent dislocation because it has an inferior extent of 46% of the humeral head radius. In the provided screenshot, there are graduations and thresholds indicated, delineating between those lesion objects having an elevated risk (corresponding to a percentage of humeral head radius being between 0 and -50%) and those lesion objects having a high risk (corresponding to a percentage of humeral head radius being between -50 and 100%). As shown in FIG. 36F, the lesion position indicator may be shown as a sliding scale. In some implementations, the lesion position indicator may be a numerical scale to indicate severity, an image, or other signal to provide a degree of inferior extent of the lesion object. The controller 110 may be configured to cause the display 120 to show the lesion position indicator to facilitate surgical planning. The configuration shown in FIG. 36F effectively summarizes the analysis of the position of the lesion object, which advantageously saves time for the user in assessing the condition of a glenohumeral joint. The automatic generation and display of the lesion position indicator allows for accurate and concise analysis to be provided to the user 116 in planning shoulder surgery to address the state of the patient shoulder anatomy.

As described above and throughout the disclosure, the controller110 may be configured to render various aspects of the glenoid 3D model 102, humerus 3D model 104, the premorbid humerus 3D model (not shown), and/or the premorbid glenoid 3D model 800 on the display 120. It should be appreciated that the glenoid 3D model 102 is the model of the patient's glenoid generated from segmentation of an image of the patient's glenoid, whereas the premorbid glenoid model 800 is a model generated to predict the patient's anatomy before the pathology occurred. With respect to Figures 37A-37C, the controller 110 may also be configured to render 2D views of the humerus and/or glenoid from the image data relative to the premorbid humerus 3D model (not shown) and/or the premorbid glenoid 3D model 103 and relative to the glenoid 3D model 102 and the humerus 3D model 104 in various 2D views, such as an axial view, a coronal view, and a sagittal view. For example, with reference to the axial 2D view of FIG. 37A, the controller 110 is configured to render image data of the humerus and the glenoid relative to outlines of the premorbid glenoid model 1300 and relative to outlines of the glenoid 3D model 102 and humerus 3D model 104. Furthermore, with respect to the coronal 2D view of FIG. 37B, the controller 110 is configured to render image data of the humerus and the glenoid relative to outlines of the premorbid glenoid model 1300 and relative to outlines of the glenoid 3D model 102 and humerus 3D model 104. Finally, with respect to the sagittal 2D view of FIG. 37C, the controller 110 is configured to render image data of the glenoid relative to outlines of the premorbid glenoid model 1300 and the glenoid 3D model 102. For reference, a 3D perspective view of the glenoid 3D model 102 is shown in FIG. 37D with aspects of the premorbid glenoid model 1300 being shown in the hashed pattern.

In one implementation, the display 120 may be configured to show the humerus 3D model 104, the humeral head portion 146 with the planar lines 242, the clockface lines 244, the lesion object 210, and the humeral head apex point 149. In addition, the display 120 may also display one or more quantitative measurements including the geodesic distance 246 and the angle 274. The geodesic distance 246 may be shown as a virtual line, plane, number, or any other representation of the distance between the humeral head apex point 149 and the medial border 213 of the lesion object 210. The angle 274 may be illustrated with an angle as shown in FIG.31, or it may be shown as a number or any other representation of the possible degrees of rotation of a humerus. Many combinations of anatomical features, the planar lines 242, the clockface lines 244, and quantitative measurements may be shown by the display 120. For example, the controller 110 may be configured to cause the display 120 to render one or more 3D models (such as the glenoid 3D model 102 and the humerus 3D model 104) along with the lesion object 210, the planar lines 242, the clockface lines 244, and/or the geodesic distance 246.

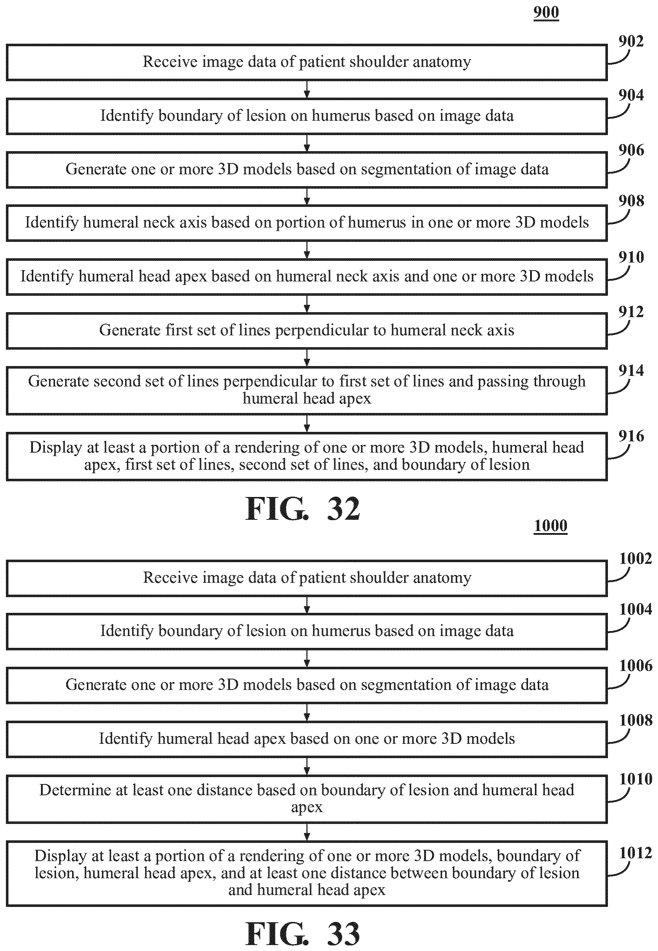

A method 900 of visualizing the patient shoulder anatomy, the planar lines 242, and the clockface lines 244 involving the controller 110 is illustrated in FIG. 32. As with the other methods provided herein, the method 900 may be executed pre-operatively or intra-operatively to plan one or more shoulder surgeries for the patient 112. At step 902, the controller 110 may receive image data of the patient shoulder anatomy, such as image data including a humerus of the patient 112. At step 904, the controller 110 may identify a boundary of a lesion on the humerus based on the image data, as described above in Section VII. At step 906, the controller 110 may generate one or more 3D models based on a segmentation of image data, as described in Section II. At step 908, the controller 110 may identify a humeral neck axis based on a portion of the humerus in the one or more 3D models, as described in Section IV. At step 910, the controller may identify a humeral head apex based on the humeral neck axis and the one or more 3D models. At step 912, the controller 110 may generate a first set of lines 242 perpendicular to the humeral neck axis, as described in Section X. At step 914, the controller 110 may generate a second set of lines 244 perpendicular to the first set of lines 242 and passing through the humeral head apex, described above in Section X. At step 916, the controller 110 may cause the display to display (simultaneously or independently) a portion of a rendering of the one or more 3D models, the humeral head apex, the first set of lines 242, the second set of lines 244, and the boundary of the lesion, shown in FIG.28.

A method 1000 of visualizing patient shoulder anatomy and measurement information involving the clockface and horizon lines using the controller 110 is illustrated in FIG. 33. At step 1002, the controller 110 may receive image data of patient shoulder anatomy, such as image data of a humerus of the patient 112. At step 1004, the controller 110 may identify the boundary of the lesion on a humerus, as described above in Section VII. At step 1006, the controller 110 may generate one or more 3D models based on a segmentation of image data, as described in Section II. At step 1008, the controller 110 may identify a humeral head apex based on the one or more 3D models, as described above in Section X. At step 1010, the controller 110 may determine at least one geodesic distance 246 based on a boundary of a lesion and the humeral head apex, as described above in Section X. At step 1012, the controller 110 may cause the display 120 to show (simultaneously or independently) the one or more 3D models, the boundary of the lesion, the humeral head apex, and the geodesic distance 246 between the boundary of the lesion and the humeral head apex, as shown in FIG. 28 and described in Section XI.

Another method 1100 of visualizing patient shoulder anatomy and measurement information involving the clockface and horizon lines using the controller 110 is illustrated in FIG. 34. At step 1102, the controller 110 may receive image data of the patient shoulder anatomy, such as image data of a humerus of the patient 112. At step 1104, the controller 110 may identify a boundary of the lesion on the humerus based on the image data, as described in Section II. At step 1106, the controller 110 may generate one or more 3D models based on a segmentation of image data, as described in Section II. At step 1108. the controller 110 may identify a humeral neck axis based on one or more 3D models and determine a lesion line based on the one or more 3D models, as described above in Section X. At step 1110, the controller 110 may determine an angle based on the lesion line and humeral neck axis, as described in Section X. At step 1112, the controller may cause the display to show (simultaneously or independently) at least a portion of a rendering of the one or more 3D models, a boundary of a lesion, and the angle between the lesion line and the humeral neck axis, as described in Section X and shown in FIG 31.

Any of the above methods may be executed independently, or concurrently with the other disclosed methods, and any of the presented steps may be executed in any combination or order not disclosed herein. Any of the above methods may be carried out by the surgical planning system 100 to aid the user 116 in performing an assessment of the patient glenohumeral joint and/or determining the likelihood of a lesion having an impact on joint engagement.

It is to be understood that the controller 110 may be configured to cause the display 120 to show renderings of the 3D model and any of the above-described anatomical characteristics or virtual objects relative to renderings of 3D models. These features may be displayed individually or in combination with each other. The user 116 may interact with the user input device 122 to select what is displayed on the display 120, and all possible configurations of the anatomical features determined by the controller 110 may be shown on the display 120. For example, the planar and the clockface lines 242, 244 may be shown relative to the lesion object 210 and the 3D model of the humeral head portion 146 in combination with the glenoid track line 174, the soft tissue insertion line 178, and the glenoid width 180, or they may be shown only in relation to the lesion object 210 and renderings of a 3D model. This disclosure does not limit the system 100 to any combination of display configurations, and any of the described display configurations may be combined with other possible configurations. Similarly, the controller 110 may be configured to cause the display 120 to show any of the above-described elements or any of the views presented in the Figures. The controller 110 may cause the display 120 to show a multitude of the views at once, or any combination of the views presented in the Figures. For example, the controller 110 may be configured to simultaneously display two windows side by side, one side showing the 3D model and the glenoid track object 170 and the other side showing the 3D model and the planar lines 242. The multitude of windows may be shown on one display 120 or multiple displays 120. It is to be further understood that the surgical planning system 100 may be utilized to evaluate many types of patient shoulder anatomy and is not limited to the exemplary configuration described herein.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit, such as controller 110. Computer-readable medlia may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can include RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. Accordingly, the terms "processor" and "processing circuitry," as used herein may refer to any of the foregoing structures or any other structure suitable for implementation of the techniques described herein.

It will be further appreciated that the terms "include," "includes," and "including" have the same meaning as the terms "comprise," "comprises," and "comprising." Moreover, it will be appreciated that terms such as "first," "second," "third," and the like are used herein to differentiate certain structural features and components for the non-limiting, illustrative purposes of clarity and consistency. The terminology which has been used is intended to be in the nature of words of description rather than of limitation. Many modifications and variations are possible in light of the above teachings may be practiced otherwise than as specifically described.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.
I. A method of visualizing a patient shoulder anatomy, comprising:
   receiving image data of the patient shoulder anatomy comprising at least a portion of soft tissue and bones of a glenohumeral joint;
   generating one or more 3D models based on a segmentation of the image data;
   determining a location of at least one insertion point of the soft tissue corresponding to an attachment of soft tissue to the bones of the glenohumeral joint;
   generating a first virtual object based on the at least one insertion point of the soft tissue;
   determining the location of a glenoid track corresponding to a contact between a humerus and
   a glenoid based on the one or more 3D models and the first virtual object;
   generating a second virtual object based on the location of the glenoid track; and
   displaying:
      at least a portion of a rendering of the one or more 3D models,
      the first virtual object, and
      the second virtual object.
II. The method of clause I, wherein:
   determining the location of at least one insertion point of the soft tissue corresponding to the attachment of soft tissue to the bones of the glenohumeral joint comprises applying a machine learning model to the image data of the patient shoulder anatomy.
III. The method of any preceding clause, wherein:
   determining the location of at least one insertion point of the soft tissue corresponding to the attachment of soft tissue to the bones of the glenohumeral joint comprises applying curvature analysis to the image data of the patient shoulder anatomy.
IV. The method of any preceding clause, wherein the one or more 3D models comprises a humerus 3D model and wherein determining a location of at least one insertion point of the soft tissue corresponding to the attachment of soft tissue to the bones of the glenohumeral joint comprises fitting a plane based on a head of the humerus 3D model and a distance derived from a radius of the head of the humerus 3D model.
V. The method of any preceding clause, further comprising:
   determining a location of a healthy glenoid track corresponding to the contact between the humerus and a healthy glenoid based on the one or more 3D models; and
   generating a third virtual object based on the location of the healthy glenoid track.
VI. The method of clause V, wherein the third virtual object is a healthy glenoid track object and further comprising displaying the healthy glenoid track object.
VII. The method of any preceding clause, wherein the one or more 3D models comprises a glenoid 3D model and determining the location of the glenoid track comprises determining a glenoid width corresponding to the patient shoulder anatomy based on the glenoid 3D model.
VIII. The method of clause VII, wherein determining the glenoid width comprises determining a bone loss measure corresponding to the patient shoulder anatomy based on the glenoid 3D model.
IX. The method of clause VIII, wherein determining the glenoid width comprises determining a healthy glenoid width corresponding to a healthy glenoid based on the glenoid 3D model.
X. The method of clause IX, wherein determining the healthy glenoid width comprises applying statistical shape model fitting to the glenoid 3D model.
XI. The method of clause IX or X, wherein determining the healthy glenoid width comprises determining a width of a contralateral glenoid of the patient shoulder anatomy.
XII. The method of clause IX or X or XI, wherein determining the healthy glenoid width comprises:
   generating a circle representative of the healthy glenoid width based on the glenoid 3D model; and
   measuring a diameter of the circle.
XIII. The method of clause VIII, wherein determining the bone loss measure comprises: generating a circle representative of a healthy glenoid width based on the glenoid 3D model; and
   determining the bone loss measure based on a diameter of the circle.
XIV. The method of clause IX, wherein the second virtual object is a glenoid track object and generating the second virtual object comprises determining a boundary of the second virtual object based on:
   the first virtual object,
   the healthy glenoid width,
   the bone loss measure, and
   a threshold value.
XV. The method of clause XIV, wherein determining the boundary of the second virtual object comprises multiplying the healthy glenoid width by the threshold value and subtracting the bone loss measure.
XVI. The method of clause XIV, wherein the boundary of the second virtual object is further defined as a first boundary and generating a third virtual object based on a location of a healthy glenoid track corresponding to the contact between the humerus and a healthy glenoid comprises determining a second boundary of the third virtual object based on:
   the first virtual object,
   the healthy glenoid width, and
   the threshold value.
XVII. The method of clause XVI, wherein determining the second boundary comprises multiplying the healthy glenoid width by the threshold value.
XVIII. The method of any preceding clause, further comprising identifying a representation of a lesion on a humeral head based on the image data by:
   providing at least a portion of the image data as an input to a deep learning network; and
   receiving the representation of the lesion as an output from the deep learning network.
XIX. The method of clause XVIII, wherein the one or more 3D models comprises a humerus 3D model and further comprising displaying the representation of the lesion on the rendering of the humerus 3D model.
XX. The method of clause XVIII, wherein identifying the representation of the lesion comprises determining a boundary of the lesion on the humerus by applying one or more algorithms to the one or more 3D models, the one or more algorithms selected from the group comprising:
   statistical shape modeling,
   watershed analysis,
   edge detection, and
   curvature analysis.
XXI. The method of clause XX, further comprising determining an impact rating representing joint engagement based on the boundary of the lesion and the boundary of the second virtual object.
XXII. The method of clause XXI, further comprising displaying an indicator based on the impact rating.
XXIII. The method of clause XXII, wherein the indicator comprises a bone width necessary to restore a glenoid width to a healthy glenoid width.
XXIV. A method of visualizing a patient shoulder anatomy, comprising:
   receiving image data of the patient shoulder anatomy comprising a glenoid and a humerus;
   generating one or more 3D models based on a segmentation of the image data, wherein the one or more 3D models comprises a glenoid 3D model;
   generating a geometric primitive based on the glenoid 3D model;
   determining a glenoid width based on the geometric primitive and the glenoid 3D model;
   determining a location of a glenoid track based on the glenoid width;
   generating a virtual object based on the location of the glenoid track; and
   displaying:
      at least a portion of a rendering of the one or more 3D models, and
      the virtual object based on the location of the glenoid track.
XXV. The method of clause XXIV, wherein determining the glenoid width based on the geometric primitive comprises:
   determining a center of the glenoid 3D model of the patient shoulder anatomy;
   determining a center of the geometric primitive;
   generating a first virtual object connecting the center of the glenoid 3D model and the center of the geometric primitive; and
   generating a second virtual object perpendicular to the first virtual object.
XXVI. The method of clause XXV, wherein determining the glenoid width based on the geometric primitive further comprises:
   determining a glenoid rim based on the patient shoulder anatomy;
   determining a bone loss edge based on the glenoid rim inside of the second virtual object; and
   determining a bone loss measure by measuring a distance between the bone loss edge and the second virtual object.
XXVII. The method of clause XXVI, further comprising determining a healthy glenoid width based on a diameter of the second virtual object.
XXVIII. The method of clause XXVII, wherein the one or more 3D models comprises a glenoid 3D model and determining the healthy glenoid width comprises applying statistical shape model fitting to the glenoid 3D model.
XXIX. The method of clause XXVII, wherein determining the healthy glenoid width comprises determining a width of a contralateral glenoid of the patient shoulder anatomy.
XXX. The method of clause XXVII, wherein the virtual object based on the location of the glenoid track is further defined as a third virtual object and the method further comprises: determining a location of a healthy glenoid track corresponding to a contact between a humerus and a healthy glenoid based on the one or more 3D models; and
   generating a fourth virtual object based on the location of the healthy glenoid track.
XXXI. The method of clause XXX, wherein the fourth virtual object is a healthy glenoid track object and further comprising displaying the healthy glenoid track object.
XXXII. The method of clause XXX, further comprising:
   determining at least one attachment point of soft tissue to a bone of the patient shoulder anatomy; and
   generating a fifth virtual object corresponding to at least one attachment point of soft tissue.
XXXIII. The method of clause XXXII, wherein the third virtual object is a glenoid track object and generating the glenoid track object comprises determining a boundary of the glenoid track object based on:
   the fifth virtual object,
   the healthy glenoid width,
   the bone loss measure, and
   a threshold value.
XXXIV. The method of clause XXXIII, wherein determining the boundary of the glenoid track object comprises multiplying the healthy glenoid width by the threshold value and subtracting the bone loss measure.
XXXV. The method of clause XXXIV, wherein the boundary of the glenoid track object is further defined as a first boundary and generating a fourth virtual object based on the location of the healthy glenoid track corresponding to the contact between the humerus and a healthy glenoid comprises determining a second boundary of the fourth virtual object based on:
   the fifth virtual object,
   the healthy glenoid width, and
   the threshold value.
XXXVI. The method of clause XXXV, wherein identifying the second boundary comprises multiplying the healthy glenoid width by the threshold value.
XXXVII. The method of clause XXIV, further comprising identifying a representation of a lesion on a humeral head based on the image data by:
   providing at least a portion of the image data as an input to a deep learning network; and
   receiving the representation of the lesion as an output from the deep learning network.
XXXVIII. The method of clause XXXVII, wherein the one or more 3D models comprises a humerus 3D model and further comprising displaying the representation of the lesion on the rendering of the humerus 3D model.
XXXIX. The method of clause XXXVII, wherein identifying the representation of the lesion comprises determining a boundary of the lesion on the humerus by applying one or more algorithms to the one or more 3D models, the one or more algorithms selected from the group comprising:
   statistical shape modeling,
   watershed analysis,
   edge detection, and
   curvature analysis.
XL. The method of clause XXXIX, wherein the virtual object based on the location of the glenoid track is further defined as a glenoid track object and the method further comprises determining an impact rating representing joint engagement based on the boundary of the lesion and the boundary of the glenoid track object.
XLI. The method of clause XL, further comprising displaying an indicator based on the impact rating.
XLII. The method of clause XLI, wherein the indicator comprises a bone width necessary to restore a glenoid width to a healthy glenoid width.
XLIII. A method of visualizing a patient shoulder anatomy, comprising:
   receiving a first virtual object representing a planned bone block for joint reconstruction, the planned bone block comprising a reconstruction dimension;
   receiving a second virtual object representing an existing engagement between a humerus and a glenoid based on the patient shoulder anatomy;
   determining a reconstruction rating representing a modified engagement between the humerus and the glenoid based on the reconstruction dimension and the second virtual object; and displaying the reconstruction rating.
XLIV. The method of clause XLIII, further comprising determining a reconstructed glenoid track width based on the reconstruction dimension and the second virtual object.
XLV. The method of clause XLIV, wherein determining the reconstructed glenoid track width comprises determining a bone loss measure corresponding to the patient shoulder anatomy.
XLVI. The method of clause XLV, wherein determining the bone loss measure comprises:
   generating a circle representative of a healthy glenoid width based on the patient shoulder anatomy; and
   determining the bone loss measure based on a diameter of the circle.
XLVII. The method of clause XLVI, wherein determining the reconstructed glenoid track width comprises multiplying a diameter of the circle by a threshold value, subtracting the bone loss measure, and adding the reconstruction dimension.
XLVIII. The method of clause XLVII, further comprising determining a reconstructed glenoid width based on the reconstruction dimension and a width of a glenoid track based on the patient shoulder anatomy.
XLIX. The method of clause XLVIII, wherein determining the reconstructed glenoid track width comprises multiplying the reconstructed glenoid width by the threshold value.
L. The method of clause XLIX, wherein determining the reconstruction dimension is based on a threshold dimension of the reconstructed glenoid track width.
LI. The method of clause XLIX, wherein determining the reconstruction dimension is based on a threshold glenoid bone loss value.
LII. The method of clause L, wherein displaying the reconstruction rating comprises showing the reconstruction dimension.
LIII. A method of assessing a patient shoulder anatomy, comprising:
   receiving characteristics of a glenoid track corresponding to an engagement between a humerus and a glenoid;
   receiving characteristics of a healthy glenoid track corresponding to an engagement between a healthy humerus and a healthy glenoid;
   determining a restoration dimension of a bone block implant based on the characteristics of the glenoid track and the healthy glenoid track; and
   displaying an indicator based on the restoration dimension.
LIV. The method of clause LIII, further comprising determining a reconstruction position based on the characteristics of the glenoid track and the healthy glenoid track.
LV. The method of clause LIII, wherein the characteristics of the glenoid track comprise a glenoid track width based on the patient shoulder anatomy.
LVI. The method of clause LV, wherein characteristics of the healthy glenoid track comprise a healthy glenoid track width based on the patient shoulder anatomy.
LVII. The method of clause LVI, wherein determining the dimension of the bone block implant comprises:
   multiplying the glenoid track width by a threshold to determine a reconstruction width; and
   comparing the reconstruction width to the healthy glenoid track width.
LVIII. The method of clause LIII, further comprising:
   determining a coracoid dimension based on image data and a statistical shape model; and
   comparing the coracoid dimension with the dimension of the bone block implant, wherein the indicator is based on the comparison of the coracoid dimension to the dimension of the bone block implant.
LIX. The method of clause LIV, further comprising displaying the reconstruction position on a portion of a rendering of one or more 3D models generated from a segmentation of image data of the patient shoulder anatomy.
LX. A method of visualizing a patient shoulder anatomy, comprising:
   receiving image data of the patient shoulder anatomy;
   identifying a boundary of a lesion on a humerus based on the image data;
   generating one or more 3D models based on a segmentation of the image data;
   identifying a humeral neck axis based on a portion of the humerus in the one or more 3D models;
   identifying a humeral head apex based on the humeral neck axis and the one or more 3D models;
   generating a first set of lines perpendicular to the humeral neck axis;
   generating a second set of lines perpendicular to the first set of lines and passing through the humeral head apex; and
   displaying:
      at least a portion of a rendering of the one or more 3D models,
      the humeral head apex,
      the first set of lines,
   the second set of lines, and
   the boundary of the lesion.
LXI. The method of clause LX, further comprising identifying a representation of the lesion based on the image data by:
   providing at least a portion of the image data as an input to a deep learning network; and
   receiving the representation of the lesion as an output from the deep learning network.
LXII. The method of clause LXI, wherein the one or more 3D models comprises a humerus 3D model and further comprising displaying the representation of the lesion on the portion of the rendering of the humerus 3D model.
LXIII. The method of clause LX, wherein the boundary of the lesion on the humerus is identified by applying one or more algorithms to the one or more 3D models, the one or more algorithms selected from the group comprising:
   statistical shape modeling,
   watershed analysis,
   edge detection, and
   curvature analysis.
LXIV. The method of clause LX, wherein identifying the humeral head apex is based on a humeral reference center.
LXV. The method of clause LX, wherein the first set of lines are axially spaced along the humeral neck axis and indicate lateral-medial positions relative to the patient shoulder anatomy.
LXVI. The method of clause LX, wherein the second set of lines are radially spaced about the humeral neck axis and indicate superior-inferior positions relative to the patient shoulder anatomy.
LXVII. A method of visualizing a patient shoulder anatomy, comprising:
   receiving image data of the patient shoulder anatomy;
   identifying a boundary of a lesion on a humerus based on the image data;
   generating one or more 3D models based on segmentation of the image data;
   identifying a humeral head apex based on the one or more 3D models;
   determining at least one distance based on the boundary of the lesion and the humeral head apex; and
   displaying:
      at least a portion of a rendering of the one or more 3D models,
      the boundary of the lesion,
      the humeral head apex, and
   at least one distance between the boundary of the lesion and the humeral head apex.
LXVIII. The method of clause LXVII, further comprising identifying a representation of the lesion based on the image data by:
   providing at least a portion of the image data as an input to a deep learning network; and
   receiving the representation of the lesion as an output from the deep learning network.
LXIX. The method of clause LXVIII, wherein the one or more 3D models comprises a humerus 3D model and further comprising displaying the representation of the lesion on the humerus 3D model.
LXX. The method of clause LXVII or LXVIII or LXIX, wherein the boundary of the lesion on the humerus is identified by applying one or more algorithms to the one or more 3D models, the one or more algorithms selected from the group comprising:
   statistical shape modeling,
   watershed analysis,
   edge detection, and
   curvature analysis.
LXXI. The method of clause LXVII, wherein identifying the humeral head apex is based on a humeral reference center.
LXXII. The method of clause LXVII, wherein the distance between the boundary of the lesion and the humeral head apex is a geodesic distance.
LXXIII. A method of visualizing a patient shoulder anatomy, comprising:
   receiving image data of the patient shoulder anatomy;
   identifying a boundary of a lesion on a humerus based on the image data;
   generating one or more 3D models based on a segmentation of the image data;
   identifying a humeral neck axis based on the one or more 3D models;
   determining a lesion line based on the one or more 3D models;
   determining an angle based on the lesion line and the humeral neck axis; and
   displaying:
      at least a portion of a rendering of the one or more 3D models,
      the boundary of the lesion, and
      the angle between the lesion line and the humeral neck axis.
LXXIV. The method of clause LXXIII, further comprising identifying a representation of the lesion based on the image data by:
   providing at least a portion of the image data as an input to a deep learning network; and
   receiving the representation of the lesion as an output from the deep learning network.
LXXV. The method of clause LXXIV, wherein the one or more 3D models comprises a humerus 3D model and further comprising displaying the representation of the lesion on the humerus 3D model.
LXXVI. The method of clause LXXIII, wherein the boundary of the lesion on the humerus is identified by applying one or more algorithms to the one or more 3D models, the one or more algorithms selected from the group comprising:
   statistical shape modeling,
   watershed analysis,
   edge detection, and
   curvature analysis.
LXXVII. The method of clause LXXIII, wherein the one or more 3D models comprises a humerus 3D model, wherein the humerus 3D model includes an articular surface and a humeral head, and wherein identifying the humeral neck axis comprises fitting an articular sphere to the articular surface of the humerus 3D model.
LXXVIII. The method of clause LXXVII, wherein identifying the humeral neck axis further comprises identifying a contour of the humeral head based on an articular margin of the humerus 3D model and generating a virtual object based on the contour.
LXXIX. The method of clause LXXVIII, wherein identifying the articular margin of the humerus 3D model comprises determining an articular margin center based on the virtual object representing the articular margin.
LXXX. The method of clause LXXIX, wherein the humeral neck axis is perpendicular to the virtual object representing the articular margin and is based on the articular surface of a humeral head and the articular margin center.
LXXXI. The method of clause LXXX, wherein the lesion line is based on the articular margin center and the boundary of the lesion.
LXXXII. The method of clause LXXXI, wherein the boundary of the lesion is further defined as a medial edge of the lesion.
LXXXIII. A non-transitory computer readable storage medium having stored therein data representing instructions executable by a programmed processor for visualizing a patient shoulder anatomy, the storage medium comprising instructions for:
   receiving image data of the patient shoulder anatomy;
   identifying a boundary of a lesion on a humerus based on the image data;
   generating one or more 3D models based on a segmentation of the image data;
   determining a location of a glenoid track corresponding to a contact between the humerus and
   a glenoid based on the one or more 3D models;
   generating a first virtual object based on the location of the glenoid track; and
   displaying:
      at least a portion of a rendering of the one or more 3D models,
      the boundary of the lesion, and
      the first virtual object.
LXXXIV. A non-transitory computer readable storage medium having stored therein data representing instructions executable by a programmed processor for visualizing a patient shoulder anatomy, the storage medium comprising instructions for:
   receiving image data of the patient shoulder anatomy comprising at least a portion of soft tissue and bones of a glenohumeral joint;
   generating one or more 3D models based on a segmentation of the image data;
   determining a location of at least one insertion point of the soft tissue corresponding to an attachment of soft tissue to the bones of the glenohumeral joint;
   generating a first virtual object based on the at least one insertion point of the soft tissue;
   determining the location of a glenoid track corresponding to a contact between a humerus and
   a glenoid based on the one or more 3D models and the first virtual object;
   generating a second virtual object based on the location of the glenoid track; and
   displaying:
      at least a portion of a rendering of the one or more 3D models,
      the first virtual object, and
      the second virtual object.
LXXXV. A non-transitory computer readable storage medium having stored therein data representing instructions executable by a programmed processor for visualizing a patient shoulder anatomy, the storage medium comprising instructions for:
   receiving image data of the patient shoulder anatomy comprising a glenoid and a humerus;
   generating one or more 3D models based on a segmentation of the image data, wherein the one or more 3D models comprises a glenoid 3D model;
   generating a geometric primitive based on the glenoid 3D model;
   determining a glenoid width based on the geometric primitive and the glenoid 3D model;
   determining a location of a glenoid track based on the glenoid width;
   generating a virtual object based on the location of the glenoid track; and
   displaying:
      at least a portion of a rendering of the one or more 3D models, and
      the virtual object based on the location of the glenoid track.
LXXXVI. A non-transitory computer readable storage medium having stored therein data representing instructions executable by a programmed processor for visualizing a patient shoulder anatomy, the storage medium comprising instructions for:
   receiving a first virtual object representing a planned bone block for joint reconstruction, the planned bone block comprising a reconstruction dimension;
   receiving a second virtual object representing an existing engagement between a humerus and a glenoid based on the patient shoulder anatomy;
   determining a reconstruction rating representing a modified engagement between the humerus and the glenoid based on the reconstruction dimension and the second virtual object; and displaying the reconstruction rating.
LXXXVII.A non-transitory computer readable storage medium having stored therein data representing instructions executable by a programmed processor for visualizing a patient shoulder anatomy, the storage medium comprising instructions for:
   receiving characteristics of a glenoid track corresponding to an engagement between a humerus and a glenoid;
   receiving characteristics of a healthy glenoid track corresponding to an engagement between a healthy humerus and a healthy glenoid;
   determining a dimension of a bone block implant based on the characteristics of the glenoid track and the healthy glenoid track; and
   displaying an indicator based on the dimension.
LXXXVIII.A non-transitory computer readable storage medium having stored therein data representing instructions executable by a programmed processor for visualizing a patient shoulder anatomy, the storage medium comprising instructions for:
   receiving image data of the patient shoulder anatomy;
   identifying a boundary of a lesion on a humerus based on the image data;
   generating one or more 3D models based on a segmentation of the image data;
   identifying a humeral neck axis based on a portion of the humerus in the one or more 3D models;
   identifying a humeral head apex based on the humeral neck axis and the one or more 3D models;
   generating a first set of lines perpendicular to the humeral neck axis;
   generating a second set of lines perpendicular to the first set of lines and passing through the humeral head apex; and
   displaying:
      at least a portion of a rendering of the one or more 3D models,
      the humeral head apex,
      the first set of lines,
   the second set of lines, and
   the boundary of the lesion.
LXXXIX. A non-transitory computer readable storage medium having stored therein data representing instructions executable by a programmed processor for visualizing a patient shoulder anatomy, the storage medium comprising instructions for:
   receiving image data of the patient shoulder anatomy;
   identifying a boundary of a lesion on a humerus based on the image data;
   generating one or more 3D models based on segmentation of the image data;
   identifying a humeral head apex based on the one or more 3D models;
   determining at least one distance based on the boundary of the lesion and the humeral head apex; and
   displaying:
      at least a portion of a rendering of the one or more 3D models,
      the boundary of the lesion,
      the humeral head apex, and
   at least one distance between the boundary of the lesion and the humeral head apex.
XC. A non-transitory computer readable storage medium having stored therein data representing instructions executable by a programmed processor for visualizing a patient shoulder anatomy, the storage medium comprising instructions for:
   receiving image data of the patient shoulder anatomy;
   identifying a boundary of a lesion on a humerus based on the image data;
   generating one or more 3D models based on a segmentation of the image data;
   identifying:
      a humeral neck axis based on the one or more 3D models, and
      a lesion line based on the one or more 3D models;
      determining an angle based on the lesion line and the humeral neck axis;
      displaying:
         at least a portion of a rendering of the one or more 3D models,
         the boundary of the lesion, and
         the angle between the lesion line and the humeral neck axis.
XCI. A computing system comprising: a memory configured to store image data of the patient shoulder anatomy; and a controller configured to:
   receive image data of the patient shoulder anatomy;
   identify a boundary of a lesion on a humerus based on the image data;
   generate one or more 3D models based on a segmentation of the image data;
   determine a location of a glenoid track corresponding to a contact between the humerus and a glenoid based on the one or more 3D models;
   generate a first virtual object based on the location of the glenoid track; and
   display:
      at least a portion of a rendering of the one or more 3D models,
      the boundary of the lesion, and
      the first virtual object.
XCII. A computing system comprising:
   a memory configured to store image data of the patient shoulder anatomy; and
   a controller configured to:
      receive image data of the patient shoulder anatomy comprising at least a portion of soft tissue and bones of a glenohumeral joint;
      generate one or more 3D models based on a segmentation of the image data;
      determine a location of at least one insertion point of the soft tissue corresponding to an attachment of soft tissue to the bones of the glenohumeral joint;
      generate a first virtual object based on the at least one insertion point of the soft tissue;
      determine the location of a glenoid track corresponding to a contact between a humerus and a glenoid based on the one or more 3D models and the first virtual object;
      generate a second virtual object based on the location of the glenoid track; and
      display:
         at least a portion of a rendering of the one or more 3D models,
         the first virtual object, and
         the second virtual object.
XCIII. A computing system comprising:
   a memory configured to store image data of the patient shoulder anatomy; and
   a controller configured to:
      receive image data of the patient shoulder anatomy comprising a glenoid and a humerus;
      generate one or more 3D models based on a segmentation of the image data, wherein the one or more 3D models comprises a glenoid 3D model;
      generate a geometric primitive based on the glenoid 3D model;
      determine a glenoid width based on the geometric primitive and the glenoid 3D model;
      determine a location of a glenoid track based on the glenoid width;
      generate a virtual object based on the location of the glenoid track; and
      display:
         at least a portion of a rendering of the one or more 3D models, and
         the virtual object based on the location of the glenoid track.
XCIV. A computing system comprising:
   a memory configured to store image data of the patient shoulder anatomy; and
   a controller configured to:
      receive a first virtual object representing a planned bone block for joint reconstruction, the planned bone block comprising a reconstruction dimension;
      receive a second virtual object representing an existing engagement between a humerus and a glenoid based on the patient shoulder anatomy;
      determine a reconstruction rating representing a modified engagement between the humerus and the glenoid based on the reconstruction dimension and the second virtual object; and display the reconstruction rating.
XCV. A computing system comprising:
   a memory configured to store image data of the patient shoulder anatomy; and
   a controller configured to:
      receive characteristics of a glenoid track corresponding to an engagement between a humerus and a glenoid;
      receive characteristics of a healthy glenoid track corresponding to an engagement between a healthy humerus and a healthy glenoid;
      determine a dimension of a bone block implant based on the characteristics of the glenoid track and the healthy glenoid track; and
      display an indicator based on the dimension.
XCVI. A computing system comprising:
   a memory configured to store image data of the patient shoulder anatomy; and
   a controller configured to: receive image data of the patient shoulder anatomy;
   identify a boundary of a lesion on a humerus based on the image data;
   generate one or more 3D models based on a segmentation of the image data;
   identify a humeral neck axis based on a portion of the humerus in the one or more 3D models;
   identify a humeral head apex based on the humeral neck axis and the one or more 3D models;
   generate a first set of lines perpendicular to the humeral neck axis;
   generate a second set of lines perpendicular to the first set of lines and passing through the humeral head apex; and
   display:
      at least a portion of a rendering of the one or more 3D models,
      the humeral head apex,
      the first set of lines,
   the second set of lines, and
   the boundary of the lesion.
XCVII. A computing system comprising:
   a memory configured to store image data of the patient shoulder anatomy; and
   a controller configured to: receive image data of the patient shoulder anatomy;
   identify a boundary of a lesion on a humerus based on the image data;
   generate one or more 3D models based on segmentation of the image data;
   identify a humeral head apex based on the one or more 3D models;
   determine at least one distance based on the boundary of the lesion and the humeral head apex; and
   display:
      at least a portion of a rendering of the one or more 3D models,
      the boundary of the lesion,
      the humeral head apex, and
   at least one distance between the boundary of the lesion and the humeral head apex.
XCVIII. A computing system comprising:
   a memory configured to store image data of the patient shoulder anatomy; and
   a controller configured to:

   receive image data of the patient shoulder anatomy;
   identify a boundary of a lesion on a humerus based on the image data;
   generate one or more 3D models based on a segmentation of the image data;
   identify a humeral neck axis based on the one or more 3D models;
   determine a lesion line based on the one or more 3D models;
   determining an angle based on the lesion line and the humeral neck axis; and
   display:
      at least a portion of a rendering of the one or more 3D models,
      the boundary of the lesion, and
      the angle between the lesion line and the humeral neck axis.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A method of visualizing a patient shoulder anatomy, comprising:
receiving image data of the patient shoulder anatomy;
identifying a boundary of a lesion on a humerus based on the image data;
generating one or more 3D models based on a segmentation of the image data;
determining a location of a glenoid track corresponding to a contact between the humerus and a glenoid based on the one or more 3D models;
generating a first virtual object based on the location of the glenoid track; and
displaying:
at least a portion of a rendering of the one or more 3D models,
the boundary of the lesion, and
the first virtual object.

2. The method of claim 1, wherein the method of visualizing the patient shoulder anatomy further comprises:
determining a location of a healthy glenoid track corresponding to the contact between the humerus and a healthy glenoid based on the one or more 3D models; and
generating a second virtual object based on the location of the healthy glenoid track, and optionally, wherein the second virtual object is a healthy glenoid track object and further comprising displaying the healthy glenoid track object.

3. The method of any preceding claim, wherein the one or more 3D models comprises a glenoid 3D model, the first virtual object is a glenoid track object, and determining the location of the glenoid track comprises determining a glenoid width corresponding to the patient shoulder anatomy based on the glenoid 3D model.

4. The method of claim 3, wherein determining the glenoid width comprises determining a bone loss measure corresponding to the patient shoulder anatomy based on the glenoid 3D model, wherein determining the bone loss measure comprises:
generating a circle representative of a healthy glenoid width based on the glenoid 3D model; and
determining the bone loss measure based on a diameter of the circle.

5. The method of claim 3 or 4, wherein determining the glenoid width comprises determining a healthy glenoid width corresponding to a healthy glenoid based on the glenoid 3D model.

6. The method of claim 5, wherein determining the healthy glenoid width comprises applying statistical shape model fitting to the glenoid 3D model, or
wherein determining the healthy glenoid width comprises determining a width of a contralateral glenoid of the patient shoulder anatomy, or
wherein determining the healthy glenoid width comprises:
generating a circle representative of the healthy glenoid width based on the glenoid 3D model; and
measuring a diameter of the circle.

7. The method of any one of claims 5 and 6, further comprising:
determining at least one attachment point of soft tissue to a bone of the patient shoulder anatomy; and
generating a third virtual object corresponding to at least one attachment point of soft tissue; and wherein generating the first virtual object comprises determining a boundary of the first virtual object based on:
the third virtual object,
the healthy glenoid width,
the bone loss measure, and
a threshold value.

8. The method of claim 7, wherein determining the boundary of the first virtual object comprises multiplying the healthy glenoid width by the threshold value and subtracting the bone loss measure.

9. The method of claim 7 or 8, wherein the boundary of the first virtual object is further defined as a first boundary and generating a second virtual object based on a location of a healthy glenoid track corresponding to the contact between the humerus and a healthy glenoid comprises determining a second boundary of the healthy glenoid track based on:
the third virtual object,
the healthy glenoid width, and
the threshold value.

10. The method of claim 9, wherein determining the second boundary comprises multiplying the healthy glenoid width by the threshold value.

11. The method of any preceding claim, further comprising identifying a representation of the lesion based on the image data by:
providing at least a portion of the image data as an input to a deep learning network; and
receiving the representation of the lesion as an output from the deep learning network;
and, optionally, wherein the one or more 3D models comprises a humerus 3D model and further comprising displaying the representation of the lesion on a rendering of the humerus 3D model.

12. The method of any preceding claim, wherein the boundary of the lesion on the humerus is identified by applying one or more algorithms to the one or more 3D models, the one or more algorithms selected from the group comprising:
statistical shape modeling,
watershed analysis,
edge detection, and
curvature analysis.

13. The method of any preceding claim, further comprising determining an impact rating representing joint engagement based on the boundary of the lesion and a boundary of the first virtual object. and displaying an indicator based on the impact rating.

14. The method of claim 13, wherein the indicator comprises a bone width necessary to restore a glenoid width to a healthy glenoid width.

15. A computing system comprising: a memory configured to store image data of a patient shoulder anatomy; and
a controller configured to perform the method of any of claims 1-14.

16. A computer program product comprising data representing instructions executable by a programmed processor for visualizing a patient shoulder anatomy, the storage medium comprising instructions for performing the method of claims 1-14.
